(19) Europäisches Patentamt
European Patent Office
Office européen des brevets

(11) **EP 2 711 021 B1**

(12) # EUROPEAN PATENT SPECIFICATION

(45) Date of publication and mention of the grant of the patent:
**09.03.2016 Bulletin 2016/10**

(51) Int Cl.:
***A61K 39/385*** *(2006.01)* ***A61K 39/39*** *(2006.01)*
***A61K 39/00*** *(2006.01)*

(21) Application number: **13189439.6**

(22) Date of filing: **05.05.2009**

(54) **Products and methods for stimulating an immune response**

Produkte und Verfahren zur Stimulation einer Immunantwort

Produits et procédés permettant de stimuler une réponse immunitaire

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **02.05.2008 US 49814 P**

(43) Date of publication of application:
**26.03.2014 Bulletin 2014/13**

(62) Document number(s) of the earlier application(s) in accordance with Art. 76 EPC:
**09738408.5 / 2 288 381**

(73) Proprietor: **BliNK Therapeutics Limited**
**Marlow**
**Buckinghamshire SL7 3HN (GB)**

(72) Inventors:
- **Batista, Facundo**
  **London Oxfordshire WC2A 3PX (GB)**
- **Eckl-Dorna, Julia**
  **London WC2A 3PX (GB)**
- **Barral, Patricia**
  **London WC2A 3PX (GB)**
- **Cerundolo, Vincenzo**
  **Oxford**
  **Oxfordshire OX3 9DS (GB)**

(74) Representative: **Hayes, Emily Anne Luxford**
**Mewburn Ellis LLP**
**City Tower**
**40 Basinghall Street**
**London EC2V 5DE (GB)**

(56) References cited:
**US-A- 5 871 747** **US-A- 6 004 763**

- **BATISTA F D ET AL: "B CELLS EXTRACT AND PRESENT IMMOBILIZATION ANTIGEN: IMPLICATIONS FOR AFFINITY DISCRIMINATION", EMBO JOURNAL, OXFORD UNIVERSITY PRESS, SURREY, GB, vol. 19, no. 4, 1 January 2000 (2000-01-01), pages 513-520, XP000910188, ISSN: 0261-4189**
- **VIDARD L ET AL: "Analysis of MHC class II presentation of particulate antigens of B lymphocytes.", JOURNAL OF IMMUNOLOGY (BALTIMORE, MD. : 1950) 15 APR 1996, vol. 156, no. 8, 15 April 1996 (1996-04-15), pages 2809-2818, XP002539639, ISSN: 0022-1767**
- **MARTIN M E D ET AL: "Polymerized serum albumin beads possessing slow release properties for use in vaccines", VACCINE, ELSEVIER LTD, GB, vol. 6, no. 1, 1 February 1988 (1988-02-01), pages 33-38, XP023709443, ISSN: 0264-410X, DOI: 10.1016/0264-410X(88)90011-4 [retrieved on 1988-02-01]**
- **SCHEERLINCK J P Y ET AL: "Systemic immune responses in sheep, induced by a novel nano-bead adjuvant", VACCINE, ELSEVIER LTD, GB, vol. 24, no. 8, 20 February 2006 (2006-02-20), pages 1124-1131, XP028010483, ISSN: 0264-410X, DOI: 10.1016/J.VACCINE.2005.09.009 [retrieved on 2006-02-20]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention relates to methods for inducing a BCR-mediated specific immune response. In particular the present invention provides methods relating to products comprising a BCR-binding antigen and an immunostimulant.

### BACKGROUND OF THE INVENTION

As antibodies are designed to specifically recognise and eliminate invading antigens, they are effective weapons employed by the immune system to combat, for example, infection or cancer. In order to elicit antibody production, B cells must be activated in a process that is initiated by specific antigen recognition through the B cell receptor (BCR) (1). Specific antigen engagement initiates two BCR-mediated processes: firstly, the transmission of intracellular signals regulating entry into cell cycle (2,3); and secondly, antigen internalisation prior to its processing and presentation in association with MHC to specific T cells (4). T-cell stimulated B cells can either differentiate into extrafollicular plasma cells (PCs), or develop into germinal centre B cells. Short-lived extrafollicular PCs mediate the secretion of the first wave of predominantly low affinity antibodies, some of which may have undergone class-switching (5). Alternatively germinal centre B cells undergo somatic hypermutation and affinity maturation leading to the selection of high-affinity B cell clones that differentiate into either long-lived plasma cells or memory B cells (6).

During the development of immune responses BCR-mediated uptake of antigen allows for its concentration and delivery to specialized late endosomes containing newly synthesized MHC class II molecules (7).

For many years, peptides were assumed to be the only antigenic determinant initiating T cell responses. However, it is now evident that T cells are also able to recognize and respond to antigenic lipids and glycolipids, presented by CD1 molecules (8). The human CD1 gene family is composed of five non-polymorphic genes (*CD1A, -B, -C, -D,* and -E), located in a small cluster on chromosome 1 (9). In contrast, mice express only CD1d molecules. CD1 genes have an intron-exon structure comparable to that of MHC class I genes and encode type I integral membrane proteins consisting of α1, α2, and α3 domains, similar to MHC class I molecules, non-covalently linked to β2 microglobulin β2). In a manner similar to MHC class II molecules, CD1 proteins mediate the presentation of antigenic lipids on the surface of APCs after they are loaded or processed in intracellular compartments (10). CD1d is expressed on various hematopoietic cells including dendritic cells, thymocytes, B cells, T cells, and monocytes.

The immunogenicity of an antigen can be enhanced by the use of an immunostimulant. α-galactosylceramide (αGalCer) for example, a marine sponge derived glycolipid, is a well characterized iNKT (invariant natural killer T) cell antigen, with proven capacity to stimulate strongly both murine and human iNKT cells (14,15). Further, αGalCer has also been suggested to be useful as an immunostimulant when administered together with the antigen (US2003/0157135) or when directly linked to an antigen (WO2007/051004).

TLR (Toll-like receptor) agonists have also been used as immunostimulants. The TLR family now consists of 13 members: although only TLR1 to TLR10 haven been identified in humans. TLRs are members of the toll/IL-IR (TIR) domain containing superfamily, and there is considerable homology between the cytoplasmic domains of all family members (Ulevitch, Nature reviews, July 2004, Vol 4, 512-520). TLRs can be divided into two categories according to the site in the cell at which they recognise their particular ligands. TLRs 1, 2, 4, 5 and 6 are expressed on the surface of the cell where they mediate recognition of bacterial products. On the other hand a subset of TLRs, such as TLRs 3, 7, 8 and 9 are localised to intracellular compartments where they can respond to nucleic acids.

At present, there remains a need for compositions and methods for inducing an efficient specific BCR-mediated immune response.

### SUMMARY OF THE INVENTION

Highly regulated activation of B cells is required for the production of specific antibodies. This process is initiated by specific recognition of antigen through the B cell receptor (BCR), leading to early intracellular signalling followed by the recruitment of specific T cell help. The inventors have devised novel products and methods for inducing BCR-mediated antigen-specific immune responses to an antigen, which products and methods generally employ the use of a support with antigen attached thereto, i.e. a particulate antigen. An immunostimulant may also be attached to the support, or may be provided in soluble form together with the particulate antigen.

The invention is based on the inventors' discovery that the specific binding of particulate antigen to BCR and subsequent internalization is dependent on the overall avidity of antigen present on the surface as seen by the BCR on a B cell. This observation is most likely interpreted as a requirement for a minimum degree of BCR clustering necessary for triggering internalisation of particulate antigen.

Use of a support allows fine regulation of the antigen density, and thereby avidity. BCR-mediated internalization of particulate antigen can occur even in response to low affinity antigen, provided a defined avidity threshold is exceeded. The particulate antigen is then efficiently taken up by a B cell via BCR-engagement, leading to an antigen-specific BCR-mediated immune response(s). Being able to regulate the density and therefore avidity on the support represents an exquisite mechanism which allows fine-control and modulation of antigen-specific immune responses. By varying the antigen density on the support, one can influence and regulate the immune response. The particulate antigen, i.e. the support with antigen attached thereto, is presented to a B cell in combination with an immunostimulant. By 'combination' it is understood that either the immunostimulant is also attached to the support, or that the immunostimulant is administered conjointly with the particulate antigen.

"Conjoint" administration as used herein refers to administration of a soluble immunostimulant and a particulate antigen as described herein either simultaneously in one composition, or simultaneously in different compositions, or sequentially. With respect to sequential administration, the immunostimulant and particulate antigen are administered separated by a time interval that still allows the immunostimulant to enhance the antigen-specific immune response.

Once the particulate antigen has been internalized by a specific B cell, the immunostimulant effects an enhancement of the specific immune response. The enhancement mechanism may for example involve presentation of antigen and/or immunostimulant by the B cell to T cells, such as iNKT cells, secretion of cytokines or chemokines, involvement of dendritic cells, or cascades of cells involved in the immune system. In any case, it will lead to a stimulatory feed-back to the B cell, resulting in an enhancement of the antigen-specific immune response.

Combining particulate BCR-binding antigen and soluble immunostimulant leads to an enhanced specific immune response compared to soluble antigen. Although the use of soluble immunostimulant, such as $\alpha$-GalCer, may lead to unspecific uptake of the immunostimulant (as discussed in more detail below), use of an antigen in particulate form together with a soluble immunostimulant increases the immunogenicity of the antigen and results in enhanced antigen-specific immune responses.

An even more enhanced immune response is achieved by attaching the antigen and the immunostimulant to the same support. Antigen and immunostimulant are thus internalized together by a target B cell in a BCR-dependent manner. Upon BCR-mediated internalization of the particulate antigen, the B cell may present the antigen, and depending on the immunostimulant, also the immunostimulant (such as immunostimulatory lipidic antigens) to one or more types of T cells. The activated T cell will in turn (directly or indirectly, i.e. via other cells) stimulate the B cell to differentiate. Alternative immunostimulants (such as endosomal TLR ligands) may trigger intracellular cascades that result in, for example, cell activation or the secretion of cytokines that may potentiate other immune cell responses. In addition it is possible that the immunostimulant may enhance the maturation of DCs (for example through the recruitment of iNKT developmental help) and as a consequence activate the presentation of antigen on their surface in combination with MHC molecules to specific CD4+ T helper cells. Following stimulation these specific CD4+ T helper cells may then provide the necessary help for the development of antigen-specific B cells. Thus through each of these mechanisms or a combination of these mechanisms the antigen-specific immune responses are enhanced through the conjugation of antigen and immunostimulant on a surface.

WO2007/051004 suggests to directly link an antigen with an immunostimulant to form a conjugate, but does not suggest the products and methods of the present invention.

While the bulk of particulate antigen will be taken up by B cells via specific BCR, thereby determining the specificity of the immune response, dendritic cells (DCs) and macrophages may take up particulate antigen via micropinocytosis and phagocytosis, which may contribute to the overall observed immune response.

As mentioned above, soluble immunostimulant is generally taken up by cells in a relatively nonspecific manner, potentially mediated by receptors such as the LDL receptor in the case of immunostimulatory lipids, such as $\alpha$GalCer. However, the conjugation of immunostimulants into a particulate form inhibits the mechanism employed by soluble immunostimulants to enter B cells. The inventors have demonstrated that immunostimulants when present in particulate form cannot enter B cells via the same non-specific mechanisms employed by soluble immunostimulants.

Thus, as a particulate immunostimulant cannot enter into B cells in a non-specific manner, it does not trigger non-specific immune responses. In order to gain entry into the target cell particulate immunostimulants require specific uptake. This is achieved through conjugation with antigen to allow BCR-mediated internalisation. This mechanism of internalisation is more efficient compared to soluble immunostimulant, and thus gives rise to enhanced and specific immune responses.

The inventors have demonstrated that particulate immunostimulants may enter specific cells through the inclusion of an antigen on the surface through a BCR-mediated mechanism of internalization. The inventors have further demonstrated that this mechanism of internalization of particulate antigen represents a more efficient mechanism of internalization compared with that observed for soluble immunostimulant, as demonstrated by enhancement in antigen-specific immune responses.

Thus, presenting an antigen and an immunostimulant on the same support results in (i) specific delivery of the particulate antigen to a BCR-expressing cell and BCR-mediated internalization (ii) enhancement of the specific immune response.

The inventors have further shown that if an agent (such as an immunostimulant) is attached to a support (in the absence of a BCR antigen on the support), the agent is not taken up by a B cell but still internalized by dendritic cells. Methods and products described herein can thus be used for directed delivery of an agent, such as an immunostimulant, to dendritic cells, i.e. preferential delivery to dendritic cells versus B cells.

With respect to immunostimulatory lipids, the inventors have found that specific BCR-mediated uptake of CD1d-presented immunostimulatory particulate lipids represents an effective means of enhancing invariant NKT (iNKT)-dependent B cell responses in vivo. This mechanism is effective over a wide range of antigen affinities but is dependent on exceeding a tightly regulated avidity threshold necessary for BCR-mediated internalization and subsequent CD1d-dependent presentation of the particulate lipid immunostimulant. Subsequently, iNKT cells provide the help required for stimulating B cell proliferation and differentiation. Interestingly iNKT-stimulated B cells develop within extrafollicular foci and mediate the production of high titres of specific IgM and early class-switched antibodies. Thus, the inventors have demonstrated that in response to particulate immunostimulatory lipids iNKT cells are recruited for the assistance of B cell activation, resulting in the enhancement of antigen specific antibody responses.

Furthermore, the inventors have found that B cells stimulated with a support having an antigen and a TLR agonist attached thereto leads to proliferation and differentiation both in vitro and in vivo and results in the enhanced production of antigen-specific antibodies. In a similar manner the observed enhanced antigen-specific immune response is dependent on exceeding a threshold of avidity required for BCR-mediated internalisation.

Thus, the inventors have utilized the BCR as a means of achieving efficient and selective internalization of particulate antigen, as well as selective delivery and internalization of an immunostimulant attached to the particulate antigen, leading to an increased specific immune response.

Disclosed herein is a product capable of BCR-mediated internalization comprising

(i) a support, and
(ii) a BCR-binding antigen attached to the support.

Disclosed herein is a pharmaceutical composition comprising a product as described herein and a suitable carrier.

Disclosed herein is a product or composition as described herein for use as a vaccine.

The invention provides a method of inducing an antigen-specific immune response according to claim 1.

Disclosed herein is a method for augmenting the immunogenicity of a BCR-binding antigen in a subject, comprising administering to the subject a product comprising a support and said antigen attached to the support, and wherein an immunostimulant is either attached to the support or administered conjointly with said product.

Disclosed herein is a process for making a product as described herein, comprising the steps of

(a) attaching a BCR-binding antigen to a support, and, optionally
(b) attaching an immunostimulant to the support,

optionally performing steps (a) and (b) in reversed order.

Disclosed herein is a method for augmenting the antigen-specific immune response to a BCR-binding antigen in a subject, the method comprising the steps of

(a) attaching the antigen to a support, and
(b) attaching an immunostimulant to the support, optionally performing steps (a) and (b) in reversed order, and
(c) administering the support to a subject,

allow specific BCR-mediated internalization and B cell activation.

Disclosed herein is a support for use in preparing a product or composition as described herein, wherein an immunostimulant is attached to the support.

Disclosed herein is a method for enhancing a BCR-mediated, immune response in a subject, comprising administering to a subject a product or composition as described herein.

Disclosed herein is a method of inducing specific uptake of an immunostimulant by a cell, comprising the steps of

(i) attaching a BCR-binding antigen to a support,
(ii) attaching the immunostimulant to said support, optionally performing steps (i) and (ii) in reversed order,
and
(iii) contacting the cell with said support to allow specific BCR-mediated internalization and B cell activation.

Disclosed herein is a method of delivering a BCR-binding antigen and an immunostimulant to a cell for eliciting an antigen-specific immune response, comprising

(i) attaching a BCR-binding antigen to a support at a first density,
(ii) attaching the immunostimulant to said support, optionally performing steps (i) and (ii) in reversed order,
(iii) contacting the support with a cell,
(iv) testing the cell for antigen-mediated activation, optionally repeating steps (ii) to (iv) with a varied density of the antigen.

Disclosed herein is a method of producing an anti-serum against an antigen, said method comprising introducing a product or composition as described herein into a non-human mammal, and recovering immune serum from said mammal.

Disclosed herein is an immune serum obtainable by methods described herein. In a further aspect the invention provides an antibody, obtainable from said serum.

Disclosed herein is specific antibody producing B cells obtainable by methods described herein.

Disclosed herein are immortalised B-cells producing specific antibody (hybridoma) obtainable by methods described herein.

Disclosed herein is a method of passive immunisation against a disease, said method comprising administering to a subject an immune serum containing antibodies as described herein.

Disclosed herein is a product or composition as describe herein for use as a medicament.

Disclosed herein is a product or composition as describe herein for use in a method for the treatment of cancer, an infectious disease, an allergy or an autoimmune disease.

Disclosed herein is the use of the product or composition as described herein in the manufacture of a medicament for the treatment of cancer, and infectious disease, an allergy or an autoimmune disease.

Disclosed herein is a method for generating an immortalized cell, said method comprising the steps of

(i) contacting a B cell with a product or composition as described herein, and
(ii) immortalising the B cell of step (i).

Immortalisation of B-cells can be achieved by various means such as for example (but not limited to) transforming with a suitable virus, fusion with another immortalised cell, or ligation with CD40.

Disclosed herein is a method for generating immortalised B lymphocytes, the method comprising the step of (i) immortalising B lymphocytes ex vivo in the presence of the product or composition described herein. Immortalisation of B-cells can be achieved by various means such as for example (but not limited to) transforming with a suitable virus, fusion with another immortalised cell, ligation with CD40.

Disclosed herein is a method for generating a clone of an immortalized B lymphocyte capable of producing a monoclonal antibody, the method comprising:

(i) contacting a B lymphocyte ex vivo with a product or composition as described herein, and
(ii) immortalising the B cell of step (i).

Immortalisation of B-cells can be achieved by various means such as for example (but not limited to) transforming with a suitable virus, fusion with another immortalised cell, or ligation with CD40. The method may further comprise the step of (iii) screening the transformed B cell for antigen specificity. The method may further comprise isolating the immortalized B cell.

Disclosed herein is a method for generating a clone of an immortalized B lymphocyte capable of producing a monoclonal antibody, the method comprising:

(i) immortalising a population of cells comprising or consisting of B lymphycytes in the presence of a product or composition as described herein.

Immortalisation of B-cells can be achieved by various means such as for example (but not limited to) transforming with a suitable virus, fusion with another immortalised cell, or ligation with CD40.

The method may further comprise (ii) screening the transformed lymphocytes for antigen specificity. The method may comprise (iii) isolating the immortalized B cell.

The B cell may be a human B cell. The B lymphocyte may be a memory B lymphocyte, preferably a human B lymphocyte.

The methods described above may further comprise the step of isolating monoclonal antibodies from said immortalised B cell. Disclosed herein are antibodies obtainable by the methods described herein.

Disclosed herein is a method for accelerating the production of antigen-specific antibodies, the method comprising introducing a product or composition as described herein into a non-human mammal, and recovering antibodies from said mammal.

Disclosed herein is a method for inhibiting non-specific uptake of an immunostimulant by a B cell, the method comprising the step of attaching said immunostimulant to a support prior to contact with said B cell.

Disclosed herein is a method of inducing or augmenting an antigen-specific immune response to a BCR-binding antigen in a subject, the method comprising administering to the subject a product comprising a support and said antigen attached to the support, and wherein an immunostimulant is either attached to the support or administered conjointly with said product, and wherein the antigen in soluble form is not capable of inducing a $T_H$ cell response when administered to a subject. The antigen may comprise or may be a carbohydrate, or a peptide. Furthermore, the antigens may comprise or may be modified peptides such as phosphorylated peptides of gylcosylated peptides.

Disclosed herein is a method of delivering an agent, such as an immunostimulant, preferentially to dendritic cells versus B cells, the method comprising

(i) attaching the agent to a support,
(ii) contacting a population of cells with the agent attached to the support, wherein the population of cells comprises B cells and dendritic cells.

The agent may be an immunostimulant as described herein. The support may be as described herein.

## BRIEF DESCRIPTION OF THE FIGURES and tables

**Fig 1. B cells internalize particulate conjugates through the BCR and present aGalCer to NKT cells.**

(A) *Top panel,* Silica beads were coated with DOPC/PE-biotin/αGalCer liposomes (αGalCer), DOPC/PE-biotin/αGalCer liposomes and biotinylated HEL (αGalCer+HEL) or DOPC/PE-biotin liposomes and biotinylated HEL (HEL). The binding of the antigen to liposome-coated beads was detected by western blot with anti-HEL antibody. *Bottom panel,* Alexa-488 labelled liposome coated-beads were detected by FACS analysis (black line) in comparison with non-labelled liposome coated beads (grey solid profile).

(B-H) Presentation of particulate αGalCer by B cells. (B) MD4 B cells were incubated with soluble αGalCer (15ng/ml) or 0.1μl of αGalCer containing particles. Cells were washed and incubated with DN32.D3 cells. αGalCer presentation to iNKT cells was measured by ELISA as IL2 production. (C) MD4 B cells were pulsed as in (B) with particles containing αGalCer (O), HEL (▲) or HEL-αGalCer (■). WT B cells (Δ) were pulsed with HEL-αGalCer particles and used as control. (D) MD4 B cells were primed with HEL-αGalCer particles and incubated for 2 h with an anti-CD1d blocking antibody (αCD1d) or an isotype control (control Ig) before coculture with NKT cells. (E) Particles containing αGalCer and different densities of HEL were analysed by FACS after staining with an anti-HEL antibody. Mean fluorescence intensity values (mfi) are shown. (F) Coculture experiments were performed with MD4 B cells pulsed with particles containing αGalCer and different densities of HEL, $HEL^{RDGN}$ or $HEL^{RKD}$ (gray scale). (G) Presentation of HEL-αGalCer conjugates by B cells expressing D1.3 IgM HEL-specific BCR (■) or a D1.3 IgM/H2 chimera (O). (H) Coculture experiments performed with MD4 B cells pulsed with particles containing HEL-αGalCer (■), αGalCer (▲), HEL-Gal(α1→2)GalCer (O) or Gal(α1→2)GalCer (Δ). (I) Coculture experiments performed with MD4 B cells pulsed with particles containing HEL-IMM47 (■) and particles containing I-MM47 alone (◇)

**Fig 2. iNKT cells help antigen-dependent B cell proliferation in vivo**

CFSE labelled MD4 B cells were transferred into WT mice challenged with particles containing αGalCer and/or HEL. Mice received HEL-αGalCer particles but not cells were used as control (mock). On day 5 spleens from recipient mice were harvested for FACS analysis. (A) Proliferation of donor HEL-binding cells was detected as CFSE dilution. (B and D) Transfer experiments were also performed using Jα18-/- mice as recipients. Percentage of $HEL^+$ (B) and $CD138^+HEL^+$ cells (D) recovered from recipient Jα18-/- (▩) or WT (■) spleens are depicted. (E) The number of HEL-specific IgM secreting cells in the spleen of WT recipient mice was determined by ELISPOT. (F) Specific anti-HEL IgMa was measured on day 5 in the sera of WT and Jα18-/- recipient mice. Specific antibodies were just detected in WT recipient mice challenged with HEL-αGalCer particles (■). (C) After MD4 transfer spleens from recipient mice were harvested at day 5 for immunofluorescence, microscopy. B cells in the follicle (Fo) are stained with B220 (red), HEL-binding cells were stained for intracellular-HEL (green) and germinal centres (GC) were stained with PNA (blue). HEL-binding extrafollicular foci (EF) of plasma cells were only detected in mice challenged with HEL-αGalCer particles.

**Fig 3. Density/affinity of antigen modulates iNKT-dependent B cell proliferation and antibody production.**

MD4 transfer experiments were performed as in Fig 2. WT recipient mice were challenged with particles containing αGalCer and two different densities of HEL or $HEL^{RKD}$. (A) MD4 proliferation was detected as CFSE dilution in the HEL-binding population. (B) Percentage of $HEL^+$ cells recovered from recipient spleens (high density: high, low density: *low*). (C) Anti-HEL specific IgMa was detected on day 5 in the sera of recipient mice challenged with beads containing αGalCer and: ■, HEL high density; □, HEL low density; ●, $HEL^{RKD}$ high density; O, $HEL^{RKD}$ low density.

(D-F) WT mice received MD4 cells and particles containing HEL-αGalCer or HEL particles and soluble αGalCer. Donor cell proliferation was detected as CFSE dilution (D). More extensive proliferation was detected in the $HEL^+$ donor cells in response to HEL-αGalCer conjugates (black line) in comparison with soluble αGalCer (grey dotted line). Mice receiving MD4 cells but no beads were used as control (solid grey profile). (E) Percentage of $HEL^+$ donor cells in mice receiving soluble (▩) or particulate (■) αGalCer-HEL. (F) Anti-HEL specific IgMa detected in recipient mice that received beads containing HEL-αGalCer (■) or HEL beads plus soluble αGalCer (O).

**Fig 4. Immunization with particulates antigen/αGalCer induces IgM and early class switched specific antibodies.**

(A and C) C57BL/6 mice (4 mice/group) were immunized with 10 μl of particles containing αGalCer (□), antigen (▩) or antigen/αGalCer (■). CGG (A) or HEL (C) were used as antigens. Specific antibodies were detected in mice sera at 7 and 14 days after immunization. (B) WT (■) and Jα18-/- (▩) mice (3 mice/group) were immunized with particles containing CGG or CGG/αGalCer and bled on day 7.

**Fig 5. Crosslinking of antigen and αGalCer enhances specific antibody responses.**

(A) C57BL/6 mice (4 mice/group) were immunized

with 1 μl of CGG-αGalCer-coated particles plus 1 μl of OVA-coated particles (▩) or 1 μl of OVA-αGalCer-coated particles plus 1 μl of CGG-coated particles (■). Anti-CGG and anti-OVA specific antibodies were measured on day 7. (B) Specific anti-CGG antibodies detected in C57BL/6 mice (3 mice/group) immunized with 1 μl of particles containing CGG-αGalCer (■) or 1 μl of CGG-particles plus 150 ng of soluble αGalCer (▩). Mice were bled on day 7, and specific anti-CGG antibodies were detected by ELISA.

**Fig 6.** Presentation of particulate αGalCer-HEL by marginal zone (MZ) and follicular (Fo) B cells. MD4 MZ and Fo B cells were sorted by FACS and pulsed with particulate αGalCer-HEL before incubation with DN32.D3 cells. αGalCer presentation was measured as IL-2 production.

**Fig. 7 Antigen affinity and density modulates the response of B cells upon stimulation with particulate antigen-CpG conjugates in vitro**

(A) CFSE labelled MD4 B cells were incubated with microspheres coated with HEL, CpG or HEL and CpG. Left upper panel, Proliferation was detected as CFSE dilution. Left lower panel, plasma cell differentiation was detected as CD138 (Syndecan-1) upregulation. Middle and right panel, IL-6 secretion and HEL specific IgMa production was measured in the supernatant of the cultures by ELISA. (B) MD4 cells were stimulated with microspheres containing the HEL affinity mutants $HEL^{RD}$, $HEL^{KD}$, $HEL^{RKD}$ in presence or absence of CpG. IL-6 and HEL specific IgMa secretion was detected by ELISA. (C) CFSE labelled MD4 B cells were stimulated with particles containing CpG and different densities of $HEL^{RD}$, $HEL^{KD}$, $HEL^{RKD}$. Upper panel, IL-6 and IgMa were measured as above. Black bars represent high density, grey bars intermediate and open bars low density. Lower panel, proliferation was detected as CFSE dilution

**Fig. 8 Particulate HEL-CpG conjugates lead to extensive proliferation and plasma cell differentiation in vivo**

(A) CFSE labelled MD4 B cells were transferred into WT mice challenged with particles containing HEL and/or CpG. On day 4 spleens from recipient mice were harvested for FACS analysis. Upper left panel, Proliferation was detected as CFSE dilution. Lower left panel, MD4 Plasma cells were identified by high intracellular HEL binding and upregulation of the surface marker CD138. Percentage of CD138 positive cells of the MD4 population is represented in the middle panel. Right panel, Detection of HEL specific IgMa in the serum by ELISA

**Fig. 9 Antigen affinity and density modulates the response of B cells upon stimulation with particulate antigen-CpG conjugates in vivo**

(A-B) CFSE labelled MD4 B cells were transferred into WT mice challenged with particles containing $HEL^{RD}$, $HEL^{KD}$, $HEL^{RKD}$ in presence or absence of CpG at high density. (A) Upper panel, proliferation was assessed by dilution of CFSE. Lower panel, MD4 plasma cells were revealed by high intracellular HEL binding and upregulation of CD138. (B-D) MD4 B cells were cotransferred with microspheres containing $HEL^{RD}$, $HEL^{KD}$, $HEL^{RKD}$ in presence or absence of CpG at high (B), intermediate (C), or low density (D). Upper panels represent the percentage of Plasma cells in the MD4 population, lower panels show the IgMa levels in the serum as measured by ELISA.

**Fig. 10 Crosslinking of particulate antigen and CpG enhances specific antibody responses in vivo.**

(A) C57BL/6 mice (3 mice/group) were immunized i.p. with 10μl particles coated either with CGG or CpG or both. CGG specific IgG, IgG1, IgG2b and IgG2c were assessed on day 14. (B) C57BL/6 mice (3 mice/group) were immunized i.p. with, Left panel 1μl of particles coated with CGG plus 1μl coated with OVA-CpG or 1μl particles coated with CGG-CpG plus 1μl coated with OVA. Right panel 10μl of particles coated with OVA plus 10μl coated with CGG-CpG or 10μl particles coated with OVA-CpG plus 10μl coated with CGG were administered to WT mice. CGG and OVA specific IgG were assessed by ELISA on day 14.

**Fig 11 Characterization of HEL and/or CpG containing particulates**

(A) Left panel, Streptavidin beads were coated with biotinylated HEL alone (black line) or in combination with biotinylated CpG (grey solid profile). Right panel, the binding of the antigen to the beads was detected by western blot with polyclonal Anti-HEL antibody. (B) Streptavidin beads were coated with biotinylated monoclonal Anti-HEL F10 antibody and CpG (dark grey solid). Intermediate (ash grey solid) and low desity (light grey solid) of F10 was achieved by competition of F10 with biotinylated CGG.

**Fig 12 Immunization with particulate phosphopeptide-DGalCer induces IgM and IgG peptide specific antibodies.**

C57BL/6 mice (3 mice/group) were immunized with 10 μl of particles containing phospho-peptide (▩) or phospho-peptide - αGalCer (■). Specific peptide antibodies were detected in mice sera at 0 and 7 days after immunization (serum dilution 1:1000).

**Fig 13**

C57BL/6 mice (three mice per group) were immu-

nized once with either 1μl OVA-CpC coated particles with 1μl CγG coated particles or 1μl CyG-CpG particles with 1μl OVA coated particles. ELISPOTs were used to quantify the number of bone marrow CyG-specific IgG secreting ASCs fourteen days and 3 months after immunization.

**Fig 14 BCR-mediated uptake of Ag-CpG conjugates is regulated by the avidity of the Ag-BCR interaction in vitro**

MD4 B cells were stimulated with 1μl fluorescent particulates left either uncoated (filled grey) or coated with HEL, HELK or HELRKD in the (upper panels) absence or (lower panels) presence of CpG. Flow cytometry was used to assess binding of particulates: gates shown indicate the percentage of live cells binding (left gate) intermediate levels and (right gate) high levels of particulates.

**Fig 15 Amount of CpG present on the Ag-containing particulates modulates the extent of B-cell proliferation and differentiation to form PCs in vitro**

(A, B) CFSE-labelled B cells were stimulated with 1μl particulate HEL conjugated with various densities of CpG. The density of CpG is represented from highest to lowest on moving from left to right. Proliferation and differentiation of MD4 B cells were measured 72h after stimulation. (A) Flow cytometry was used to measure CFSE dilution in stimulated (black line) and unstimulated (filled grey) MD 4 cells. (B) (Left panel) IL-6 and (right panel) IgMa secretion were assessed by ELISA.

**Fig 16 Particulate Ag-CpG promotes B-cell proliferation and differentiation to form short-lived EF PCs in vivo**

(A-D) CFSE-labelled MD4 B cells were adoptively transferred into C57BL/6 mice, and challenged with 10μl particulates containing either HELRD alone, CpG alone or HELRD-CpG. (A) Four days after transfer, flow cytometry was used to measure (left upper panels) CFSE dilution and, (left lower panels) CD138 upregulation in HEL binding cells in the spleen of recipient mice; (right upper panel) the percentage of MD4 PCs (HEL intracellularhi, CD138+) present shown as a proportion of total splenocytes; (right lower panel) serum HEL-specific IgMa measured by ELISA (B) Flow cytometry was used to measure CFSE dilution in HEL-binding cells in the spleens of recipient mice in stimulated (black line) and unstimulated (filled grey) MD 4 cells at the indicated times following challenge. (C) (Left panel) ELISPOTs were used to detect splenic HEL-specific ASCs, and (right panel) ELISAs were used to measure serum HEL-specific IgMa. (D) After five days, immunofluorescence microscopy (Zeiss LSM 510 meter) was used to detect HEL-binding cells (intracellular HEL, green Alexa488) and splenic follicular B cells (anti-B220, red Alexa543). Magnification 10x, NA 0.3.

**Fig 17 Avidity of the Ag-BCR interaction and TLR9 signalling strength modulate the extent of PC formation in vivo**

CFSE-labelled MD4 B cells were adoptively transferred into C57BL/6 mice, and challenged with 10μl particulates containing HEL and various densities of CpG. The density of CpG is represented from highest to lowest on moving from left to right. Flow cytometry was used to quantify (upper panel) the percentage of live HEL binding B cells that have undergone proliferation, and (lower panel) the percentage of MD4 PCs (HEL intracellularhi, CD138+) as a proportion of total splenocytes.

**Fig 18**

(E) HyHEL10 B cells were adoptively transferred into C57BL/6 mice and challenged with 10μl particulates coated with HEL alone, CpG alone or HEL-CpG. After seven days ELISAs were used to measure the levels of HEL-specific Igs in the serum of recipient mice. (Left panel) IgM (open bars) and IgG (filled bars) and (right panel) IgG subtypes IgG1 (light grey bar), IgG2b (middle grey bar) and IgG2c (dark grey bars). (F) HyHEL10 B cells were stimulated with 1μl of particulate HEL alone, particulate CpG alone or particulate HEL-CpG for seven days. ELISAs were used to measure the levels of HEL-specific Igs secreted into the culture medium as described in (E).

**Fig 19 Stimulation with HEL-CpG particulates gives rise to sustained p38 phosphorylation and is dependent on a functional TLR9 in vitro** (A) Biotinylated Ags (left panel) OVA and (right panel) CyG were immobilised on streptavidin-coated particles and were visualized by flow cytometry following binding of anti-OVA followed by anti-Mouse IgG-AlexaFluor-488 or anti-CyG FITC in the presence (filled grey) or absence (black line) of CpG. (B) Binding of biotinylated HEL to streptavidin-coated microspheres in the presence (grey filled) or absence (black line) of CpG was detected by: (upper panel) Western blotting using a polyclonal anti-HEL and (lower panel) flow cytometry via binding of anti-HEL F10 AlexaFluor-488. (C) MD4 B cells (upper panel) and bone marrow derived DCs (lower panel) were either not stimulated (filled grey) or stimulated with 0.2 μl particulates that were coated with HEL alone, CpG alone or HEL-CpG (solid line). Flow cytometry was used to assess binding of particulates. Gates indicate percentage of live cells binding to the particulates.

**Fig 20**

MD4 B cells were stimulated with 1 μl particulate

HEL alone (open bars), particulate CpG alone (grey bars) or particulate HEL-CpG (filled bars). Samples were taken at the indicated times after stimulation, and equal amounts of whole cell-lysates were subjected to SDS-PAGE. (Upper panel) Subsequently Western blotting was performed using specific antibodies to phosphorylated p38 (pp38), total p38 and actin (loading control) for detection. (Lower panel) The density of the bands was quantified by densitometry, corrected for background, normalized to the density of the actin band in the same sample, and then made relative to the unstimulated zero time point for each condition.

**Fig 21 Stimulation of B-cell proliferation and differentiation by Ag-CpG particulates is dependent on Ag avidity in vitro**

MD4 B cells were stimulated with 1 μl particulate CpG coated together with either (left panels) HEL or (right panels) $HEL^K$ at (upper panels and filled bars) high or (lower panels and open bars) low density for 72h. (A) CFSE dilution of stimulated (black line) and unstimulated cells (filled grey) B cells was assessed by flow cytometry. (B) (Left panel) IL-6 and (right panel) $IgM^a$ secretion were assessed by ELISA.

## Detailed DESCRIPTION OF THE INVENTION

Binding of antigen to BCR leads to antigen internalization and presentation to T cells, a critical step in the initiation of the humoral immune response. The ability of B cells to internalize and process particulate antigen has been describe by several groups (22-24). This is particularly relevant, as in vivo antigen is often encountered in insoluble form or tethered to a cell surface (23).

The interaction of a protein (P, such as a BCR) with its ligand (L, such as an antigen) to form a complex (PL) can be described by a number of different parameters. As such in biochemistry, the equilibrium dissociation constant can be used to give a measure of the affinity of the interaction between the protein and the ligand.

$$PL \underset{k_{on}}{\overset{k_{off}}{\longleftrightarrow}} P + L$$

The equilibrium dissociation constant ($K_D$) is defined when the rate of the forward and backward reactions is equal such that

$$k_{off}\ [PL] = k_{on}\ [P]\ [L],$$

thus defining the equilibrium dissociation constant as:

$$K_D = k_{off} / k_{on} = [P][L]/[PL].$$

The dissociation constant has molar units (M), which correspond to the concentration of ligand [L] at which the binding site on a particular protein is half occupied, i.e. the concentration of ligand, at which the concentration of protein with ligand bound [PL] equals the concentration of protein with no ligand bound [P]. The smaller the dissociation constant, the more tightly bound the ligand is, or the higher the affinity between ligand and protein. For example, a ligand with a nanomolar (nM) dissociation constant binds more tightly to a particular protein than a ligand with a micromolar (μM) dissociation constant. Alternatively the affinity is commonly described by the association constant ($K_A$, given in $M^{-1}$) which is given by the inverse of the $K_D$.

In addition, the lifetime of the PL complex is described in terms of the half-life of the complex and as such:

$$t_{1/2} = \ln 2/k_{off}$$

A longer $t_{1/2}$ represents a more stable interaction, and results from a slower rate constant $k_{off}$. The particular lifetime of the complex will determine the stimulation/activation of the protein in question if the ligand is an agonist. This is important quantity in biochemistry as reactions are not simply dependent therefore on the 'affinity' of the interaction ($K_A$ or $K_D$) but rather on the avidity of the ligand seen by the protein.

While the term affinity describes the strength of a single bond, avidity is the term used to describe the combined strength of multiple bond interactions.

As a ligand can be present at different densities on a surface this can affect the likelihood of re-binding following collapse of a PL complex of short life-time. Thus, at higher densities ligands with rapid $k_{off}$ may be able to induce similar responses to ligands with high affinity.

The overall avidity of the particulate antigen to the target cell, typically a B cell, is thus dependent both on the affinity of the specific antigen and on the density of the antigen on the surface of the support.

The inventors have found that the specific binding of particulate antigen to BCR and subsequent internalization is dependent on the overall antigen avidity. Use of a support, such as a bead, allows fine regulation of the antigen density (and thereby avidity). This therefore also ensures that even antigens of low "affinity" in terms of the $K_A$ may be able to stimulate specific immune responses if they are presented on a surface at sufficient density. An immunostimulant, also attached to the support, is internalized by the cell together with the antigen, leading to an enhancement of the specific immune response. The enhancement observed can result from the

intracellular stimulation of activation or through the recruitment of other cellular factors that can lead to stimulation of activation.

As discussed above, the density of the antigen on the support also influences the avidity.

US2007/0104776 (Ishii et al) describes the use of liposomes containing ovalbumin and a-Galactosyl Ceramide, where the ovalbumin is encapsulated in the liposome. The liposomes show an inhibitory effect on antibody production.

Disclosed herein is a product capable of BCR-mediated internalization comprising

(i) a support, and
(ii) a BCR-binding antigen attached to the support.

In other words, the product is suitable to be internalized by a target cell, typically a B cell, via BCR-engagement. The product may further comprise an immunostimulant attached to the support. Upon BCR-mediated internalization the product elicits an antigen-specific immune response.

Any suitable support may be used. The support must be suitable for attaching an antigen and/or an immunostimulant thereto and must be of a suitable size to be internalized and processed by a BCR-expressing cell. The support may be a particle, for example a bead. The support may be a microsphere. The term "microsphere' refers to a spherical shell made of any material that has a very small diameter, usually in the micron or nanometer range.

The support may be of any suitable material, for example, polymer or silica supports may be used, such as polymer or silica beads or microspheres. Suitable beads are known in the art.

The particle may be a liposome. Liposomes are generally understood to be vesicle structures made up of one or more lipid bilayers surrounding an aqueous core. Each lipid bilayer is composed of two lipid monolayers, each of which has a hydrophobic "tail" region and a hydrophilic "head" region. In the bilayer, the hydrophobic "tails" of the lipid monolayers orient toward the inside of the bilayer, while the hydrophilic "heads" orient toward the outside of the bilayer. Methods for making liposome preparations are described by Bangham (Bangham et al, 1965, J Mol Biol, 13:238), and compositions and methods for stabilizing liposome suspensions are described in WO2006/002642.

Beads coated with liposome may be used in accordance with the invention.

The support/particle may be in the size of between 1nm and 10$\mu$m, preferably in the range of 10 nm to 10$\mu$m, preferably in the range of 10 nm to 1$\mu$m, preferably in the range of 50 nm to 1$\mu$m, preferably in the range of 100nm to 1$\mu$m, more preferably in the range of 100nm to 500nm. More preferably the size of the support is in the range of 100nm to 150 nm, more preferably in the range of 100nm to 130nm. However, the support/particle may be smaller or larger.

The particle may be 100 nm or 130 nm in size.

In some embodiments, it may be preferable that the size of the particle resembles the size of a natural antigen-carrier, such as a pathogen.

Any method of attaching the immunostimulant and/or the antigen to the support may be employed as long as the immunostimulant and the antigen are still capable of eliciting the desired enhanced and specific immune response. For example, a biotin-streptavidin linker system may be used. In addition, liposomes may be used in order to coat silica microspheres with particular immunostimulant lipids.

The products and methods of the present invention allow fine regulation of the antigen amount and antigen density on the support. Depending on the particular antigen used, more or less antigen may be attached to the support surface, thereby increasing or decreasing the antigen density (and therefore avidity). Different antigens may require different densities, dependent on their different $K_A$ and $k_{off}$ in order to exceed the required avidity threshold for BCR-mediated internalization. For example, a low affinity antigen may require a higher density on the support surface compared to a high affinity antigen.

For example, the present inventors have utilised antigen with a range of affinities covering those that would be contained within the physiological repertoire prior to immunisation with antigen ($K_A$ in the range of 8 x $10^5$ $M^{-1}$ to 2.1 x $10^{10}$ $M^{-1}$). With respect to the tested antigens, a minimum $K_A$ 8 x $10^5$ $M^{-1}$ was required (with corresponding $k_{off}$ of 2.5 $sec^{-1}$) to stimulate specific antibody production from transferred antigen-specific B cells in vivo.

It is emphasized that any affinity values described herein are merely for reasons of illustration and are in no way limiting the scope of the present invention. Any antigen suitable to induce a BCR-mediated internalization may be used in accordance with the invention. The invention is not limited to antigens with a particular low or high affinity.

Methods of measuring the amount of antigen on the support surface and adjusting therefore its density are known in the art. For example, the precise amount of antigen associated with the beads, and thus its density, can be quantified using quantitative Western blotting or through the incorporation of a fluorescent label into the antigen to determine the amount of antigen present by FACS.

Disclosed herein is a product comprising (i) a support, (ii) at least one BCR-binding antigen attached to the support, and,optionally, (iii) at least one immunostimulant linked to the support.

It is understood that the products of the present invention comprise a support with antigen present at a sufficient density (and thus avidity) to allow BCR-mediated internalization by a target cell, typically a B cell. They are therefore suitable for BCR-mediated internalization.

The necessary density of a particular antigen required to obtain an overall avidity sufficient for BCR-mediated internalization can for example be determined by (i) generating supports with different amounts of antigen attached thereto, (ii)contacting said supports with a target cell, such as a B cell, (iii) testing for target cell activation. The activation of the target cell indicates specific uptake of the particulate antigen, which indicates that the antigen on that particular support has a sufficient density and avidity to trigger internalization in accordance with the invention.

In order to generate different density levels on the support, one may, for example, first generate supports with the highest possible density by saturating the support with antigen. One may then test whether the obtained support is capable of inducing BCR-mediated internalisation. The density may then be reduced by contacting the antigen-coated support with different amounts of an agent that competes out the antigen bound on the surface of the support. For example, if the antigen is attached to the support via a biotin-based attachment mechanism, the competing agent may be a biotinylated agent, for example a biotinylated non-immunogenic protein. The generated supports may then be contacted with a B cell to determine whether the obtained density of the particular antigen is suitable for BCR-mediated B cell activation. If the avidity falls below the required threshold for a particular antigen, no sufficient cell activation, and thus no sufficient immune response, is observed. By stepwise reducing the density on the supports, one can determine the minimum density (and thus avidity) required for each antigen.

If multiple antigens are present on the support, the density of each antigen can be optimized.

Activation of the B cell may be measured by any suitable method. For example, activation of the B cell may be tested by measuring the secretion of specific antibodies, cytokines or chemokines in response to the uptake of particulate antigen. One may also measure the activation of CD1d-mediated lipid immunostimulant presentation on the surface of B cells by measurement of the secretion of IL-2 into the culture medium following in vitro incubation with iNKT cells.

In particular the following tests may be employed, to test whether antigen is present at sufficient avidity on the support to elicit an antigen-specific immune response. One may employ the following in vitro assays in transgenic antigen-specific mouse B cells. (The cells are transgenic' in that they are enriched for B cells expressing BCR specific for the antigen in question.)

For example, if supports with antigen and CpG are used, IL-6 secretion in the supernatant of the cultures can be measured by ELISA as early as 24 or 48 hours after stimulation with the particulates. Production of IgM can be assessed after 72h hours by ELISA. Both IL-6 and IgM can only be detected if sufficient stimulation has taken place.

Furthermore if B cells are labelled with CFSE before stimulation, proliferation (dilution of CFSE) as a readout of B cell activation can be assessed 72h after stimulation by FACS.

If, for example, a support with antigen and aGalcer is used, it is possible to assay the amount of aGalCer-CD1d presented on the surface of B cells, by examination of the ability of these cells to activate iNKT cells. As detailed in the methods and material of the Examples herein, one may use iNKT cells derived from the DN32.D3 hybridoma for these assays. In order to present aGalCer-CD1d on their surface the antigen on the support must have exceeded the avidity threshold for BCR-mediated internalisation. Antigen-specific transgenic B cells are incubated with the particles overnight and then washed thoroughly in PBS. The B cells are subsequently incubated with iNKT cells, and after 20h the secretion of IL-2 by iNKT cells as measured by ELISA indicates gives a measure of aGalCer-CD1d presentation on the B cell surface.

Multiple supports may be taken up by each B cell. The number of supports contacting the B cell maythus influence the resulting immune response. One may optimise the number of supports to achieve a desired response by varying the number of supports used.

One may also employ an immunisation strategy to identify the threshold of antigen avidity required for stimulation of specific immune responses in vivo. For example, the production of specific antibodies may be measured by carrying out ELISAs or other suitable immunological assays on the serum of individuals immunized with the particulate antigen described herein.

Use of the support not only allows one to modulate the density of the antigen and/or immunostimulant on the support, but also the ratio between the antigen and the immunostimulant. This represents a further way to optimise the specific immune response to a particular antigen. As weaker antigens, such as used in many antibacterial vaccines, lack associated T cell epitopes, such antigens may require a higher amount of immunostimulant. The support affords the opportunity to increase the amount of conjugated immunostimulant compared to what would be required for stronger antigens. Indeed the inventors have demonstrated that enhanced and specific immune responses may even occur in the absence of specific T cell help, using for example immunization with the model antigen hen egg lysozyme in the C57/BL6 background. Presenting the antigen and the immunostimulant on the same support ensures the simultaneous uptake by the cell, leading to an even more enhanced specific response compared to uptake of the antigen alone or uptake of antigen with soluble immunostimulant.

In some embodiment, multiple antigen molecules may be attached to the support.

In some embodiments, different types of antigens may be attached to the support; for example 2, 3, 4 or more different antigens may be attached to the support. Typically each antigen is present in a sufficient density/avidity to activate BCR-expressing cells, i.e. each

antigen will trigger an antigen-specific immune response. A support with multiple antigens will thus stimulate immune responses to each antigen, and would thus allow for the production of immune responses tailored to the antigenic composition of the pathogen. This strategy would ensure optimal protection from an invading pathogen, preventing the selection of single 'escape' mutations. (Often viruses will mutate some of their proteins (and thus antigens) to escape detection by the immune system, so that they can no longer be bound by immune cells or recognised by a specific receptor). Thus, attaching different antigens to the support and raising antibodies to a number of antigens at the same time, increases the chance that a single escape mutant will still be fought off by the immune system.

In some embodiments, multiple immunostimulant molecules may be attached to the support.

In some embodiments, different types of immunostimulants may be attached to the support. For example, 2, 3, 4 or more different types of immunostimulants may be attached to the support. Since different types of immunostimulants may use different mechanisms of enhancing the immune response, combining different immunostimulants may lead to cooperation yielding an even more strongly enhanced immune response.

In some embodiments, a combination of different types of antigens and different types of immunostimulants may be present on the support surface.

Further, in some embodiments, a support with one or more antigens and one or more immunostimulants attached thereto, may be combined with one or more soluble immunostimulant to further boost the specific immune response. The soluble immunostimulant(s) may be the same immunostimulant used on the support, or may be a different one(s).

In some embodiments, the support with one or more types of antigens attached thereto, and optionally one or more types of immunostimulants attached thereto, may be administered conjointly with one ore more soluble immunostimulant.

Immunostimulants

The term "immunostimulant" is used herein to describe a substance which evokes, increases and/or prolongs an immune response to an antigen. While the present application distinguishes between an "antigen" and an "immunostimulant" it should be noted that this is merely for reasons of clarity and ease of description. It should be understood that the immunostimulant could have, and in many cases preferably has, antigenic potential itself. Thus, in the examples, the "immunostimulatory lipid", for example, is thus also referred to as the "antigenic lipid". The distinction between "antigen" and "immunostimulant" herein rather refers to the fact that the induced specific BCR-mediated immune response will be directed towards the antigen, as the "antigen" determines the specific binding to the BCR.

Compounds with immunostimulatory activity are known in the art. Any suitable immunostimulant may be used in accordance with the invention, such as for example a protein, polypeptide, peptide, polysaccharide such as a glycan, polysaccharide conjugates, peptide and non-peptide mimics of polysaccharides and other molecules, small molecules, lipids, glycolipids, and carbohydrates.

In some embodiments, the immunostimulant is an iNKT agonist or a TLR agonist, as described in more detail below.

iNKT agonists

For many years, peptides were assumed to be the only antigenic determinant initiating T cell responses. However, it is now evident that T cells are also able to recognize and respond to antigenic lipids and glycolipids, presented by CD1 molecules (8). T cells recognise a diverse range of potential antigens through their highly polymorphic T cell receptor (TCR). A subset of T cells known as iNKT (invariant natural killer T) cells are defined by their expression of a restricted TCR repertoire, consisting of a canonical V$\alpha$14-J$\alpha$18 or V$\alpha$24-J$\alpha$18 $\alpha$ chain in mice and humans respectively. iNKT cells recognise and become activated in response to self or foreign antigenic lipids presented by non-polymorphic CD1d molecules expressed on the surface of APCs (8,11). iNKT cells are activated in response to a variety of infections, and during inflammatory and autoimmune diseases (12,13). iNKT cells provide a means of linking and co-ordinating innate and adaptive immune responses, as their stimulation can induce the downstream activation of DCs, NK cells, B and T cells (11). It has been demonstrated in vitro that iNKT cells stimulate B cell proliferation and antibody production (16), though this appears independent of the presentation of exogenous iNKT cell-ligands and BCR specificity. As the effective operation of the immune system requires the tightly regulated control of B cell activation in vivo, it would be expected that iNKT-mediated activation must be subject to stringent regulation however the mechanism by which this occurs remains uncharacterised. In the present application the inventors demonstrate that specific BCR internalization enhances B cell presentation of particulate lipid antigens to iNKT cells in vivo. Subsequently activated iNKT cells help specific B cell proliferation, differentiation to extrafollicular PCs and secretion of high titres of specific IgM and early class-switched antibodies.

It has been previously shown that NKT cells can be activated by $\alpha$-galactosyl-ceramide ($\alpha$-GalCer) or its synthetic analog KRN 7000 (US 2003/0157135). It has further been shown that $\alpha$-GalCer can stimulate NK activity and cytokine production by NKT cells and exhibits potent antitumor activity in vivo. As the immunoregulatory functions of $\alpha$-GalCer are absent in both CD1d$^{-/-}$ and NKT-deficient mice, this indicates that $\alpha$-GalCer has to be presented by the MHC class I-like molecule CD1d

(US 2003/0157135).

US2003/0157135 further states that α-GalCer and related glycosylceramides not only function as antigens but can also be employed as soluble adjuvants capable of enhancing and/or extending the duration of the protective immune responses induced by other antigens.

Thus, in some embodiments of the present invention the immunostimulant may be an iNKT cell agonist. The agonist may be an exogenous or endogenous agonist. It may be a glycosidic agonist (such as alpha-galactasylceramide).or a non-glycosidic agonist (such as threitolceramide).

In some embodiments, the immunostimulant may be a lipid or a glycolipid. Glycolipids presented by CD1 can be grouped into different classes including for example diacylglycerolipids, sphingolipids, mycolates and phosphomycoketides (Zajonc and Krenenberg, Current Opinion in Structural Biology, 2007, 17:521-529). Microbial antigens from pathogenic mycobacteria, such as glucose monomycolates (GMM), mannosyl phosphomycoketides and phosphatidylinositol mannosides are known to be potent ligands for human T cells when presented by group I CD1 molecules (Zajonc an Kronenberg, supra). The immunostimulant of the present invention may be a glycosylceramide, for example alpha-galactosylceramide (KRN 7000, US2003/0157135) or an analogue thereof, such as for example threitolceramide (IMM47) or other non-glycosidic iNKT cell agonists (as described in Silk et al. Cutting Edge J. Immunol, 2008). Further analogues which may be used in accordance with the invention and methods of producing such analogues are disclosed in WO2007/050668. For example, analogues useful in accordance with the invention include compounds having formula I

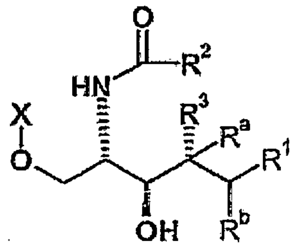

I

in which

$R^1$ represents a hydrophobic moiety adapted to occupy the C' channel of human CD1d, $R^2$ represents a hydrophobic moiety adapted to occupy the A' channel of human CD1d, such that R1 fills at least 30% of the occupied volume of the C' channel compared to the volume occupied by the terminal $nC_{14}H_{29}$ of the sphingosine chain of alpha-galactosylceramide when bound to human CD1d and R2 fills at least 30% of the occupied volume of the A' channel compared to the volume occupied by the terminal $nC_{25}H_{51}$ of the acyl chain of alpha-galactosylceramide when bound to human CD1d,

R3 represents hydrogen or OH,

Ra and Rb each represent hydrogen and in addition, when R3 represents hydrogen, Ra and Rb together may form a single bond,

X represents or -CHA(CHOH)nY or -P(=O)(O-)OCH2(CHOH)mY, in which Y represents CHB1B2,

n represents an integer from 1 to 4, m represents 0 or 1,

A represents hydrogen,

one of B1 and B2 represents H, OH or phenyl, and the other represents hydrogen or one of B1 and B2 represents hydroxyl and the other represents phenyl,

In addition, when n represents 4, then A together with one of B1 and B2 together forms a single bond and the other of B1 and B2 represents H, OH, or $OSO_3H$ and pharmaceutically acceptable salts thereof.

The immunostimulant may be arabinitol-ceramide, glycerol-ceramide, 6-Deoxy and 6-Sulfono-myo-insitolceramide.

The inventors show that BCR-mediated uptake allows efficient presentation of particulate immunostimulatory lipids to iNKT cells. This mechanism permits CD1-mediated presentation of lipids following BCR recognition of even low affinity antigen, so long as a defined avidity threshold is surpassed. As a result, activated iNKT cells provide help for specific B cell proliferation, extrafollicular plasma B cell differentiation and production of high titres of specific IgM and early class-switched antibodies.

The inventors have utilized the BCR both as a means of achieving the selectivity in delivery and efficient internalization of particulate antigenic lipids by specific B cells.

The ability of iNKT cells to induce B cell proliferation has been characterised predominantly in vitro (16). The inventors show that even in the absence of specific CD4 T cells, iNKT cells can help B cell proliferation and antibody production in vivo. These observations are in line with previous investigations where immunization of MHC II-deficient mice with antigen and αGalCer induced detectable levels of IgG, indicating that iNKT cells can replace CD4 T helper functions (27). Thus this has implications for the stimulation of immune responses of weaker antigens such as those often utilised for antibacterial vaccines that lack associated T cell epitopes. iNKT cells have an antigen-experienced phenotype and they can respond very rapidly to CD1d-presented antigens without the need for clonal expansion. The inventors have demonstrated that direct iNKT cell help after BCR engagement leads to extrafollicular PC differentiation and early antibody production. Extrafollicular PCs are responsible for the generation of the initial wave of antibodies in a T-dependent response (5). These antibodies can have neutralizing effects for the protection

against virus and bacteria. This suggests a main role for direct iNKT cell help to B cells in the induction of early immune responses against different pathogens. In line with this, iNKT-deficient mice exhibit severe defects in the clearance of several microorganisms including *Streptococcus pneumoniae, Sphingomonas* ssp., and *Plasmodium berghei* (28).iNKT cells have been proposed as players in the development of early antibody responses following infection with *Plasmodium berghei* in the model for cerebral malaria (29). In this case, specific antibody production in CD1-/- mice is particularly reduced at early time points following parasitic challenge.

Thus, iNKTs can play a critical role in shaping early antibody responses in vivo and thus offer enhanced protection from invading pathogen.

Thus, what is the functional significance of the observations described herein in the context of an immune response? iNKT cells are activated by glycolipids from LPS-negative bacteria like *Sphingomonas, Erlichia* and *Borrelia* (25) (26) (30). Alternatively, iNKT cells can recognize a still undefined endogenous lipidic ligand, via CD1d presentation, up-regulated by DCs in response to TLR signalling (25) (31) (32) (33). While all B cells are known to express CD1d, this expression is enhanced in marginal zone B cells, such that they present a $CD21^{high}$ $CD23^{low}$ $CD1d^{high}$ phenotype. This phenotype suggests that marginal zone B cells may play an important role in CD1d-dependent iNKT activation following infection. Indeed these cells are localised in the marginal sinus of the spleen where they can provide early immune responses to blood-borne particulate antigen (34) (35). We postulate that marginal zone B cells expressing BCR specific for bacterial glycolipids allow for the more efficient recruitment of iNKT cell help and associated generation of specific antibody responses. Alternatively, B cells capable of internalizing particulate microbes may receive TLR signals and subsequent iNKT cell help after the upregulation of a CD1d-restricted endogenous ligand.

The results presented herein identify BCR internalization of particulate antigen and immunostimulatory lipids as a means of modulating iNKT-mediated B cell responses in vivo. The collaboration between B and iNKT cells leads to the development of early specific antibody responses, emphasising the importance of iNKT cells in coordinating innate and adaptive immune responses.

TLR agonists

Intracellular TLRs such as TLRs 3, 7, 8 and 9 recognise nucleic acids. As such synthetic oligodeoxynucleotides (ODN) such as the TLR9 agonist CpG have previously been used as immunostimulants (Klinman, 2004, Nature Reviews, 4:249). These TLR immunostimulants operate by a different mechanism than that employed by lipids such as αGalCer. These immunostimulants directly activate the cell that they are taken up by, culminating in, for example, the secretion of cytokines and chemokines that result in the further stimulation of immune responses.

The TLR expression pattern is specific for each cell type (Chiron et al, 2009). TLR expression in human B cells is characterized by high expression of TLR 1, 6, 7, 9 and 10, with the expression pattern varying during B-cell differentiation.

Soluble CpG ODNs are rapidly internalized by immune cells and interact with TLR9 that is present in endocytic vesicles. Cellular activation by most members of the TLR family (including TLR9) involves a signalling cascade that proceeds through myeloid differentiation primary response gene 88 (MYD88), interleukin-1 (IL-1), receptor-activated kinase (IRAK) and tumour-necrosis factor receptor (TNFR)-associated factor 6 (TRAF6), and culminates in the activation of several transcription factors, including nuclear factor -Kb (NF-κB), activating protein 1 (AP1), CCAAT-enhancer binding protein (CEBP) and cAMP-responsive element binding protein (CREB). These transcription factors directly upregulate cytokine/chemokine gene expression. B cells and plasmacytoid dendritic cells (pDCs) are the main human cell types that express TLR9 and respond directly to CpG stimulation. Activation of these cells by CpG DNA initiates an immunostimulatory cascade that culminates in the indirect maturation, differentiation and proliferation of natural killer (NK) cells, T cells and monocytes/macrophages. Together, these cells secrete cytokines and chemokines that create a pro-inflammatory (IL-1, IL-6, IL-18 and TNF) and $T_H1$-biased (interferon-γ, IFN-γ, and IL-12) immune milieu (Klinman, 2004, Nature Reviews, 4:249).

Thus, in some embodiments the immunostimulant is a TRL agonist. For example, it is an endosomal TLR agonist, in particular a nucleic acid, such as for example DNA, RNA (either double or single stranded). The immunostimulant may, for example comprise a CpG oligodeoxynucleotide or a poly-U nucleic acid.

**Generation of immortalised cells**

The method for antigen-specific delivery of immunostimulants, as described herein may be employed in the generation of immortalised B cells and B cell lines for the production of antigen-specific monoclonal antibodies.

Disclosed herein is a method for generating an immortalized cell, said method comprising the steps of

(i) contacting a B cell with a product or composition as described herein, and
(ii) immortalising the B cell of step (i).

Step (ii) above may be performed after step (i), or both steps may be performed at the same time.

Step (i) may be performed ex vivo, i.e. outside the organism from which the B cell originates.

Immortalisation of B-cells can be achieved by any suitable method, such such as for example (but not

limited to) transformation with a suitable virus, and/or fusion with another immortalised cell and/or ligation with CD40.

Methods for immortalising B cells with a transforming virus and as well as suitable viruses are known in the art. (Lanzavecchia et al, Curr Opin Biotechnol, 2007 Dec;18(6):523-8, Epub 2007 Dec 11). Any suitable transforming virus may be used. The virus may for example be Epstein-Barr virus (EBV). EBV is suitable for transforming the B cells of most primates, but for other organisms suitable viruses can be selected if necessary. For example, following delivery of a support coated with antigen and for example a TLR agonist, infection by Epstein Barr virus transformation would be greatly enhanced only in antigen-specific B cells that had received TLR stimulation (using the method described in Traggiai E et al (Traggiai E, Becker S, Subbarao K, Kolesnikova L, Uematsu Y, Gismondo MR, Murphy BR, Rappuoli R, Lanzavecchia A. Nature Medicine 2004 vol. 10 pp. 871-5). The efficiency of transformation is dramatically increased in the TLR activated cells compared to non-activated B cells. Thus the overall proportion of antigen-specific cells amongst those that are transformed into immortalised cell lines is significantly increased, i.e. the transformed population is specifically enriched with antigen-specific B cells. Thus, disclosed herein is a method for the rapid production of immortalised B cell lines specific for the particular antigen required, without laborious screening procedures following EBV transformation.

Immortalisation can for example be achieved by fusion with another immortalised cell. Suitable methods and materials are known to the skilled person. For example, Shirahata et al, Methods Cell Biol, 1998; 57:111-45, describe such methods. (In Shirahata et al NAT-30 and HO-323, human parent cell lines with high fusion efficiencies, were established to prepare many hybridoma cell lines producing cancer-specific human monoclonal antibodies. Because NAT-30 and HO-323 cell lines are IgM producers, it is difficult to obtain IgG-producing hybridomas because the types of immunoglobulin produced by hybridomas are strongly affected by the characteristics of parent cells. Thus a nonimmunoglobulin-producing human parent cell line, A4H12, derived from human T lymphoma was established that can efficiently obtain IgG-producing human hybridomas. Another problem with preparing human hybridomas is that it is difficult to obtain B lymphocytes immunized with optional antigens for ethical reasons. To overcome this problem, the authors describe in vitro immunization methods that have been developed to allow exposure of a large number of B lymphocytes to cultured cancer cell or soluble antigens. The authors discuss human fusion partners, in vitro immunization methods, and the preparation of human-human hybridomas using an electrofusion method.)

Immortalisation can for example be achieved by stimulation of CD40, (CD40 ligation) as for example reported by Wiesner et al, PLoS ONE, Jan 2008, issue 1:1-13. (The authors show that human B lymphocytes can be activated and induced to proliferate in vitro by triggering their surface receptor CD40 in the presence of interleukin-4, a combination of signals mimicking B cell activation by T helper cells. To establish EBV-free CD40-stimulated B cell cultures, unseparated peripheral blood mononuclear cells (PBMC) from EBV-positive donors were plated in different numbers per microculture on CD40L-expressing stimulator cells in the presence of interleukin-4 and cyclosporin A. Cells were restimulated every 5 to 7 days with fresh stimulator cells and expanded when outgrowth became prominent. The authors observed that rapid outgrowth of B cells was favoured if small initial cell numbers per culture were used ($2\times10^4$ to $5\times10^5$ PBMC). After 27 days, cultures from five donors set up with $10^5$ PBMC had proliferated on average 282-fold with respect to B cells and were dominated by B cells ($87\pm5\%$ CD19+ cells).)

The immortalized cell may then be taken into culture to establish an immortalized cell line, using methods known in the art

While the methods described above are suitable to be used in any species, they are particularly useful for the generation of monoclonal human antibodies. Common methods for generating human or human-like monoclonal antibodies include for example the humanization of murine antibodies, the isolation of antibodies from libraries of different complexity and the production of hybridomas using the classic method in mice transgenic for human Ig Loci (the "xeno-mouse"). However, humanization of murine monoclonal antibodies is a laborious and imcomplete procedure. Random antibody libraries represent an unbiased repertoire and can therefore be used to select antibody specificities against highly conserved antigens, but lead to antibodies of low affinity. Libraries selected from antigen primed B cells are enriched for a particular specificity, but do not preserve the original VH-VL pairing and generally lead to antibodies that have a lower affinity for the antigen than those present in the original antibody repertoire. The xeno-mouse can be immunized against an antigen of choice, but this system shares with the classical murine hybridoma technology the limitation that the antibodies are selected in a species other than human.

The B cell in step (i) may be a naive B cell, i.e. the B cell has not been contacted with the antigen of interest before. Thus, disclosed herein are improved methods for the *de novo* generation of antigen specific antibodies.

The B cell may be a human B cell.

Disclosed herein is a method for producing immortalised B lymphocytes, the method comprising the step of (i) immortalising B lymphocytes ex vivo in the presence of a product or composition as described herein, i.e. a product capable of BCR-mediated internalization comprising a support and a BCR binding antigen attached to the support; or a composition comprising the product and a suitable carrier.

Immortalisation may be achieved by any suitable method, as discussed above.

Disclosed herein is provided a method for producing a clone of an immortalized B lymphocyte capable of producing a monoclonal antibody with a desired antigen specificity, the method comprising:

(i) contacting a B lymphocyte ex vivo with a product or composition as described herein, and
(ii) immortalising the B cell of step (i).

Immortalisation may be achieved by any suitable method, for example the methods discussed above.

The method may further comprise screening the transformed lymphocytes for antigen specificity. The method may further comprise isolating an immortalized B lympohocyte

Steps (i) and (ii) may be performed one after the other, or simultaneously.

Disclosed herein is a method for producing a clone of an immortalized B lymphocyte capable of producing a human monoclonal antibody with a desired antigen specificity, the method comprising:

(i) immortalising a population of cells comprising or consisting of B lymphycytes in the presence of a product or composition as described herein.

The method may further comprise the step of

(ii) screening the transformed lymphocytes for antigen specificity.

The method may further comprise the step of (iii) isolating an immortalized B lympohocyte.

In the methods described above, the B lymphocytes can be undifferentiated or naive B lymphocytes or memory B lymphocytes or a mixture of both. While the B cell may be of any origin, such as mammalian (or non-human mammalian), it preferably is a human B cell.

The B cell to be immortalised may be a memory B lymphocyte obtained from an organism that has been previously in contact with an antigen of interest. The products or compositions as described herein may thus be used to re-activate memory B lymphocytes. The memory B lymphocyte may be human. WO2004/076677, describes a method of producing immortalised human B memory lymphocytes, comprising the step of transforming human B memory lymphocytes using a transforming virus in the presence of a polyclonal B cell activator. Polyclonal B cell activators (such as CpG sequences) were found to enhance the efficiency of EBV immortalization and of cloning EBV-immortalized cells.

Li et al (Li et al, PNAS March 7, 2006, vol 103:3557-3562) describes a process employing primary B cells for generating cell lines producing specific antibodies. The process requires exposing the mixture of primary B cells and T cells with said antigen ex vivo for generation of immortalised B cells secreting specific antibody. The methods described above can be carried out in the absence of T cells.

The methods described above may further comprise use of B lymphocytes in the absence of T-cells.

The B cells to be transformed can come from any suitable species and from various sources *(e.g.* from whole blood, from peripheral blood mononuclear cells (PBMCs), from blood culture, from bone marrow, from organs, etc.). Preferably, the B cell is a human B cell. Suitable methods for obtaining human B cells are well known in the art. Samples may include cells that are not memory B cells e.g. other blood cells.

A specific B lymphocyte subpopulation exhibiting a desired antigen specificity may be selected before the immortalisation step by using methods known in the art.

*Screening and isolation of B cells*

Transformed B cells are screened for those having the desired antigen specificity, and individual B cell clones can then be produced from the positive cells. The screening step may be carried out by ELISA, by staining of tissues or cells (including transfected cells), a neutralisation assay or one of a number of other methods known in the art for identifying desired antigen specificity. The assay may select on the basis of simple antigen recognition, or may select on the additional basis of a desired function *e.g.* to select neutralising antibodies rather than just antigen-binding antibodies, to select antibodies that can change characteristics of targeted cells, such as their signalling cascades, their shape, their growth rate, their capability of influencing other cells, their response to the influence by other cells or by other reagents or by a change in conditions, their differentiation status, *etc.*

The cloning step for separating individual clones from the mixture of positive cells may be carried out using limiting dilution, micromanipulation, single cell deposition by cell sorting or another method known in the art. The cloning may be carried out using limiting dilution.

The methods disclosed herein produce immortalised B cells that produce antibodies having a desired antigen specificity. Disclosed herein is an immortalised B cell clone obtainable or obtained by the methods disclosed herein. These B cells can be used in various ways e.g. as a source of monoclonal antibodies, as a source of nucleic acid (DNA or mRNA) encoding a monoclonal antibody of interest, for delivery to patients for cellular therapy, for research, etc.

Disclosed herein is a monoclonal antibody obtainable or obtained from a B cell clone of the invention. Disclosed herein are fragments of these monoclonal antibodies, particularly fragments that retain the antigen-binding activity of the antibodies.

Antigen

Any suitable antigen may be used in accordance with the present invention.

If used in pharmaceutical compositions intended for prophylactic or therapeutic vaccination or for treatment of a disease, such as for example a malignant, infectious disease or an allergy, the antigens can be derived from a eukaryotic cell (e.g. tumor, parasite, fungus), bacterial cell, viral particle or any portion thereof.

Examples of antigens useful in accordance with the present invention include, but are not limited to, (i) malaria-specific antigens such as irradiated plasmodial sporozoites or synthetic peptide antigens comprising at least one T cell and/or B cell epitope of the malarial circumsporozoite (CS) protein (see below); (ii) viral protein or peptide antigens such as those derived from influenza virus (e.g. surface glycoproteins hemagluttinin (HA) and neuraminidase (NA) [such as turkey influenza HA or an avian influenza A/Jalisco/95 H5 HA); immunodeficiency virus (e.g. a feline immunodeficiency virus (FIV) antigen, a simian immunodeficiency virus (SIV) antigen, or a human immunodeficiency virus antigen (HIV) such as gp120, gp160, p18 antigen [described in Example 2, infra]), Gag p17/24, Tat, Pol, Nef, and Env; herpesvirus (e.g. a glycoprotein, for instance, from feline herpesvirus, equine herpesvirus, bovine herpesvirus, pseudorabies virus, canine herpesvirus, herpes simplex virus (HSV, e.g., HSV tk, gB, gD), Marek's Disease Virus, herpesvirus or turkeys (HVT), or cytomegalovirus (CMV), or Epstein-Barr virus); hepatitis virus (e.g. Hepatitis B surface antigen (HBsAg)); papilloma virus; bovine leukemia virus (e.g., gp51,30 envelope antigen); feline leukemia virus (FeLV) (e.g., FeLV envelope protein, a Newcastle Disease Virus (NDV) antigen, e.g., HN or F); rous associated virus (such as RAV-1 env); infectious bronchitis virus (e.g., matrix and/or preplomer); flavivirus (e.g. a Japanese encephalitis virus (JEV) antigen, a Yellow Fever antigen, or a Dengue virus antigen); Morbillivirus (e.g. a canine distemper virus antigen, a measles antigen, or rinderpest antigen such as HA or F); rabies (e.g., rabies glycoprotein G): parvovirus (e.g., a canine parvovirus antigen); poxvirus (e.g., an ectromelia antigen, a canary poxvirus antigen, or a fowl poxvirus antigen); chicken pox virus (varicella zoster antigen); infectious bursal disease virus (e.g., VP2, VP3, or VP4); Hantaan virus; mumps virus; (iii) bacterial antigens such as lipopolysaccharides isolated from gram-negative bacterial cell walls and staphylococcus-specific, streptococcus-specific, pneumococcus-specific (e.g., PspA [see PCT Publication No. WO 92/14488]), *Neisseria gonorrheaa-specific* Borrelia-specific (e.g., OspA, OspB, OspC antigens of Borrelia associated with Lyme disease such as *Borellia burgdorferi, Borrelia afzelli,* and *Borrelia garinii* [see, e.g., US Pat. No. 5,523,089; PCT Publication Nos. WO 90/04411, WO 91/09870, WO 93/04175, WO 96/06165, WO 93/08306; PCT/US92/08697; Bergstrom et al., Mol. Microbiol., 3: 479-486, 1989; Johnson et al., Infect. and Immun. 60: 1845-1853, 1992; Johnson et al., Vaccine 13: 1086-1094, 1995; The Sixth International Conference on Lyme Borreliosis: Progress on the Development of Lyme Disease Vaccine, Vaccine 13: 133-135, 1995]), and pseudomonas-specific proteins or peptides; (iv) fungal antigens such as those isolated from candida, trichophyton, or ptyrosporum, and (v) tumor-specific proteins such as ErbB receptors, Melan A [MART1], gp100, tyrosinase, TRP-1/gp75, and TRP-2 (in melanoma); MAGE-1 and MAGE-3 (in bladder, head and neck, and non-small cell carcinoma); HPV EG and E7 proteins (in cervical cancer); Mucin [MUC-1] (in breast, pancreas, colon, and prostate cancers); prostate-specific antigen [PSA] (in prostate cancer); carcinoembryonic antigen [CEA] (in colon, breast and gastrointestinal cancers) and such shared tumor-specific antigens as MAGE-2, MAGE-4, MAGE-6, MAGE-10, MAGE-12, BAGE-1, CAGE-1,2,8, CAGE-3 to 7, LAGE-1, NY-ESO-1/LAGE-2, NA-88, GnTV, and TRP2-INT2.

Examples of antigens to be used in accordance with the invention and method of making them are described in US2007/0231344. Methods for selecting and preparing antigens for the compositions and methods of treatment described herein are known to those skilled in the art. Representative examples of such antigens include but are not limited to antigens derived from the coat or outer membrane of a pathogenic bacterium, mycobacterium or virus; a lipid, glycan or peptide derived from a cell, e.g. a cancer cell; or a synthetic molecule. In cases where a specific antigenic determinant is known this may be used in coupling reactions and, for the purposes of the present invention will be considered as the "antigen" in these cases.

Antigens include, but are not limited to, proteins, polypeptides, peptides, polysaccharides such as glycans, polysaccharide conjugates, peptide and non-peptide mimics of polysaccharides and other molecules, small molecules, lipids, glycolipids, and carbohydrates.

Using the products and methods described herein, it is possible to raise an immune response to antigens which are typically weak immunogens, such as for example small peptides, modified peptides such as for example phosphopeptides, or carbohydrates. An antigen to be used in accordance with the invention may thus comprise, for example, a peptide, a modified peptide such as for example a phosphopeptide, or a carbohydrate. The antigen may, for example, be a peptide, a phosphopeptide, or a carbohydrate.

Antigenic peptides are presented by antigen presenting cells on MHC molecules. T helper cells ($T_H$ cells) interact with MHC class II molecules. In response to activation, TH cells secrete cytokines that contribute to the activation of B cells. MHC class II molecules bind peptides that are generally between about 15 and 24 amino acids long. Smaller or longer peptides may not be presented via MHC class II and thus fail to induce a $T_H$ cell response.

However, even peptides with an appropriate

length may fail to induce a potent TH cell response, if the $T_H$ cell receptor is not able recognize the peptide bound to the MHC molecule due to its specific sequence (i.e. the peptide might lack T cell epitopes).

The products and methods of the present invention thus allow to induce immune responses to antigens, which do not induce a $T_H$ cell response (when administered in soluble form, i.e. not attached to a support as described herein).

Disclosed herein is a method for inducing or augmenting an antigen-specific immune response to a BCR-binding antigen in a subject, the method comprising administering to the subject a product comprising a support and said antigen attached to the support, and wherein an immunostimulant is either attached to the support or administered conjointly with said product, and wherein the antigen in soluble form, i.e. not attached to a support, is not able to induce a $T_H$ cell response when administered to a subject. "Inducing" in this context refers to a situation, where an antigen in soluble form is not able to induce an antigen-specific immune response, while an immune response can be induced when the antigen is attached to a support as described herein. "Augmenting" in this context refers to a situation, where an antigen in soluble form only induces a weak antigen-specific immune response, while a stronger immune response can be induced when the antigen is attached to a support as described herein. The antigen may, for example, comprise a peptide, a phosphopeptide, or a carbohydrate. The antigen may, for example, be a peptide, a phosphopeptide, or a carbohydrate.

Methods for administering an antigen to a subject are well known in the art. In general, an antigen as described herein, i.e. a particulate antigen, may be administered directly to the subject by any means, such as, e.g., parenteral administration such as intravenous, intramuscular, or subcutaneous administration, transdermal, mucosal, intranasal, intradermal or intratracheal administration or oral administration. The antigen can be administered systemically or locally.

The antigens may be derived from bacteria, mycobacteria, viruses, fungi and parasites. It is to be understood that antigens derived from a particular microorganism can be used to prevent and/or treat an infection of that microorganism. Below is provided a list of microorganisms from which antigens may be derived.

Bacteria include, but are not limited to, gram negative and gram positive bacteria. Gram positive bacteria include, but are not limited to Pasteurella species, Staphylococci species, and Streptococcus species. Gram negative bacteria include, but are not limited to, Escherichia coli, Pseudomonas species, and Salmonella species. Specific examples of infectious bacteria include but are not limited to Helicobacter pyloris, Borelia burgdorferi, Legionella pneumophilia, Mycobacteria sps (e.g. M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcus faecalis, Streptococcus bovis, Streptococcus (anaerobic species.), Streptococcus pneumoniae, pathogenic Campylobacter sp., Enterococcus sp., Haemophilus influenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacilliis moniliformis, Treponema pallidium, Treponema pertenue, Leptospira, Rickettsia, and Actinomyces israelli.

Viruses include, but are not limited to, interoviruses (including, but not limited to, viruses that the family picornaviridae, such as polio virus, coxsackie virus, echo virus), rotaviruses, adenovirus, hepatitus. Specific examples of viruses that have been found in humans include but are not limited to Retroviridae (e.g. human immunodeficiency viruses, such as HIV-1 (also referred to as HTLV-III, LAV or HTLV-III/LAV, or HIV-III; and other isolates, such as HIV-LP; Picornaviridae (e.g. polio viruses, hepatitis A virus; enteroviruses, human Coxsackie viruses, rhinoviruses, echoviruses); Calciviridae (e.g. strains that cause gastroenteritis); Togaviridae (e.g. equine encephalitis viruses, rubella viruses); Flaviridae (e.g. dengue viruses, encephalitis viruses, yellow fever viruses); Coronoviridae (e.g. coronaviruses); Rhabdoviradae (e.g. vesicular stomatitis viruses, rabies viruses); Coronaviridae (e.g. coronaviruses); Rhabdoviridae (e.g. vesicular stomatitis viruses, rabies viruses); Filoviridae (e.g. ebola viruses); Paramyxoviridae (e.g. parainfluenza viruses, mumps virus, measles virus, respiratory syncytial virus); Orthomyxoviridae (e.g. influenza viruses); Bungaviridae (e.g. Hantaan viruses, bunga viruses, phleboviruses and Nairo viruses); Arena viridae (hemorrhagic fever viruses); Reoviridae (e.g. reoviruses, orbiviurses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus; Poxviridae (variola viruses, vaccinia viruses, pox viruses); and Iridoviridae (e.g. African swine fever virus); and unclassified viruses (e.g. the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e. Hepatitis C); Norwalk and related viruses, and astroviruses).

Viruses that infect both human and non-human vertebrates, include retroviruses, RNA viruses and DNA viruses. This group of retroviruses includes both simple retroviruses and complex retroviruses. The simple retroviruses include the subgroups of B-type retroviruses, C-type retroviruses and D-type retroviruses. An example of a B-type retrovirus is mouse mammary tumor virus

(MMTV). The C-type retroviruses include subgroups C-type group A (including Rous sarcoma virus (RSV), avian leukemia virus (ALV), and avian myeloblastosis virus (AMV)) and C-type group B (including murine leukemia virus (MLV), feline leukemia virus (FeLV), murine sarcoma virus (MSV), gibbon ape leukemia virus (GALV), spleen necrosis virus (SNV), reticuloendotheliosis virus (RV) and simian sarcoma virus (SSV)). The D-type retroviruses include Mason-Pfizer monkey virus (MPMV) and simian retrovirus type 1 (SRV-1). The complex retroviruses include the subgroups of lentiviruses, T-cell leukemia viruses and the foamy viruses. Lentiviruses include HIV-1, but also include HIV-2, SIV, Visna virus, feline immunodeficiency virus (FIV), and equine infectious anemia virus (EIAV). The T-cell leukemia viruses include HTLV-I, HTLV-II, simian T-cell leukemia virus (STLV), and bovine leukemia virus (BLV). The foamy viruses include human foamy virus (HFV), simian foamy virus (SFV) and bovine foamy virus (BFV).

Examples of other RNA viruses that are antigens in vertebrate animals include, but are not limited to, members of the family Reoviridae, including the genus Orthoreovirus (multiple serotypes of both mammalian and avian retroviruses), the genus Orbivirus (Bluetongue virus, Eugenangee virus, Kemerovo virus, African horse sickness virus, and Colorado Tick Fever virus), the genus Rotavirus (human rotavirus, Nebraska calf diarrhea virus, murine rotavirus, simian rotavirus, bovine or ovine rotavirus, avian rotavirus); the family Picornaviridae, including the genus Enterovirus (poliovirus, Coxsackie virus A and B, enteric cytopathic human orphan (ECHO) viruses, hepatitis A virus, Simian enteroviruses, Murine encephalomyelitis (ME) viruses, Poliovirus muris, Bovine enteroviruses, Porcine enteroviruses, the genus Cardiovirus (Encephalomyocarditis virus (EMC), Mengovirus), the genus Rhinovirus (Human rhinoviruses including at least 113 subtypes; other rhinoviruses), the genus Apthovirus (Foot and Mouth disease (FMDV); the family Calciviridae, including Vesicular exanthema of swine virus, San Miguel sea lion virus, Feline picornavirus and Norwalk virus; the family Togaviridae, including the genus Alphavirus (Eastern equine encephalitis virus, Semliki forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1. Sendai virus, Hemadsorption virus, Parainifluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); the family Rhabdoviridae, including the genus Vesiculovirus (VSV), Chandipura virus, Flanders-Hart Park virus), the genus Lyssavirus (Rabies virus), fish Rhabdoviruses, and two probable Rhabdoviruses (Marburg virus and Ebola virus); the family Arenaviridae, including Lymphocytic choriomeningitis virus (LCM), Tacaribe virus complex, and Lassa virus; the family Coronoaviridae, including Infectious Bronchitis Virus (IBV), Mouse Hepatitis virus, Human enteric corona virus, and Feline infectious peritonitis (Feline coronavirus).

Illustrative DNA viruses that infect vertebrate animals include but are not limited to the family Poxviridae, including the genus Orthopoxvirus (Variola major,

Variola minor, Monkey pox Vaccinia, Cowpox, Buffalopox, Rabbitpox, Ectromelia), the genus Leporipoxvirus (Myxoma, Fibroma), the genus Avipoxvirus (Fowlpox, other avian poxvirus), the genus Capripoxvirus (sheeppox, goatpox), the genus Suipoxvirus (Swinepox), the genus Parapoxvirus (contagious postular dermatitis virus, pseudocowpox, bovine papular stomatitis virus); the family Iridoviridae (African swine fever virus, Frog viruses 2 and 3, Lymphocystis virus of fish); the family Herpesviridae, including the alpha-Herpesviruses (Herpes Simplex Types 1 and 2, Varicella-Zoster, Equine abortion virus, Equine herpes virus 2 and 3, pseudorabies virus, infectious bovine keratoconjunctivitis virus, infectious bovine rhinotracheitis virus, feline rhinotracheitis virus, infectious laryngotracheitis virus) the Beta-herpesviruses (Human cytomegalovirus and cytomegaloviruses of swine, monkeys and rodents); the gamma-herpesviruses (Epstein-Barr virus (EBV), Marek's disease virus, Herpes saimiri, Herpesvirus ateles, Herpesvirus sylvilagus, guinea pig herpes virus, Lucke tumor virus); the family Adenoviridae, including the genus Mastadenovirus (Human subgroups A,B,C,D,E and ungrouped; simian adenoviruses (at least 23 serotypes), infectious canine hepatitis, and adenoviruses of cattle, pigs, sheep, frogs and many other species, the genus Aviadenovirus (Avian adenoviruses); and non-cultivatable adenoviruses; the family Papoviridae, including the genus Papillomavirus (Human papilloma viruses, bovine papilloma viruses, Shope rabbit papilloma virus, and various pathogenic papilloma viruses of other species), the genus Polyomavirus (polyomavirus, Simian vacuolating agent (SV-40), Rabbit vacuolating agent (RKV), K virus, BK virus, JC virus, and other primate polyoma viruses such as Lymphotrophic papilloma virus); the family Parvoviridae including the genus Adeno-associated viruses, the genus Parvovirus (Feline panleukopenia virus, bovine parvovirus, canine parvovirus, Aleutian mink disease virus, etc). Finally, DNA viruses may include viruses which do not fit into the above families such as Kuru and Creutzfeldt-Jacob disease viruses and chronic infectious neuropathic agents (CHINA virus).

Fungi are eukaryotic organisms, only a few of which cause infection in vertebrate mammals. Because fungi are eukaryotic organisms, they differ significantly from prokaryotic bacteria in size, structural organization, life cycle and mechanism of multiplication. Fungi are classified generally based on morphological features, modes of reproduction and culture characteristics. Although fungi can cause different types of disease in subjects, such as respiratory allergies following inhalation of fungal antigens, fungal intoxication due to ingestion of toxic substances, such as amatatoxin and phallotoxin produced by poisonous mushrooms and aflotoxins, produced by aspergillus species, not all fungi cause infectious disease.

Infectious fungi can cause systemic or superficial infections. Primary systemic infection can occur in normal healthy subjects and opportunistic infections, are most frequently found in immuno-compromised subjects. The most common fungal agents causing primary systemic infection include blastomyces, coccidioides, and histoplasma. Common fungi causing opportunistic infection in immuno-compromised or immunosuppressed subjects include, but are not limited to, candida albicans (an organism which is normally part of the respiratory tract flora), cryptococcus neoformans (sometimes in normal flora of respiratory tract), and various aspergillus species. Systemic fungal infections are invasive infections of the internal organs. The organism usually enters the body through the lungs, gastrointestinal tract, or intravenous lines. These types of infections can be caused by primary pathogenic fungi or opportunistic fungi.

Fungi include but are not limited to microsporum or traicophyton species, i.e., microsporum canis, microsporum gypsum, tricofitin rubrum, Cryptococcus neoformans, Histoplasma capsulatum, Coccidioides immitis, Blaslomyces dermatitidis, Chlamydia trachomatis, and Candida albicans.

Parasites include but are not limited to Plasmodium falciparum, Plasmodium ovale, Plasmodium malariae, Plasmdodium vivax, Plasmodium knowlesi, Babesia microti, Babesia divergens, Trypanosoma cruzi, Toxoplasma gondii, Trichinella spiralis, Leishmania major, Leishmania donovani, Leishmania braziliensis and Leishmania tropica, Trypanosoma gambiense, Trypanosmoma rhodesiense and Schistosoma mansoni.

Other medically relevant microorganisms have been described extensively in the literature, e.g., see C. G. A Thomas, Medical Microbiology, Bailliere Tindall, Great Britain 1983. Each of the foregoing lists is illustrative, and is not intended to be limiting.

Other pathogens include Gonorrhea, H. pylori, Staphylococcus spp., Streptococcus spp. such as Streptococcus pneumoniae, Syphilis; viruses such as SARS virus, Hepatitis virus, Herpes virus, HIV virus, West Nile virus, Influenza virus, poliovirus, rhinovirus; parasites such as Giardia, and Plasmodium malariae (malaria); and mycobacteria such as M. tuberculosis.

Antigens may include toxins or other molecules produced from microorganisms. Examples of such molecules are provided below.

Examples of toxins include abrin, ricin and strychnine. Further examples of toxins include toxins produced by Corynebacterium diphtheriae (diphtheria), Bordetella pertussis (whooping cough), Vibrio cholerae (cholera), Bacillus anthracis (anthrax), Clostridium botulinum (botulism), Clostridium tetani (tetanus), and enterohemorrhagic Escherichia coli (bloody diarrhea and hemolytic uremic syndrome), Staphylococcus aureus alpha toxin, Shiga toxin (ST), cytotoxic necrotizing factor type 1 (CNF1), E. coli heat-stable toxin (ST), botulinum, tetanus neurotoxins, S. aureus toxic shock syndrome toxin (TSST), Aeromonas hydrophila aerolysin, Clostridium perfringens perfringolysin O, E. coli hemolysin, Listeria monocytogenes listeriolysin O, Streptococcus pneumoniae pneumolysin, Streptococcus pyogenes streptolysine O, Pseudomonas aeruginosa exotoxin A, E. coli DNF, E. coli LT, E. coli CLDT, E. coli EAST, Bacillus anthracis edema factor, Bordetella pertussis dermonecrotic toxin, Clostridium botulinum C2 toxin, C. botulinum C3 toxin, Clostridium difficile toxin A, and C. difficile toxin B.

Further examples of bacteria include but are not limited to Streptococcus spp., Staphylococcus spp., Pseudomonas spp., Clostridium difficile, Legionella spp., Pneumococcus spp., Haemophilus spp. (e.g., Haemophilus influenzae), Klebsiella spp., Enterobacter spp., Citrobacter spp., Neisseria spp. (e.g., N. meningitidis, N. gonorrhoeae), Shigella spp., Salmonella spp., Listeria spp. (e.g., L. monocytogenes), Pasteurella spp. (e.g., Pasteurella multocida), Streptobacillus spp., Spirillum spp., Treponema spp. (e.g., Treponema pallidum), Actinomyces spp. (e.g., Actinomyces israelli), Borrelia spp., Corynebacterium spp., Nocardia spp., Gardnerella spp. (e.g., Gardnerella vaginalis), Campylobacter spp., Spirochaeta spp., Proteus spp., Bacteriodes spp. and H. pylori.

Further examples of viruses include but are not limited to HIV, Herpes simplex virus 1 and 2 (including encephalitis, neonatal and genital forms), human papilloma virus, cytomegalovirus, Epstein Barr virus, Hepatitis virus A, B and C, rotavirus, adenovirus, influenza A virus, respiratory syncytial virus, varicella-zoster virus, small pox, monkey pox and SARS virus.

Further examples of fungi that can be used include but are not limited to candidiasis, ringworm, histoplasmosis, blastomycosis, paracoccidioidomycosis, crytococcosis, aspergillosis, chromomycosis, mycetoma, pseudallescheriasis, and tinea versicolor.

Further examples of parasites that can be used include but are not limited to protozoa and nematodes such as amebiasis, Trypanosoma cruzi, Fascioliasis (e.g., Facioloa hepatica), Leishmaniasis, Plasmodium (e.g., P. falciparum, P. knowlesi, P. malariae,) Onchocerciasis, Paragonimiasis, Trypanosoma brucei, Pneumocystis (e.g., Pneumocystis carinii), Trichomonas vaginalis, Taenia, Hymenolepsis (e.g., Hymenolepsis nana), Echinococcus, Schistosomiasis (e.g., Schistosoma mansoni), neurocysticercosis, Necator americanus, and Trichuris trichuria.

Further examples of pathogens that can be used include but are not limited to Chlamydia, M. tuberculosis, and M. leprosy, and Rickettsiae.

An antigen that can be used in subjects having or at risk of developing cancer includes but is not limited to a cancer antigen. Cancer antigens include but are not limited to HER 2 (p185), CD20, CD33, GD3 ganglioside, GD2 ganglioside, carcinoembryonic antigen (CEA), CD22, milk mucin core protein, TAG-72, Lewis A antigen, ovarian associated antigens such as OV-TL3 and MOv18, high Mr melanoma antigens recognized by antibody 9.2.27. HMFG-2. SM-3, B72.3. PR5C5, PR4D2, and the like. Other cancer antigens are described in U.S. Pat. No. 5,776,427.

Cancer antigens can be classified in a variety of ways. Cancer antigens include antigens encoded by genes that have undergone chromosomal alteration. Many of these antigens are found in lymphoma and leukemia. Even within this classification, antigens can be characterized as those that involve activation of quiescent genes. These include BCL-1 and IgH (Mantel cell lymphoma), BCL-2 and IgH (Follicular lymphoma), BCL-6 (Diffuse large B-cell lymphoma), TAL-1 and TCR- or SIL (T-cell acute lymphoblastic leukemia), c-MYC and IgH or IgL (Burkitt lymphoma), MUN/IRF4 and IgH (Myeloma), PAX-5 (BSAP) (Immunocytoma).

Other cancer antigens that involve chromosomal alteration and thereby create a novel fusion gene and/or protein include RAR-, PML, PLZF, NPM or NuMA (Acute promyelocytic leukemia), BCR and ABL (Chronic myeloid/acute lymphoblastic leukemia), MLL (HRX) (Acute leukemia), E2A and PBX or HLF (B-cell acute lymphoblastic leukemia), NPM, ALK (Anaplastic large cell leukemia), and NPM, MLF-1 (Myelodysplastic syndrome/acute myeloid leukemia).

Other cancer antigens are specific to a tissue or cell lineage. These include cell surface proteins such as CD20, CD22 (Non-Hodgkin's lymphoma, B-cell lymphoma, Chronic lymphocytic leukemia (CLL)), CD52 (B-cell CLL), CD33 (Acute myelogenous leukemia (AML)), CD10 (gp100) (Common (pre-B) acute lymphocytic leukemia and malignant melanoma), CD3/T-cell receptor (TCR) (T-cell lymphoma and leukemia), CD79/B-cell receptor (BCR) (B-cell lymphoma and leukemia), CD26 (Epithelial and lymphoid malignancies), Human leukocyte antigen (HLA)-DR, HLA-DP, and HLA-DQ (Lymphoid malignancies), RCAS1 (Gynecological carcinomas, bilary adenocarcinomas and ductal adenocarcinomas of the pancreas), and Prostate specific membrane antigen (Prostate cancer).

Tissue- or lineage-specific cancer antigens also include epidermal growth factor receptors (high expression) such as EGFR (HER1 or erbB1) and EGFRvIII (Brain, lung, breast, prostate and stomach cancer), erbB2 (HER2 or HER2/neu) (Breast cancer and gastric cancer), erbB3 (HER3) (Adenocarcinoma), and erbB4 (HER4) (Breast cancer).

Tissue- or lineage-specific cancer antigens also include cell-associated proteins such as Tyrosinase, Melan-A/MART-1, tyrosinase related protein (TRP)-1/gp75 (Malignant melanoma), Polymorphic epithelial mucin (PEM) (Breast tumors), and Human epithelial mucin (MUC1) (Breast, ovarian, colon and lung cancers).

Tissue- or lineage-specific cancer antigens also include secreted proteins such as Monoclonal immunoglobulin (Multiple myeloma and plasmacytoma), Immunoglobulin light chains (Multiple Myeloma), --fetoprotein (Liver carcinoma), Kallikreins 6 and 10 (Ovarian cancer), Gastrin-releasing peptide/bombesin (Lung carcinoma), and Prostate specific antigen (Prostate cancer).

Still other cancer antigens are cancer testis (CT) antigens that are expressed in some normal tissues such

as testis and in some cases placenta. Their expression is common in tumors of diverse lineages and as a group the antigens form targets for immunotherapy. Examples of tumor expression of CT antigens include MAGE-A1, -A3, -A6, -A12, BAGE, GAGE, HAGE, LAGE-1, NY-ESO-1, RAGE, SSX-1, -2, -3, -4, -5, -6, -7, -8, -9, HOM-TES-14/SCP-1, HOM-TES-85 and PRAME. Still other examples of CT antigens and the cancers in which they are expressed include SSX-2, and -4 (Neuroblastoma), SSX-2 (HOM-MEL-40), MAGE, GAGE, BAGE and PRAME (Malignant melanoma), HOM-TES-14/SCP-1 (Meningioma), SSX-4 (Oligodendrioglioma), HOM-TES-14/SCP-1, MAGE-3 and SSX-4 (Astrocytoma), SSX member (Head and neck cancer, ovarian cancer, lymphoid tumors, colorectal cancer and breast cancer), RAGE-1, -2, -4, GAGE-I, -2, -3, -4, -5, -6, -7 and -8 (Head and neck squamous cell carcinoma (HNSCC)), HOM-TES14/SCP-1, PRAME, SSX-1 and CT-7 (Non-Hodgkin's lymphoma), and PRAME (Acute lymphoblastic leukemia (ALL), acute myelogenous leukemia (AML) and chronic lymphocytic leukemia (CLL)).

Other cancer antigens are not specific to a particular tissue or cell lineage. These include members of the carcinoembryonic antigen (CEA) family: CD66a, CD66b, CD66c, CD66d and CD66e. These antigens can be expressed in many different malignant tumors and can be targeted by immunotherapy.

Still other cancer antigens are viral proteins and these include Human papilloma virus protein (cervical cancer), and EBV-encoded nuclear antigen (EBNA)-1 (lymphomas of the neck and oral cancer).

Still other cancer antigens are mutated or aberrantly expressed molecules such as but not limited to CDK4 and beta-catenin (melanoma).

Still other cancer antigen may be selected from the group consisting of MART-1/Melan-A, gp100, adenosine deaminase-binding protein (ADAbp), FAP, cyclophilin b, colorectal associated antigen (CRC)--C017-lA/GA733, carcinoembryonic antigen (CEA), CAP-1, CAP-2, etv6, AML1, prostate specific antigen (PSA), PSA-1, PSA-2, PSA-3, prostate-specific membrane antigen (PSMA), T-cell receptor/CD3-zeta chain, and CD20. The cancer antigen may also be selected from the group consisting of MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5). In still another embodiment, the cancer antigen is selected from the group consisting of GAGE-1, GAGE-2, GAGE-3, GAGE-4, GAGE-5, GAGE-6, GAGE-7, GAGE-8, GAGE-9. And in yet a further embodiment, the cancer antigen is selected from the group consisting of BAGE, RAGE, LAGE-1, NAG, GnT-V, MUM-1, CDK4, tyrosinase, p53, MUC family, HER2/neu, p21ras, RCAS1, [alpha]-fetoprotein, E-cadherin, [alpha]-catenin, P-catenin, [gamma]-catenin, p120ctn, gp100<Pmel117>, PRAME, NY-ESO-1, cdc27, adenomatous polyposis coli protein (APC), fodrin, Connexin 37, Ig-idiotype, p15, gp75, GM2 ganglioside, GD2 ganglioside, human papilloma virus proteins, Smad family of tumor antigens, lmp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2.

The antigen may be a proteinaceous antigen. It may be an enzymatic antigen, such as a non-human mammalian enzyme. It may for example be hen egg lysozyme (HEL) or chicken gamma globulin (CGG). It may be an antigen useful for an in vitro model system.

In a further aspect, the present invention further provides a pharmaceutical composition comprising a product of the invention in association with one or more pharmaceutically acceptable carriers or diluents, and the use of said compositions in methods of immunotherapy for treatment or prophylaxis of a human or animal subject. The pharmaceutical composition may further comprise a soluble immunostimulant.

Pharmaceutically acceptable carriers or diluents include those used in formulations suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy.

The products and compositions described herein may be used to elicit an enhanced immune response in vitro or in vivo. They may be used to increase the immunogenicity of an antigen. The invention provides methods comprising the step of contacting the products and compositions described herein with a target cell, typically a B cell, in order to elicit an antigen-specific BCR-mediated immune response. The density (and thus avidity) of the antigen bound to the support is adjusted in the ways described herein to achieve the desired effect.

The products and compositions of the disclosed herein may be administered to a subject to treat, prevent or alleviate a disease. Said diseases may be any disease amenable to the treatment with the compositions and products of the invention, for example a malignant disease such as cancer, and infectious disease, an allergy or an autoimmune disease. Treatment of a subject with products and compositions disclosed herein may be combined with other treatments.

Disclosed herein is a method of inducing an antigen-specific BCR-mediated immune response in a subject comprising the step of administering to a subject an effective amount of a product or composition as described herein, to allow specific BCR-mediated internalization and B cell activation.

Disclosed herein is a method of inducing a BCR-mediated immune response to an antigen comprising the step of administering to a subject an effective amount of a product, the product comprising (i) a support, (ii) at least one BCR-binding antigen attached to the support, and,

optionally, (iii) at least one immunostimulant attached to the support, or composition comprising said product. The antigen is present at a sufficient density (and thus avidity) on the support to allow BCR-mediated activation of antigen specific immune responses. The method may be used for prophylactic or therapeutic vaccination.

Disclosed herein is a method of inducing a BCR-mediated immune response to an antigen comprising the step of administering to a subject an effective amount of a product (or a composition comprising the product), the product comprising (i) a support, and (ii) at least one BCR-binding antigen attached to the support, the method further comprising conjointly administering to a subject a soluble immunostimulant. The antigen is present at a sufficient density (and thus avidity) on the support to allow BCR-mediated activation of antigen specific immune responses. The method may be used for prophylactic or therapeutic vaccination.

Suitable ways of administering the products and compositions of the present invention are known in the art. Any suitable method of administration may be used.

The subject may be a non-human mammal, for example a rabbit, a sheep, a guinea pig etc. The subject may be a human. The subject may be a healthy subject, or may be suffering from a disease, or suspected of suffering from a disease, amenable for treatment with the products, compositions or methods described herein. The diseases may, for example and without limitation, be a malignant disease, such as cancer, an infectious disease, such as infection with a parasite, or an allergy, or generally an autoimmune disease.

Disclosed herein is a method for augmenting the immunogenicity of a BCR-binding antigen in a subject, comprising administering to the subject a product comprising a support and said antigen attached to the support, and wherein an immunostimulant is either attached to the support or administered conjointly with said product.

Disclosed herein is a method for augmenting the immunogenicity of a BCR-binding antigen in a subject, comprising administering to the subject a product or composition as described herein, wherein the antigen is present at a sufficient density (and thus avidity) on the support to allow activation of antigen-specific immune responses.

"Augmenting" is understood to mean enhancing or extending the duration of an immune response. Thus, even antigens that would not elicit an immune response, or a weak immune response, when administered alone in soluble form, can elicit a strong immune response when administered on a product as described herein. The immune response to the antigen is thus enhanced compared to administering the antigen alone in soluble or particulate form. The immune response is also enhanced compared to administering the antigen together with soluble immunostimulant.

Disclosed herein is a process for making a product as describe herein, comprising the steps of

(a) attaching a BCR-binding antigen to a support, and, optionally,

(b) attaching an immunostimulant to the support. Step (b) may optionally be performed prior to step (a).

Disclosed herein is a method for augmenting the antigen-specific immune response to a BCR-binding antigen in a subject, the method comprising the steps of

(a) attaching the antigen to a support, and

(b) attaching an immunostimulant to the support, optionally performing steps (a) and (b) in reversed order, and

(c) administering the support to a subject,

wherein the antigen is present at a sufficient density (and thus avidity) on the support to allow specific BCR-activation.

Alternatively, the immunostimulant is not attached to the support, but administerd conjointly to the subject.

Disclosed herein is a support for use in preparing a product as described herein, wherein an immunostimulant is attached to the support.

Disclosed herein is a method for enhancing a BCR-mediated, antigen-specific immune response in a subject, comprising administering to a subject a product or composition as described herein.

Disclosed herein is a method of inducing specific uptake of an immunostimulant by a cell, comprising the steps of

(i) attaching a BCR-binding antigen to a support,

(ii) attaching the immunostimulant to said support, optionally performing steps (i) and (ii) in reversed order, and (iii) contacting the cell with the support so prepared to allow specific BCR-mediated internalization and B cell activation.

The cell expresses BCR and is typically a B cell. The support so prepared is thus suitable to be internalized by a B cell. The cell may be present in a tissue or a subject.

Disclosed herein is a method for inhibiting non-specific uptake of an immunostimulant by a B cell, the method comprising the step of attaching said immunostimulant to a support prior to contact with said B cell. An antigen may be attached to the support, such as a BCR-binding antigen.

Inhibition comprises partial inhibition, i.e. restricting or limiting the non-specific uptake. Presenting the immunostimulant on a support prevents unspecific uptake by B cells. If a BCR-binding antigen is attached to the support, preventing or restricting un-specific uptake leads to increased specific uptake of the support via BCR-engagement.

Disclosed herein is a method of delivering a BCR-binding antigen and an immunostimulant to a cell

for eliciting an antigen-specific immune response, comprising

(i) attaching a BCR-binding antigen to a support at a first density,
(ii) attaching the immunostimulant to said support, optionally performing steps (i) and (ii) in reversed order,
(iii) contacting the support so obtained with a cell,
(iv) testing the cell for antigen-mediated activation, optionally repeating steps (ii) to (iv) varying the density of the antigen on the support.

Vaccines - generation of antibodies - medical uses

Disclosed herein is the use of the products and methods described herein for prophylactic or therapeutic vaccination for a BCR-mediated immune response.

Disclosed herein are products, compositions and methods which may be used for stimulating immune responses in humans and/or other subjects, which may be beneficial for (but is not limited to) preventing and/or treating diseases. As used herein, to treat a subject means to provide some therapeutic or prophylactic benefit to the subject. This may occur by reducing partially or completely symptoms associated with a particular condition. Treating a subject is not however limited to curing the subject of the particular condition.

The products, compositions and methods described herein may be used as a vaccine. They may be used for prophylactic or therapeutic vaccination.

The products or compositions described herein stimulate an immune response leading to the production of immune molecules, including antibodies that bind to antigens. Disclosed herein are vaccines sufficient to reduce the number, severity and/or duration of symptoms. The vaccine may also contain antigens in free form and such antigens may be the same as or different from those on the support.

In addition to product or composition as described above, a vaccine may include salts, buffers, adjuvants and other substances, or excipients which may be desirable for improving its efficacy. Examples of suitable vaccine components as well as a general guidance with regard to methods for preparing effective compositions may be found in standard texts such as Remington's Pharmaceutical Sciences (Osol, A, ed., Mack Publishing Co., (1990)). In all cases, the product or composition as described herein should be present in an effective amount, i.e. an amount that produces the desired effect. Other components of the vaccine should be physiologically acceptable. The vaccine of the present invention may be administered by either single or multiple dosages of an effective amount of product or composition.

The vaccine is generally administered in effective amounts, i.e. amounts which are sufficient to induce the desired immune response.

Vaccines may be administered to subjects by any route known in the art, including parenteral routes (e.g. injection), inhalation, topical or by oral administration. Suitable methods include, for example, intramuscular, intravenous, or subcutaneous injection, or intradermal or intranasal administration. Suitable carriers that may be used in preparations for injection include sterile aqueous (e.g., physiological saline) or non-aqueous solutions and suspensions such as propylene glycol, polyethylene glycol, vegetable oils such as olive oil, and injectable organic esters such as ethyl oleate. Treatment and dosing strategies may be developed using guidance provided by standard reference works (see e.g. N. Engl. J. Med. 345 (16):1177-83(2001) for treatment of children, and Arch. Intern. Med 154(22):2545-57 (1994) for adult treatments; see Arch. Intern. Med 28, 154(4):373-7 (1994) for a review of clinical trials.

Vaccines may be administered to a subject to treat a disease after symptoms have appeared. In these cases, it will be advantageous to initiate treatment as soon after the onset of symptoms as possible and, depending on the circumstances, to combine vaccine administration with other treatments, e.g. the administration of antibiotics, or anti-cancer treatments such as chemotherapy or radiotherapy.

Disclosed herein is a method of producing immune molecules, such as antibodies, against an antigen, said method comprising introducing a product or composition of the invention into a non-human mammal, and recovering immune serum from said mammal. The immune serum obtainable by this method is also disclosed herein.

Methods of raising serum and antibodies, including monoclonal antibodies, as well as different forms of antibodies, including antibody fragments, are known in the art (Roitt's Essential Immunology, eleventh edition, Blackwell Publishing).

Methods of producing antibodies include immunising a mammal (e.g. mouse, rat, rabbit, horse, goat, sheep or monkey) with the products or compositions described herein. Antibodies may be obtained from immunised animals using any of a variety of techniques known in the art, and might be screened, preferably using binding of antibody to antigen of interest.

For instance, Western blotting techniques or immunoprecipitation may be used (Armitage et al, 1992, Nature 357: 80-82). Antibodies may be polyclonal or monoclonal.

Antibodies may be modified in a number of ways. Indeed the term "antibody" should be construed as covering any specific binding substance having a binding domain with the required specificity. Thus, this term covers antibody fragments, derivatives, functional equivalents and homologues of antibodies, including any polypeptide comprising an immunoglobulin binding domain, whether natural or synthetic. As an alternative or supplement to immunising a mammal, antibodies with appropriate binding specificity may be obtained from a recombinantly produced library of expressed immunoglobulin variable domains, e.g. using lambda bacteriophage or filamentous bacteriophage which display functional immunoglobulin binding domains on their surfaces; for instance see WO92/01047.

Disclosed herein is a method for accelerating the production of specific antibodies, the method comprising introducing a product or composition as described herein into a non-human mammal, and recovering antibodies from said mammal.

Using the methods, products and/or compositions described herein in the generation of an antigen-specific serum or specific antibodies shortens the time interval between immunisation and the time when specific antibodies are generated in the immunised subject, compared to immunisation with antigen (with or without soluble immunostimulant). In other words, when using the methods described herein, specific antibodies can be detected and recovered earlier compared to conventional immunisation methods.

This has advantages in clinical practice. For example, shortened vaccination intervals are beneficial for people that require reliable immunisation within a constrained timescale, such as for example travellers.

As discussed above, the products and methods described herein also result in augmentation of immune responses to a given antigen. This offers further clinical and practical benefits. For example, the number of administrations of a vaccine may be reduced due to the enhanced immune response. For example, the HBV prophylactic vaccine is typically given with 3 administrations at 0, 1 and 6 months. Often, people receive the vaccine at 0 and 1 month, but fail to return for the final 6 month administration. Being able to reduce the number of administrations necessary to achieve a reliable immunisation is thus beneficial. Being able to induce an enhanced immune response is further beneficial for the vaccination of individuals with a weakened or less responsive immune system, such as for example the elderly. Typically, if the normal administration cycle with a vaccine fails to establish a reliable immunization in these individuals, they have to receive a further administration. Using the products and methods of the invention, a strong response can also be achieved in these individuals, making an extra administration step unnecessary.

The methods and products disclosed herein thus allow to achieve immunisation within a shorter time and/or with less administrations.

Shortened response times and enhanced responses are also beneficial in treatment and therapy, where a quick and strong response to an administered agent is critical for treatment or allows faster recovery of the patient and/or faster relief of symptoms.

Thus, described herein are methods, products and compositions for accelerating an antigen-specific BCR-mediated immune response.

The immune molecules produced by immunization with vaccines, e.g. antibodies, may be transferred to another individual, thus passively transferring immunity.

Disclosed herein is a method of passive immunisation against a disease, said method comprising administering to a subject an immune serum containing antibodies obtainable by the methods described herein.

The products and compositions of the present invention find utility as medicaments.

Preferential delivery of an agent to dendritic cells vs B cells

The authors have shown that while soluble immunostimulant is unspecifically taken up by B cells, an immunostimulant attached to a support is not taken up by B cells (Fig 1B,C), but still internalised by dendritic cells (Fig 19). By attaching an agent to a support it is therefore possible to prevent uptake by B cells and instead achieve preferential delivery to dendritic cells. Since the number of B cells in the blood of, for example, a human is larger than the number of dendritic cells, a large proportion of agent molecules is thus generally taken up by B cells if the agent is administered in a soluble form. More agent must therefore be administered to achieve sufficient delivery to dendritic cells. Using the methods disclosed herein, directed (specific) delivery to dendritic cells (preferential delivery to dendritic cells versus B cells) can be achieved. This has several benefits. Less agent can be administered, since it is delivered more efficiently to dendritic cells and not taken up by B cells. Further, fewer or less severe side effects can be expected since there is reduced unspecific activation of B cells.

Disclosed herein is a method of delivering an agent preferentially to dendritic cells versus B cells, the method comprising

(i) attaching the agent to a support,
(ii) contacting a population of cells with the agent attached to the support, wherein the population of cells comprise B cells and dendritic cells.

The support is free of BCR antigen so that no BCR-mediated uptake by B cells can be induced (or free of BCR antigen in an amount that would induce BCR-mediated uptake).

The method may be performed in vivo or in vitro.

Disclosed herein is a method of delivering an agent preferentially to dendritic cells versus B cells in a subject, the method comprising

(i) attaching the agent to a support,
(ii) administering the agent on the support to a subject.

The subject may be a mammal, preferably a human.

The agent may be an immunostimulant as described above.

## EXAMPLES

### Example 1 - Immunostimulatory Lipids

### MATERIALS AND METHODS

#### Antigens, lipid preparation and microsphere coating

HEL and OVA were purchased from Sigma and CGG from Jackson Immuno Research. If required, antigens were biotinylated by using sulfo-NHS-LC-LC-biotin (Pierce). 1,2-Dioleoyl-sn-Glycero-3-Phosphocholine (DOPC) and N-Cap biotinyl-phosphatidylethanolamine (PE-biotin) were purchased from Avanti Polar Lipids. αGalCer was purchased from Alexis Biochemical. IMM47 was a gift from Dr Vincenzo Cerundolo (WO2007/050668). The synthesis of αGalCer-Alexa 488 was based on the methodology utilised for the synthesis of biotinylated αGalCer (36) using Alexa Fluor 488 (Invitrogen). For the preparation of liposomes containing DOPC/PE-biotin (98/2, m/m) or DOPC/PE-biotin/αGalCer (88/2/10, m/m/m) lipids were dried under argon and resuspended in Tris 25 mM/NaCl 150 mM, pH 7.0 with vigorous mixing.

Silica microspheres (100 nm) were purchased from Kisker GbR. For coating, microspheres were incubated with liposomes followed by addition of streptavidin and saturating amounts of biotinylated proteins. For beads coated with different HEL densities/affinities, antigens were bound to the particles by using a biotinylated (Fab'$_2$)-F10 anti-HEL antibody. Where different antigen densities were required, the biotinylated antibody was competed with different amounts of biotinylated CGG. Density of HEL on the beads was detected by FACS using an anti-HEL monoclonal antibody. The binding of antigen to liposome-coated beads was detected by western blot.

For quantification of the amount of αGalCer bound to particles, they were coated with liposomes containing αGalCer-Alexa 488. Fluorescence intensity from liposome-coated beads was measured by using an EnVision Multilabel Reader and related to the fluorescence intensity of different amounts αGalCer-Alexa 488 containing liposomes.

#### Mice and cell lines

MD4, D1.3, D1.3-H2, and Jα18-/- mice were bred and maintained at the animal facility of Cancer Research UK and of the John Radcliffe Hospital, Oxford. C57BL/6 mice were purchased from Charles River. All experiments were approved by the Cancer Research UK Animal Ethics Committee and the United Kingdom Home Office. iNKT hybridoma DN32.D3 was kindly provided by A. Bendelac (University of Chicago, Chicago, IL).

#### B cell purification and presentation assays

Splenic B cells were enriched by negative selection to > 99% purity using B cell purification kit (Miltenyi Biotec). For analysis of αGalCer presentation, B cells were incubated overnight with particles containing αGalCer and/or HEL, extensively washed and cultured at 5 x $10^4$ cells per well with the same number of DN32.D3 cells. NKT activation was assayed by measuring IL-2 production in the culture supernatant. For blocking experiments MD4 B cells were incubated with an antiCD1d blocking antibody (25 μg/ml; clone 1B1) for 2h before incubation with iNKT cells.

#### Adoptive transfer and FACS analyses

Five to ten millions of MD4 B cells were labelled with 2 μM CFSE (Molecular Probes) and adoptively transferred by tailvein injection into WT C57BL/6 or Jα18-/- mice together with particles containing αGalCer and/or HEL. Five days later spleens from recipient mice were harvested and splenocytes were stained for surface molecules and intracellular HEL-binding as previously described (37).

#### Immunizations

Mice were immunized intraperitoneally with 1-10μl of beads containing different antigens and/or αGalCer. Antigen-specific Ig levels were determined in mice sera by ELISA.

#### ELISA and ELISPOT

Concentration of IL-2 in the supernatant of the cultures was determined by ELISA using the JES6-1A12 capture antibody (BD Pharmingen). Biotinylated JSE6-5H4 (BD Pharmingen) was used for detection. Specific antibody in mice sera were measured using plates coated with antigen (HEL, OVA or CGG) and serial dilutions of sera. Bound antibodies were detected with biotin-labelled goat anti-mouse IgM, IgG, IgG1, IgG2b, IgG2c or IgG3 (BD Pharmingen).

The frequency of anti-HEL antibody secreting cells was detected by ELISPOT. Single cell suspensions of splenocytes were incubated for 15-18 hours in HEL-coated multiscreen filtration plates (Millipore). Spots were revealed with goat anti mouse biotinilated IgMa followed by streptavidin-peroxidase and 3-Amino-9-ethylcarbazole (Sigma).

#### Immunohistochemistry

Cryostat sections (10 μm thickness) of spleens were fixed and stained with rat anti-mouse CD45R/B220 (BD Biosciences). HEL+ cells were detected by addition of HEL (200 ng/ml) followed by anti-HEL F10 antibody alexa-488. Germinal centres were stained with PNA-biotin (Vector Labs) and streptavidin-alexa 633.

## RESULTS

We aspired to investigate the impact of targeting iNKT cell help to antigen specific B cells during the development of an immune response. To this end we have coated silica beads (100nm) with liposomes containing DOPC and PE-biotin in the presence of αGalCer (Fig. 1A). Alexa-488-labeled-αGalCer was used to quantify the amount of αGalCer loaded on the beads, and we found this to be 150ng per μl of beads ($10^8$ beads/μl). To assess the capacity of B cells to present αGalCer in vitro, B cells were stimulated for 20h with particulate or soluble αGalCer prior to their incubation with iNKT cells derived from a mouse hybridoma (DN32.D3). The secretion of IL-2 into the culture medium was used to measure iNKT cell activation. In agreement with previous reports (17), soluble αGalCer was efficiently presented by B cells and induced IL-2 production (Fig. 1B). In contrast, on stimulation with particulate αGalCer we observed a severe attenuation of IL-2 production from iNKT cells. Thus, B cells took up far less particulate lipid antigen through the non-selective manner observed for soluble αGalCer.

### CD1d-dependent presentation of particulate antigenic lipids to iNKT cells is enhanced by BCR-mediated uptake

We have bound biotinylated hen egg lysozyme (HEL), through a streptavidin linker to particles loaded with or without αGalCer. The total amount of bound protein was estimated using Western blotting (Fig. 1A). To assess the ability of B cells to mediate BCR-specific uptake and presentation of these particulates, we have used HEL-specific transgenic primary B cells (MD4) and DN32.D3 cells. MD4 B cells stimulated with particulate HEL-αGalCer induced strong iNKT activation (Fig. 1C). In contrast, IL-2 production was not detected following incubation with particulate HEL or αGalCer alone. In addition, no iNKT activation was observed following stimulation of WT B cells with particulate HEL conjugated with αGalCer. Interestingly, marginal zone MD4 B cells (purified by FACS sorting of the $CD21^{high}$ $CD23^{low}$ B cell population) were more efficient in presenting particulate HEL-αGalCer to iNKT cells than follicular B cells (Fig. 6). Importantly, the activation of iNKT cells is completely blocked by pre-incubating B cells with a monoclonal antibody against CD1d, indicating that this process is absolutely dependent on CD1d-mediated presentation (Fig. 1D). These observations demonstrate that specific antigen recognition by BCR can dramatically enhance presentation of particulate lipid antigen to iNKT cells.

To explore the effect of altering the antigen affinity we have utilized various HEL proteins representing a wide-range of affinities for the MD4 BCR ($HEL^{WT}$ $K_a$ = 2.1 x $10^{10}$ $M^{-1}$; $HEL^{RDGN}$ $K_a$ = 5.2 x $10^7$ $M^{-1}$; $HEL^{RKD}$ $K_a$ = 8.0 x $10^5$ $M^{-1}$) (18). As shown in Fig. 1 E and 1F, at the highest density of antigen we observed similar levels of iNKT cell activation regardless of the affinity of antigen conjugated with particulate αGalCer. Interestingly, even the low affinity $HEL^{RKD}$ antigen enhances the presentation particulate αGalCer to iNKTs. However, decreasing the density of this antigen on the particle surface 20 times abolished the presentation of particulate αGalCer (Fig. 1F). Thus these findings indicate that even very low affinity antigens can induce efficient particulate αGalCer presentation providing they exceed a tightly regulated avidity threshold for stimulation of the BCR.

Although BCR antigen recognition is necessary, it is not sufficient to drive optimal B cell presentation. We observed that transgenic B cells expressing a signalling-deficient BCR (IgM-H2) with high affinity for HEL, exhibited diminished ability to induce iNKT activation (Fig. 1G). In line with these findings particulate HEL conjugated with either αGalCer or Gal(α1→2)αGalCer gave rise to equally potent activation of iNKT cells (Fig. 1H). As Gal(α1→2)αGalCer requires intracellular processing before it can be effectively recognised by iNKT cells (19), it can be concluded that lipid-containing beads are internalised within B cells prior to presentation to iNKT cells.

In addition, the inventors have demonstrated the wider applicability of the described observations by using another iNKT cell agonist known as IMM47. Similar to αGalCer, we observed that particulate IMM47 when conjugated with antigen led to the stimulation of iNKT cells in vitro through production of IL-2 (Fig. 1I).

Thus, BCR-mediated antigen recognition and internalisation are required for B cell mediated presentation of particulate αGalCer to iNKT cells.

### BCR-mediated uptake of particulate αGalCer leads to recruitment of iNKT cell help resulting in extensive B cell proliferation and antibody production in vivo

Having demonstrated that BCR-mediated uptake of particulate antigenic lipid leads to iNKT cell activation in vitro, we investigated the impact of BCR-mediated uptake of particulate □GalCer on the activation and fate of B cells in vivo. To this end, CFSE-labelled HEL-specific B cells from MD4 mice were adoptively transferred into C57BL/6 recipients challenged intravenously with particulate HEL with or without conjugated αGalCer, in absence of any other adjuvant. Five days after stimulation, spleens of recipient mice were harvested and the dilution of CFSE in the HEL-specific B cell population was used as a measure of B cell proliferation.

As shown in Fig. 2A, extensive proliferation of the HEL-specific B cells in response to particulate HEL conjugated with αGalCer was observed - after five days MD4 B cells constitute more than 7% of the total splenic lymphocytes. These HEL-specific B cells originated from the adoptively transferred MD4 cells, as they were not detected following particulate HEL-αGalCer stimulation of C57BL/6. Importantly no proliferation was detected following challenge with either particulate αGalCer or HEL

alone, as HEL itself is incapable of eliciting a T-cell dependent response on the C57BL/6 background (20). In addition, HEL-specific B cell proliferation was dependent on the presence of iNKT cells, as it was not observed in similar adoptive transfer experiments using Jα18-/- mice as recipients (Fig.,2B).

Immunohistological analysis of spleen sections confirmed expansion of HEL-specific B cells in response to HEL-αGalCer particles (Fig. 2C). Interestingly, these expanded HEL-specific B cells were predominantly located as extrafollicular foci in the bridge channels and red pulp of the spleen and exhibited an intense cytoplasmic HEL staining characteristic of PCs (Fig. 2C). The presence of specific PCs was confirmed by flow cytometry, on the basis of high intracellular HEL staining and CD138 expression (Fig, 2D). PC differentiation was accompanied by the production of high IgMa anti-HEL antibodies titres (Fig. 2F). These antibodies were derived exclusively from the transferred MD4 B cells, as antibodies produced by C57BL/6 mice would be of the $IgH^b$ allotype. HEL-specific PCs or anti-HEL antibodies were not detected in mice challenged with particulate HEL or αGalCer alone (Fig. 2D and F). Antibody secreting cells were also identified by HEL-specific ELISPOT only in recipients challenged with HEL-αGalCer containing particles (Fig. 2E). In addition, splenic $HEL^+$ $CD138^+$ cells were not present following particulate HEL-αGalCer stimulation of MD4 B cells adoptively transferred into Jα18-/- mice (Fig. 2D and F). Hence PC differentiation of HEL-specific B cells in response to particulate HEL-αGalCer was dependent on the presence of iNKT cells.

Notably, and in line with our in vitro observations, B cell proliferation and antibody production were dependent on the avidity of the BCR for the antigen present on the particles (Fig. 3A-C). A reduction of the affinity or density of HEL on the particulate αGalCer resulted in diminished B cell proliferation and antibody production. Thus an avidity threshold for the BCR-mediated internalisation of particulate αGalCer is also present in vivo. However it is evident that even low affinity antigen can efficiently induce αGalCer presentation to iNKTs, allowing stimulation of B cell responses.

Linking the protein antigen and lipid antigenic immunostimulant via the particles gave rise to greatly enhanced B cell proliferation and antibody production than observed for particulate HEL alone administered with soluble αGalCer (Fig. 3D-F). Significantly this indicates that specific BCR uptake of antigenic lipids represents a more efficient means of internalization than that employed by soluble αGalCer. Stimulation with particulate HEL-αGalCer results in greater stimulation of iNKT-mediated specific B cell responses. Thus we have identified a strategy involving particulate antigenic lipid to enable enhanced and specific B cell proliferation and development of functional extrafollicular PCs in vivo.

**Immunization with particulate antigen conjugated with αGalCer enhances specific antibody responses**

Given the impact of particulate αGalCer with conjugated antigen on B cell fate, we sought to investigate their ability to induce a systemic immune response in vivo. In order to assess this we have used a single-dose intraperitoneal immunization strategy, employing chicken gamma globulin (CGG) as antigen. It has been demonstrated previously that CGG induces strong T cell-dependent responses in mice. C57BL/6 and Jα18 -/- mice were challenged with particulate CGG conjugated with αGalCer, and after 7 and 14 days specific antibody responses were analyzed using ELISA.

We detected specific anti-CGG antibody production, comprising high titers of IgM and class switched IgG, as early as 7 days after immunization of C57BL/6 mice with particulate CGG conjugated with αGalCer (Fig. 4A). No specific antibodies were detected at this time point in mice immunized with particulate CGG or αGalCer alone. Immunization of both C57BL/6 and Jα18 - /- mice with particulate CGG gave rise to similar specific antibody responses throughout the measured time-course after immunization (Fig. 4B). In contrast, immunization with particulate CGG conjugated with αGalCer induced a dramatic increase in specific CGG antibody production in C57BL/6 mice compared with Jα18-/- mice. This demonstrates the requirement of iNKT for specific antibody production in response to particulate αGalCer conjugated with antigen. Importantly, the same pattern of class-switched antigen-specific antibodies observed using CGG as antigen, was detected in response to immunization of C57BL/6 mice with particulate HEL conjugated with αGalCer (Fig. 4C). Hence, following immunization iNKT cells can efficiently activate specific antibody production and class switch without the recruitment of specific CD4+ helper T cells.

Thus far we have demonstrated that specific BCR recognition is required for the efficient B cell presentation of particulate antigenic lipids to iNKT cells, and that the recruitment of iNKT cell help can modulate B cell activation. However, we were keen to assess the impact of co-uptake of antigen and αGalCer in the production of specific antibodies during the development of a systemic immune response. To address this we immunized C57BL/6 mice with two different combinations of particles: A first group of mice was immunized with particulate CGG conjugated with αGalCer alongside particulate ovalbumin (OVA), while a second group received particulate OVA conjugated with αGalCer alongside particulate CGG (Fig. 5A). Increased levels of specific antibodies were generated in each group in response to the antigen conjugated with αGalCer, showing that co-uptake of antigen-αGalCer enhances specific B cell activation and antibody production (Fig. 5A). Notably we also observed an enhancement in the production of specific antibodies in response to immunizations with particulate CGG conjugated with αGalCer compared with particulate

CGG and soluble αGalCer (Fig. 5B). This result, in line with our in vivo proliferation experiments, demonstrates that BCR-mediated uptake of particulate αGalCer is more efficient than that of soluble αGalCer uptake, resulting in enhanced specific B cell responses.

**Example 2 - TLR agonists**

**MATERIALS AND METHODS** (where different from those described in Example 1)

**Materials**

Biotinylated CpG OD1668 with phosporothioate bond was purchased from Sigma. Streptavidin coated polystyrene microspheres (130nm) were purchased from Bangs Laboratories.

**B cell purification and in vitro proliferation**

Splenic B cells were enriched by negative selection to >99% purity using B cell purification kit (Miltenyi Biotec). MD4 B cells were labelled with 2μm CFSE, cultured at $1x10^6$ cells and incubated with particles containing HEL and/or CpG. After 72h, cells were harvested and subjected to Flow cytometry analysis. The supernatant was collected and IL-6 and HEL specific IgMa secretion was determined by ELISA.

**Particulate antigen-CpG stimulates B cell proliferation and differentiation in vitro**

We have used the immunostimulant CpG, an agonist of TLR9, as a model system for triggering of intracellular TLR responses. First we wanted to investigate if antigen-BCR mediated uptake was required for particulate CpG to be internalized. Therefore we coated streptavidin polystyrene micrsopheres comparable to the size of a virus (130nm) with the biotinylated model antigen hen egg lysozyme (HEL) in the presence or absence of biotinylated CpG. We have utilised streptavidin polystyrene beads, comparable in diameter to that of a typical viral pathogen, in order to investigate the mechanism by which particulate CpG initiates TLR9-mediated B cell responses. The successful coating of the particles with HEL was demonstrated by staining with the HEL-specific monoclonal antibody F10 (Fig. 11A), by flow cytometry and by detection of HEL with polyconal antibody by Western blotting. The presence of CpG was assessed by competition of HEL on the surface of the beads and thereby it appears as a reduction of F10 binding to HEL CFSE-labelled MD4 HEL-specific transgenic B cells (Goodnow et al., 1988) were harvested three days after in vitro stimulation with HEL and CpG coated beads. Flow cytometry was used to monitor B cell proliferation and PC differentiation, through dilution of CFSE and up-regulation of CD138 expression respectively. In addition, IL-6 secretion, associated with TLR9 stimulation (Barr et al., 2007), and IgM secretion upon plasma cell differentiation were detected in the supernatant of the cultures.

Following stimulation with beads containing HEL and CpG, MD4 B cells were observed to undergo extensive proliferation and plasma cell differentiation, as demonstrated by their CFSE dilution and CD1138 upregulation (Fig. 7A, left panel). This correlated with secretion of IL-6 and IgMa by the B cells as detected in the supernatant (Fig. 7A, middle and right panels). Importantly, on stimulation with beads containing either HEL alone or CpG alone, no proliferation or plasma cell differentiation was observed (Fig. 7A).

It is therefore evident that the uptake of particulate CpG is dependent on antigen-mediated internalisation by the BCR in order to be available for its receptor. Furthermore the co-engagement of TLR9 in B cells results in proliferation and differentiation to PCs.

**Antigen avidity influences the response of B cells to particulate antigen-CpG in vitro**

In Example 1, the response of B cells to BCR stimulation by particulate antigen was demonstrated to be dependent on the overall antigen avidity so we examined the influence of antigen avidity on B cell proliferation and differentiation following stimulation with beads containing HEL and CpG in vitro. We have utilised three HEL mutants covering a range of BCR affinities, as described previously (Batista and Neuberger, 1998): high affinity mutant $HEL^{RD}$ ($K_a$ 8 x $10^8$ $M^{-1}$); intermediate affinity mutant $HEL^{KD}$ ($K_a$ 4 x $10^6$ $M^{-1}$) ; and low affinity mutant $HEL^{RKD}$ ($K_a$ 8 x $10^5$ $M^{-1}$). To ensure the amounts of antigen immobilised to the beads were comparable, biotinylated F10 was used as a linker for various HEL antigens to streptavidin beads. In addition, we also generated beads with different densities by including various concentrations of biotinylated CGG during the initial coating phase to compete with biotinylated F10 for binding to the streptavidin beads (Fig. 11B).

We observed that MD4 B cells stimulated with beads containing high or intermediate affinity HEL and CpG underwent substantial proliferation and differentiation (Fig. 8A). However following stimulation with beads containing low affinity HEL and CpG, MD4 B cells did not secrete either IL-6 or IgMa. It therefore appears that a threshold of antigen affinity is required in order to trigger BCR-mediated internalisation and stimulate TLR9-dependent B cell proliferation and PC differentiation. Having observed this antigen affinity threshold we examined the dependence of the B cell response on the density of antigen coated on the beads. In the case of the high and intermediate affinity antigen, we observed that B cell proliferation and differentiation (Fig. 7C and data not shown), and the production of IgMa and IL-6 (Fig. 7C) were dependent on the antigen density. At the lowest antigen affinity, no B cell proliferation and differentiation (Fig. 7C and data not shown), or IgMa and IL-6 could be detected, even at the highest density of antigen-coated beads.

Thus we have demonstrated the importance of antigen avidity, in terms of both affinity and density, in determining the extent of TLR9-mediated B cell proliferation and differentiation in vitro. We suggest therefore that the overall strength of interaction between BCR and antigen must exceed a defined threshold for efficient uptake of beads and following TLR9 engagement to take place.

**Particulate antigen-CpG stimulates B cell proliferation and differentiation in vivo**

As we have observed TLR9-mediated B cell proliferation and plasma cell differentiation by stimulation with particulate antigen-CpG in vitro, we wanted to ascertain if similar B cell behaviour was induced following administration of particulate antigen-CpG in vivo. To address this CFSE-labelled MD4 B cells and microspheres containing HEL and CpG were co-administered to wild type recipient mice. Four days after the adoptive transfer, splenic B cells were analysed as above for CFSE dilution. Plasma cells were revealed by CD138 upregulation and high intracellular binding for HEL as described previously. In addition HEL specific IgMa levels were determined in the serum.

We observed that co-injection of MD4 B cells and beads containing both HEL and CpG, led to extensive proliferation of HEL-specific B cells (Fig. 8A). Furthermore, approximately 65% of these cells formed PCs as determined by flow cytometry. Their presence was confirmed by IgMa secretion measured in the serum (Fig. 2A right panel). In contrast, co-injection of MD4 B cells and beads containing HEL alone was not sufficient to stimulate B cell proliferation or differentiation into PCs in the absence of specific T cell help Fig. 8A). In addition, stimulation with particulate CpG alone was not able to trigger B cell proliferation and differentiation. Hence we have demonstrated that the BCR-mediated internalisation of particulate CpG is required to promote PC differentiation in vivo.

**Antigen avidity influences the response of B cells to particulate antigen-CpG in vivo**

A relationship was observed between the overall avidity of antigen and the B cell response following stimulation with particulate antigen and CpG in vitro, hence we used the various HEL and CpG beads generated above to stimulate CFSE-labelled MD4 B cells in vivo.

We observed extensive B cell proliferation following stimulation with beads containing CpG conjugated with either the high or intermediate affinity HEL mutant (Fig. 9A upper panel). This resulted in similar levels of PC differentiation, as demonstrated by the up-regulation of CD138, intracellular, HEL staining and production of IgMa (Fig. 9A and 9B). In contrast, the beads containing low affinity HEL mutant and CpG gave rise to a far less prominent stimulation of B cell proliferation and a three-fold reduction in $CD138^+$ HEL-specific PCs (Fig. 9A). As expected, co-injection of B cells with beads containing antigen alone or CpG alone did not lead to any observable B cell proliferation, regardless of the affinity of the antigen (data not shown). Reducing the antigen density of the high affinity HEL mutant from high to intermediate in the presence of CpG did not change dramatically the extent of proliferation and PC differentiation (Fig. 9C upper panel). However, a further reduction in density results in a reduction in the percentage of PCs formed (Fig. 9B and D, upper panels). The impact of antigen density is more apparent using the lower affinity HEL antigens, indicating that a certain threshold exists, above which beads containing antigen and CpG can be taken up and hence proliferation and differentiation is stimulated. It appears that this antigen avidity threshold observed in vivo is lower than that observed in vitro, presumably this is a consequence of the more favourable environment for B cell proliferation and differentiation in vivo.

Taken together, we observed that the avidity of the antigen-BCR interaction determines the extent of the particulate antigen-CpG TLR9-mediated B cell proliferation and plasma cell differentiation response in vivo.

**Immunization with particulate antigen conjugated with CpG enhances specific antibody responses**

Given the impact of particulate CpG with conjugated antigen on B cell fate, we sought to investigate their ability to induce a systemic immune response in vivo. In order to assess this we have used a single-dose intraperitoneal immunization strategy, employing chicken gamma globulin (CGG) as antigen. It has been demonstrated previously that CGG induces strong T cell-dependent responses in mice. C57BL/6 mice were challenged with particulate CGG conjugated with CpG, and after 7 and 14 days specific antibody responses were analyzed using ELISA.

We detected specific anti-CGG antibody production, comprising class switched IgG, as early as 7 days after immunization of C57BL/6 mice with particulate CGG conjugated with CpG (data not shown). No specific antibodies were detected at this time point in mice immunized with particulate CGG or CpG alone. At day 14, IgG could be detected by immunization with CGG alone, the titers however where ten times higher if both CGG and CpG were present on the microspheres. Interestingly, IgG of the subtype IgG1 could be detected upon immunization with either antigen alone or antigen and CpG whereas class switch to the subclasses IgG2b and IgG2c only occurred if particles were coated with both CGG and CpG (Fig10A).

Thus far we have demonstrated that specific BCR recognition is required for the efficient intracellular TLR stimulation in B cells. However, we were keen to assess the impact of co-uptake of antigen and CpG in the production of specific antibodies during the development of a systemic immune response. To address this we immunized C57BL/6 mice with two different combinations of particles: A first group of mice was immunized with particulate CGG conjugated with CpG alongside particulate ovalbumin (OVA), while a second group received particulate OVA conjugated with CpG alongside particulate CGG (Fig. 10B). Increased levels of specific antibodies were generated in each group in response to the antigen conjugated with CpG, showing that co-uptake of antigen and CpG enhances specific B cell activation and antibody production.

**Example 3**

**Immunization with particulate phospho-peptide conjugated with αGalCer enhances specific antibody response**

The inventors sought to investigate the ability of particulate phospho-peptide-αGalCer conjugates to induce a systemic immune response in vivo. In order to assess this the inventors have used a single-dose intraperitoneal immunization strategy, employing phosphopeptide as antigen. C57BL/6 mice (3 group) were challenged with particulate phospho-peptide conjugated with aGalCer (10 μl/mouse) or particulate phospho-peptide alone and specific antibody responses were analyzed using ELISA at days 0 and 7 after immunization.

Material and Methods

**Antigens, lipid preparation and microsphere coating**

For the preparation of liposomes containing 1,2-Dioleoyl-sn-Glycero-3-Phosphocholine (DOPC) and N-Cap biotinyl-phosphatidylethanolamine (PE-biotin) (both Avanti Polar Lipids), DOPC/PE-biotin (98/2, m/m) or DOPC/PE-biotin/aGalCer (88/2/10, m/m/m), lipids were dried under argon and resuspended in 25 mM Tris, 150 mM NaCl, pH 7.0 with vigorous mixing. αGalCer was purchased from Alexis Biochemical. For coating, silica microspheres (100nm; Kisker GbR) were incubated with liposomes followed by streptavidin and biotinylated proteins.
The phopspo-peptide had the sequence: Biotin-GDTTST(phospho)FCGTPNY-amide

**Immunizations and ELISA**

WT C57BL/6 mice were purchased from Charles River. Mice were immunized intraperitoneally with 10μl of beads containing phospho-peptide and/or aGalCer, and levels of sera antigen-specific Ig levels were determined by ELISA.

Sera antibodies were measured by ELISA by using BSA-peptide coated plates. Different dilutions of mice sera were added to the plates, washed and peptide specific IgM and IgG antibodies in mice sera were detected by using biotin-labelled goat anti-mouse IgM or IgG for detection (BD Pharmingen).

Results

The inventors detected specific anti-peptide antibody production, comprising high titers of IgM and IgG, as early as 7 days after immunization of C57BL/6 mice with particulate phospho-peptide conjugated with αGalCer (Fig. 14). No specific antibodies were detected at this time point in mice immunized with particulate phospho-peptide alone.

**Example 4**

**Materials and Methods**

HEL and OVA (Sigma-Aldrich); CγG (Jackson Immuno Research); CFSE (Invitrogen); 5' biotinylated CpG 1668 (Sigma-Genosys); and recombinant IL-6 (BD Biosciences). HELRD, HELK, HELKD and HELRKD were described previously35. 0.13μm streptavidin-coated microspheres (Bangs Inc) and 0.2μm FluoSpheres Neutravidin microspheres (Invitrogen).

**Antibodies**

Monoclonal antibodies against mouse Ags: Anti-CD138-PE (Clone 281-2) and -biotin; Anti-CD45.2-PerCP-Cy5.5 (10G); Anti-IL-6 (MP5-20F3); Anti-IL-6-biotin (MP5-32C11); Anti-CD16/32 (2.4G2); Anti-IgMa-biotin (DS-1); Anti-IgG1-biotin (A85-1); Anti-IgG2b-biotin (R12-3); Anti-IgG3-biotin (R40-82); and Anti-CD45R/B220 (RA3-6B2) (all BD Biosciences). Monoclonal anti-HEL F10 has been described36. Polyclonal Anti-mouse-IgG-HRP (Pierce); monoclonal Anti-β-Actin (AC-15) and monoclonal Anti-Chicken Egg Albumin (OVA-14) (both Sigma Aldrich); Anti-Phospho-p38 MAP Kinase (Thr180/Tyr182) and Anti-p38 MAP Kinase Antibody (both Cell Signalling). The polyclonal antibodies against mouse Ags: Anti-IgM-biotin; Anti-IgG-biotin; Anti-IgG2c-biotin; Anti-IgM; Anti-IgG; Anti-IgG1; Anti-IgG2b; Anti-IgG2c and Anti-IgG3 (all Southern Biotech Inc). Anti-Chicken Lysozyme (United States Biological), AlexaFluor-488 goat anti-mouse IgG(H+L) and AlexaFluor- 546 goat anti-rat IgG(H+L) (both Invitrogen).

**Microsphere coating**

Streptavidin-coated microspheres were washed prior to addition of biotinylated CpG and/or biotinylated Ag (OVA, HEL or CγG) and resuspended in PBS. To generate microspheres coated with HEL mutants at various densities, biotinylated anti-HEL F10 and/or biotinylated CpG was used for initial coating, as described previously36.

### B-cell purification, labelling and culture

Splenic B cells were enriched by negative selection to >99% purity using a B cell purification kit (Miltenyi Biotec) and labelled with 2μM CFSE. B cells were cultured in RPMI-1640 media (Invitrogen) supplemented with 10% FCS (PAA Labs), 50 μM β-mercaptoethanol (Sigma-Aldrich), 25mM Hepes (Invitrogen) and 10 units/ml Penicillin/Streptomycin (Invitrogen). 1x106 CFSE labelled B cells stimulated with 1μl particulates were harvested after 72h incubation to assess proliferation and differentiation.

### DC purification and culture

Bone marrow-derived DCs were generated by culturing precursors from mice femurs in the media described above supplemented with recombinant GMCSF (R&D Systems). After 5 days, DCs were enriched to >99% purity using CD11c+ microbeads (Miltenyi Biotec).

### Adoptive transfer and immunizations

1-5x106 CFSE-labelled MD4 or HyHe110 B cells together with 1-10μl particulates containing HEL and/or CpG were adoptively transferred by tailvein injection into WT C57BL/6 mice. Mice were immunized intraperitoneally with 1-10μl particulates coated with OVA or CγG (as Ag) and/or CpG.

### Flow cytometry

Detection of proliferation and PC differentiation in the spleen was based on a method described previously40. Briefly, single cell suspensions of the spleen were prepared and samples were blocked with purified anti-CD16/32. HELspecific B cells were detected with HEL followed with anti-HEL F10-AlexaFluor-647. PCs were identified through their binding to anti-CD138-PE.

To detect intracellular HEL binding, B were fixed in PFA and permeablized using 0.1% saponin. The extent of cellular particulate uptake stimulated with 1μl/106 cells fluorescent particulates was assessed by flow cytometry after 3h.

### Immunological assays and immunohistochemistry

ELISAs were used to quantify the production of sera antigen-specific IgMa and IgG and IL-6, in a manner similar to that described previously36. ELISPOT plates coated with 100μg/ml HEL or CγG in PBS, were blocked and incubated with splenocytes as described36 prior to development with either anti-IgG-HRP or anti-IgMa-biotin. Immunohistochemistry of splenic sections was performed as described previously36.

### Results

### Direct linkage of CpG to Ag enhances specific antibody responses following in vivo immunization

The inventors initially sought to investigate the impact that direct conjugation of Ag and CpG has on humoral immune responses in vivo. To achieve this they have designed an approach involving the direct conjugation of both Ag and CpG onto streptavidin polystyrene beads, comparable in diameter to that of a typical viral pathogen. The successful generation of particulate conjugates, following coating with biotinylated Ag and/or CpG, was confirmed by flow cytometry **(Figure 19A).** C57BL/6 mice were immunized simultaneously with two particulate Ags, chicken-γ-globulin (CγG) and ovalbumin (OVA). For each group of mice immunized one of the particulate Ags was conjugated with CpG. The production of Ag-specific IgG antibodies was measured by ELISA 14 days after particulate administration. Selective enhancement in Ag-specific antibody titres following immunization was observed for the particulate Ag carrying conjugated CpG (Ag-CpG). This enhanced response to particulate Ag-CpG was accompanied by the production of class-switched Ag-specific antibodies, predominantly of the IgG2b and IgG2c isotypes. Interestingly antibodies of the IgG2 isotype are particularly effective mediators of immune responses associated with virus neutralization41, and their production has been associated with TLR9 stimulation. Furthermore, the inventors observed Ag-specific antibody secreting cells (ASCs) within the bone marrow for up to three, months after single-dose immunization with particulate CγG-CpG **(Figure 13).** Thus we have demonstrated that immunization with particulates directly linked to both Ag and CpG enhances Ag-specific antibody titres and induces classswitching mainly to the IgG2 subtype in vivo.

### BCR-mediated uptake of Ag-CpG conjugates is required to stimulate TLR-dependent differentiation into PCs in vitro

To elucidate the mechanism underlying the enhancement in specific antibody titres, the inventors have employed transgenic MD4 B cells expressing BCR specific for hen egg lysozyme (HEL). CFSE-labelled MD4 B cells were stimulated with particles containing HEL (as Ag) and/or CpG in vitro **(Figure 19B).** Three days after stimulation, flow cytometry was used to monitor B-cell proliferation and PC differentiation by dilution of CFSE and up-regulation of CD138 expression respectively. Extensive proliferation of MD4 B cells, together with differentiation to form PCs was observed after stimulation with particulate HEL-CpG. These B-cell responses correlated with secretion of both IL-6 and HEL-specific IgMa, in line with previous reports. Similar results were obtained with particulates of various sizes, provided their diameter did not exceed 0.5μm (data not shown). Interestingly, no proliferation or differentiation was observed upon stimulation with particulates containing either HEL or CpG alone.

To identify the critical features of the particulates that enable stimulation of Bcell responses, the inventors used flow cytometry to detect cellular uptake of fluorescently-labelled particulates. While B cells were able to take up particulate HEL-CpG, we observed that they could not capture particulate CpG alone **(Figure 19C).** Thus particulates containing CpG alone cannot stimulate non-specific B-cell responses in the same manner as has been observed for soluble CpG. The failure of particulate CpG to enter B cells is not due to a general exclusion of these particulates from cells, as the uptake of CpG by dendritic cells is not impaired by conjugation to particulates **(Figure 19C).** However, we observed that Ag on the particulate enables entry into the B-cell **(Figure 19C)** and suggesting that the BCR is involved in the mechanism utilised to allow the entry of these conjugates into B cells.

**A tight-Ag-BCR avidity threshold regulates Ag-CpG stimulated B-cell activation in vitro**

Previous observations have suggested that binding strength of the Ag-BCR interaction influences the outcome of B-cell differentiation5. As antigen recognition by the BCR is required for particulate Ag-CpG to stimulate B-cell responses, the inventors examined the influence of Ag avidity, in terms of both affinity and density, on TLR9-dependent proliferation and differentiation in vitro. To address this the inventors utilised a number of previously described recombinant HEL mutants that bind the BCR with diminished affinity: wild type HEL (Ka 2 x 1010 M-1); HELRD (Ka 7.9 x 108 M-1); HELK (Ka 8.7 x 106 M-1); HELKD (Ka 4.0 x 106 M-1); and HELRKD (Ka 0.8 x 106 M-1). The various HEL proteins were immobilised to the particulates through the biotinylated monoclonal anti-HEL F10 as a bridge to ensure comparable coating densities. Decreasing the affinity of HEL by around 5000-fold (Ka from 2 x 1010 to 4.0 x 106 M-1) had little impact on either B-cell proliferation or IL-6 production when Ag was present on the particulate CpG at high density **(Figures 21A and B).** However a further 5-fold reduction in HEL affinity (to Ka 0.8 x 106 M-1) dramatically reduced the capacity of the CpG particulates to stimulate B-cell activation. It is therefore apparent that a minimum threshold of Ag affinity must be surpassed in order to trigger BCRmediated Ag internalization, associated B-cell proliferation and IL-6 secretion. Observing such a threshold in Ag affinity suggests that the response induced by particulate Ag-CpG is dependent on the amount of particles taken up by the B cells. To corroborate this hypothesis we used fluorescent particles to compare the capacity of B cells to acquire the different HEL conjugates. As shown in **Figure 14,** above the affinity threshold the amount of particles taken up were comparable throughout a widerange of affinities (from Ka 2 x 1010 to 4.0 x 106 M-1). In contrast, below this threshold we observed a marked reduction in the amount of particles acquired by the B cells. Therefore it is evident that above a tight threshold, the affinity of Ag for the BCR influences the amount of Ag-CpG particulates internalized.

As the binding strength of the Ag-BCR interaction is dependent on the avidity of Ag seen by the BCR, we postulated that further discrimination of stimulatory capacity might be observed by reducing the density of HEL on particulates. To generate particulates containing reduced densities of HEL we included an irrelevant biotinylated protein during the initial coating phase to compete with biotinylated F10 for binding to the streptavidin microspheres. As expected, stimulation with CpG particulates containing reduced densities of the various HEL proteins gave rise to lesser amounts of B-cell proliferation and IL-6 secretion. A 2-fold reduction in the density of HELRKD on CpG-containing particulates yielded them unable to stimulate B-cell proliferation and IL-6 production. Similarly, a 4-fold reduction in the density of the higher affinity HELRD protein also rendered CpG-containing particulates unable to stimulate B-cell responses. Interestingly the avidity threshold of the BCRAg interaction to induce IgMa secretion appears lower than that required for stimulation of proliferation and IL-6 production. It is therefore likely that Ag must be present at a sufficient avidity to induce the minimum degree of BCR clustering required for internalization of particulate Ag-CpG. Thus even a low affinity Ag present at sufficiently high density might surpass the threshold required to enable BCR-mediated internalization and associated stimulation of B-cell responses. Thus the inventors have demonstrated the importance of Ag avidity in dictating the extent of TLR9-mediated B-cell proliferation and differentiation in vitro. We suggest therefore that the overall strength of the interaction between BCR and Ag must exceed a defined threshold to allow for efficient of uptake of particulates and subsequent TLR9 stimulation by particulate CpG.

**The extent of B-cell differentiation by particulate Ag-CpG is determined by the strength of TLR9 stimulation in vitro**

As the total amount of particulate Ag-CpG taken up by B cells is dependent on the overall avidity of the Ag-BCR interaction, it is likely that the CpG component of the particulate impacts the extent of B-cell activation induced. The inventors therefore sought to investigate the effect of varying the amount of CpG conjugated to particulate HEL on the B-cell responses induced. To achieve this, particulates were coated with different amounts of CpG but the same overall amount of HEL to ensue constant Ag avidity and thus BCR-mediated entry to the cell. As shown in **Figure 15,** a reduction in the amount of CpG present on the particles correlated directly with a decrease in B-cell proliferation, and secretion of IL-6 and IgMa. As such, particulate HEL containing the largest amount of conjugated CpG stimulated differentiation to form PCs to the greatest extent. However, B-cell differentiation was scarcely detectable following stimulation with particulates containing a 10-fold reduction in amount of CpG conjugated.

It therefore is evident that the amount of CpG acquired by B cells is critical for the generation of EF PCs in response to particulate Ag-CpG indicating that the extent of signalling through TLR9 is important in determining the outcome of B-cell differentiation.

**Particulate HEL-CpG stimulates differentiation to form EF PC in vivo**

The inventors were keen to ascertain if their in vitro observations were representative of B-cell responses to Ag-CpG particulates in vivo. To assess this CFSE-labelled MD4 B cells and particles containing HEL and/or CpG were co-administered to wild-type recipient mice. The proliferation of MD4 splenic B cells was assessed by CFSE dilution at indicated time points after adoptive transfer. In addition the presence of CD138+ intracellular HEL+ MD4 splenic PCs was quantified using multicolour flow cytometry.

Extensive proliferation of HEL-specific B cells was observed upon co-injection of MD4 B cells and particulate HELRD-CpG **(Figure 16A, left panel).** This proliferation reached a maximum three days after stimulation **(Figure 16B)** and was coincident with the formation of PCs and the appearance of HEL-specific IgMa in the serum **(Figure 16A** middle and right panels; **Figure 16C).** This CD138+ PC population appeared short-lived in nature, as their number peaked around three to four days after stimulation. In line with this, similar kinetics were observed for the population of HEL-specific PCs formed in the EF region of the spleen **(Figure 16D).** Furthermore these responses were not detected on stimulation of wild type MD4 B cells with either particulate CpG or HEL alone **(Figure 16A).** As such, and in agreement with the inventors' in vitro observations, the sequential engagement of BCR and TLR9 is required to stimulate robust B-cell responses in vivo. Thus they have demonstrated that particulate HEL-CpG stimulates the TLR9-mediated proliferation and differentiation of B cells to short-lived EF PCs in vivo. Analogous to the in vitro findings, they observed a threshold in the strength of the BCR-Ag interaction requires to be surpassed in order to induce B-cell activation in vivo. As such, a greater than 2000-fold reduction in HEL affinity (from Ka 2 x 1010 to 8.7 x 106 M-1) does not diminish B-cell responses stimulated. However a further 2-fold decrease in Ag affinity leads to severely impaired B-cell proliferation and differentiation following stimulation by HEL-CpG particulates. Furthermore particulates coated with a lower density of HEL continue to enable B-cell proliferation and differentiation, albeit at slightly reduced levels. In contrast particulates containing a low density of HELK are unable to yield significant B-cell responses. Thus provided a lower affinity Ag is present at sufficient density, particulates can be used to stimulate B-cell proliferation and differentiation in vivo. These observations demonstrate the requirement for a threshold of BCR binding strength to be surpassed prior to TLR9-mediated stimulation of B-cell responses to particulate Ag-CpG in vivo. Finally, we were keen to establish the impact of varying the amount of TLR9 stimulation on the outcome of B-cell differentiation in vivo. As shown in **Figures 17D,** the extent of B-cell proliferation and formation of HELspecific PCs was dependent directly on the density of CpG conjugated with the particulate HEL. Hence, as in vitro, the amount of TLR9 stimulation is important in determining B-cell responses in vivo, and could potentially be useful as a mechanism for fine-tuning the outcome of stimulation with Ag-CpG particulates. Taken together, it is evident that following BCR-mediated internalization, particulate Ag-CpG conjugates stimulate B-cell proliferation and differentiation to form EF PCs in vivo, therefore establishing the physiological significance of our in vitro observations.

**Particulate HEL-CpG stimulates the production of Ag-specific classswitched antibodies**

The inventors' observations indicated that immunization of mice with particulate Ag-CpG led to the production of antigen-specific class-switched antibodies. However, as the transgenic MD4 B cells utilized in our investigations thus far are unable to undergo class-switch, we have employed an alternative transgenic model system to further investigate this phenomenon. This transgenic mouse system yields B cells expressing the high-affinity HyHEL10 BCR and able to undergo class-switch recombination. HyHEL10 B cells adoptively transferred into a wild-type recipient underwent extensive proliferation and differentiation to form PCs in response to particulate HEL-CpG, in a manner similar to that observed for MD4 B cells (data not shown). Analysis of the isotype of antibodies secreted revealed class-switch recombination occurred following stimulation with particulate HEL-CpG, resulting in the production of IgG predominantly of the IgG2 subtype **(Figure 18E).** Importantly, as a similar pattern of antibody isotypes was detected following simulation of HyHEL10 B cells alone in culture, the class-switch we observed in vivo was likely to be independent of CD4+ T cell help **(Figure 17F).** These findings are consistent with our observations from the original immunizations performed. Thus it is evident that, following BCR-mediated internalization, particulate Ag- CpG stimulates not only B-cell proliferation and differentiation to form shortlived PCs, but also class-switch recombination to the IgG2 isotype.

Discussion

The TLR9 agonist CpG has the capacity to stimulate a plethora of responses associated with activation of both the innate and adaptive branches of the immune system. Here the inventors have established that the direct conjugation of CpG with Ag gives rise to enhanced

and specific B-cell responses. This study involved developing a strategy to generate particulates with both Ag and CpG immobilized on the surface, to enable their uptake by B cells through the BCR. The inventors observed that receptor-mediated uptake is characterised by a tightly-regulated avidity threshold and results in delivery of CpG to TLR9 intrinsic to the B-cell in an Ag-specific manner. Importantly particulate CpG alone is prohibited from utilizing non-specific means of entering B cells, rendering particulate Ag-CpG highly selective in its capacity to stimulate TLR9-mediated responses. Furthermore the inventors have shown that following BCR mediated uptake, TLR9 engagement triggers a dramatic enhancement in B cell proliferation and formation of short-lived EF PCs. Several previous investigations into the impact of TLR9 stimulation on B-cell behaviour have employed soluble CpG. Such studies report that stimulation of TLR9 leads to the enhanced proliferation of B cells and differentiation to form PCs capable of producing isotype-switched antibodies. Here we observed that particulate CpG, unlike soluble CpG, cannot enter B cells via non-specific fluid-phase pinocytosis. In contrast, the conjugation of CpG to a particulate did not prevent its uptake by dedicated phagocytes such as dendritic cells. These phagocytes retain the capacity to acquire particulates potentially through pattern recognition receptors associated with innate immune cell function. In contrast B cells require the presence of an Ag-specific and signalling-competent BCR to enable efficient uptake of particulate Ag-CpG. The necessity of the BCR in the internalization of immune complexes containing TLR9-stimulatory ligands has been demonstrated during the development of autoimmune diseases.

The inventors have demonstrated that the avidity of the Ag-BCR interaction influences the outcome of B-cell activation following stimulation with particulate Ag-CpG. Early investigations of T cell-dependent B-cell responses introduced the notion that the decision of activated B cells to differentiate into either PCs or GCs is a stochastic process. However two more recent studies have utilized a variety of Ag and BCR affinities to investigate the impact of the overall BCR-Ag avidity on the outcome of B-cell differentiation. As Ags that induced greater signalling through the BCR preferentially drive B cells to become EF PCs, Brink's group have proposed an elegant model whereby the signalling strength of the BCR-Ag interaction controls the outcome of B-cell differentiation5. Here, above a defined threshold, the inventors observed a correlation between the avidity of the Ag-BCR interaction, the internalization of particulates and the amount of differentiation to form EF PCs. Thus it appears that following internalization, TLR stimulation may override the BCR-dependent signalling to determine the outcome of Bcell differentiation. Furthermore, a similar mechanism may underlie previous observations following stimulation with NP as Ag in the presence of adjuvant. The inventors therefore suggest that the results presented here are not contrary, but rather complementary, to that of the previous Ag-BCR avidity studies, in that, as anticipated by the Brink study 5, the differentiation of B cells is controlled through a combination of factors. The concept of combinatorial signalling functioning to shape the outcome of B-cell activation has been suggested previously31. Indeed the authors demonstrated that the sustained survival and differentiation of naive human B cells required engagement of the BCR with Ag, the availability of T cell help and signalling through the TLR system.

Here the inventors have shown that, provided the avidity threshold required for BCR mediated internalization is met, stimulation of intracellular TLR9 by particulate Ag-CpG influences the outcome of B-cell differentiation. Furthermore, using an adoptive transfer strategy, they have shown conclusively that B-cell proliferation and differentiation to form short-lived PCs is dependent on stimulation of intrinsic TLR9 in vivo. As such, increasing the extent of TLR9- mediated stimulation, by increasing the amount of CpG conjugated to the particulate Ag, enhances the generation of EF PCs in a quantitative manner.

These observations are in agreement with previous investigations that have identified a critical role for intrinsic TLR-mediated signalling in the differentiation of activated B cells. Moreover, it has been demonstrated very recently that TLR-mediated signalling, but not CD4+ T cell help, is absolutely required for the activation and EF development of auto-reactive B cells48. Interestingly, and in support of our observations, it has been found that TLR9-mediated signalling can be dissociated temporally from initial stimulation of the BCR31. The ability of TLR-mediated signalling to override BCR-dependent signals and stimulate the production of EF PCs is likely to play an important role during the early stages of the immune response through the rapid production of first-wave protective antibodies. Following BCR-mediated uptake, the inventors observed that particulate Ag-CpG gains access to stimulate TLR9 within the B-cell. Previous studies have demonstrated that BCR stimulation results in formation of an intracellular complex formed by the fusion of many endosomal-like vesicles49. This complex is the site where internalized receptors become localised, and appears similar to the autophagosome-like compartment rich in TLR9 observed after BCR stimulation20. The functional significance of directed localization of endocytosed BCR together with associated Ag was first appreciated through the demonstration that newly-synthesized MHC-II molecules were also located within these endosomal compartments50. Accordingly BCR-mediated internalization facilitates processing and efficient loading of Ag onto MHC-II for subsequent presentation to specific CD4+ helper T cells necessary for full B-cell activation. Furthermore it has been demonstrated that the MHC-like molecule CD1d acquires lipidic Ags, such as αGalCer, within endosomal compartments prior to its surface presentation to iNKT cells. A similar mechanism of BCR-mediated internalization was required for the stimulation of specific iNKT-mediated Bcell proliferation and differentiation to EF PCs in response to particulate Ag-αGalCer36. These observations taken together implicate endosomal or endosomal-like compartments as sites critical for the co-ordination of intracellular communications that ultimately govern the outcome of cellular processes such as differentiation. We demonstrate here the selective and controlled stimulation of Ag-specific humoral immune responses through the use of particulate Ag-CpG conjugates. As these particulates provide a means of directing the immunostimulatory capacity of CpG to a specific population of cells, they are of enormous value as effective adjuvants in the future design of successful vaccination strategies. As such, the use of CpG in this particulate form is envisaged to guard against the development of autoimmune diseases associated with non-specific TLR9-stimulation and lymphoid follicle destruction53 associated with repeated administration of soluble CpG. In addition, variation of their precise composition, particulate Ag-CpG could be used to offer intricate control of the immune responses stimulated on immunization. Indeed inclusion of multiple Ags within the particulate CpG conjugates could potentially allow for more effective induction of protective immune responses. The inventors have developed an approach for the direct conjugation of Ag and the immunostimulant CpG on the surface of a particulate. These particulates gain selective entry into Ag-specific B cells through BCR mediated endocytosis, allowing engagement of intracellular TLR9. Stimulation with these particulates results in enhanced B-cell proliferation and differentiation to form EF PCs competent to secrete Ag-specific classswitched antibodies in vivo. Investigations employing these particulates are useful not only in elucidating principles concerning the involvement of TLR9 during the development of the primary immune responses, but also in advancement of Ag-specific immunostimulants required in vaccinations.

**REFERENCES**

1. Rajewsky K (1996) Clonal selection and learning in the antibody system. Nature 381: 751-758.
2. Reth M, Wienands J (1997) Initiation and processing of signals from the B cell antigen receptor. Annu Rev Immunol 15: 453-479.
3. Kurosaki T (2002) Regulation of B cell fates by BCR signaling components. Curr Opin Immunol 14: 341-347.
4. Lanzavecchia (1985) Antigen-specific interaction between T and B cells. Nature 314: 537-539.
5. MacLennan I, Toellner K, Cunningham A, Serre K, Sze D, Zúñiga E, Cook M, Vinuesa C (2003) Extrafollicular antibody responses. Immunol Rev 194: 8-18.
6. Moller (1987) Role of somatic mutation in the generation of lymphocyte diversity. Immunological reviews 96: 1-162.
7. Amigorena S, Drake J, Webster P, Mellman I (1994) Transient accumulation of new class II MHC molecules in a novel endocytic compartment in B lymphocytes. Nature 369: 113-120.
8. Brigl M, Brenner M (2004) CD1: antigen presentation and T cell function. Annu Rev Immunol 22: 817-890.
9. Calabi F, Milstein C (2000) The molecular biology of CD1. Semin Immunol 12: 503-509.
10. Moody D, Porcelli S (2003) Intracellular pathways of CD1 antigen presentation. Nat Rev Immunol 3: 11-22.
11. Bendelac A, Savage P, Teyton L (2007) The biology of NKT cells. Annu Rev Immunol 25: 297-336.
12. Godfrey D, Kronenberg M (2004) Going both ways: immune regulation via CD1d-dependent NKT cells. J Clin Invest 114: 1379-1388.
13. Van Kaer L (2007) NKT cells: T lymphocytes with innate effector functions. Curr Opin Immunol 19: 354-364.
14. Kawano T, Cui J, Koezuka Y, Toura I, Kaneko Y, Motoki K, Ueno H, Nakagawa R, Sato H, Kondo E, et al. (1997) CD1d-restricted and TCR-mediated activation of valpha14 NKT cells by glycosylceramides. Science 278: 1626-1629.
15. Spada F, Koezuka Y, Porcelli S (1998) CD1d-restricted recognition of synthetic glycolipid antigens by human natural killer T cells. J Exp Med 188: 1529-1534.
16. Galli G, Nuti S, Tavarini S, Galli-Stampino L, De Lalla C, Casorati G, Dellabona P, Abrignani S (2003) CD1d-restricted help to B cells by human invariant natural killer T lymphocytes. J Exp Med 197: 1051-1057.
17. Bezbradica J, Stanic A, Matsuki N, Bour-Jordan H, Bluestone J, Thomas J, Unutmaz D, Van Kaer L, Joyce S. (2005) Distinct roles of dendritic cells and B cells in Va14Ja18 natural T cell activation in vivo. J Immunol 174: 4696-4705.
18. Fleire SJ, Goldman JP, Carrasco YR, Weber M, Bray D, Batista FD (2006) B cell ligand discrimination through a spreading and contraction response. Science 312: 738-741.
19. Prigozy T, Naidenko O, Qasba P, Elewaut D, Brossay L, Khurana A, Natori T, Koezuka Y, Kulkarni A, Kronenberg M (2001) Glycolipid antigen processing for presentation by CD1d molecules. Science 291: 664-667.
20. Gammon G, Shastri N, Cogswell J, Wilbur S, Sadegh-Nasseri S, Krzych U, Miller A, Sercarz E (1987) The choice of T-cell epitopes utilized on a protein antigen depends on multiple factors distant from, as well as at the determinant site. Immunol Rev 98: 53-73.
21. van den Elzen P, Garg S, León L, Brigl M, Leadbetter E, Gumperz J, Dascher C, Cheng T, Sacks F, Illarionov P, et al. (2005) Apolipoprotein-mediated pathways of lipid antigen presentation. Nature 437:

906-910.

22. Vidard L, Kovacsovics-Bankowski M, Kraeft S, Chen L, Benacerraf B, Rock K (1996) Analysis of MHC class II presentation of particulate antigens of B lymphocytes. J Immunol 156: 2809-2818.

23. Batista F, Neuberger M (2000) B cells extract and present immobilized antigen: implications for affinity discrimination. EMBO J 19: 513-520.

24. Lin K, Freeman S, Zabetian S, Brugger H, Weber M, Lei V, Dang-Lawson M, Tse K, Santamaria R, Batista F, et al. (2008) The rap GTPases regulate B cell morphology, immune-synapse formation, and signaling by particulate B cell receptor ligands. Immunity 28: 75-87.

25. Mattner J, Debord K, Ismail N, Goff R, Cantu C, Zhou D, Saint-Mezard P, Wang V, Gao Y, Yin N, et al. (2005) Exogenous and endogenous glycolipid antigens activate NKT cells during microbial infections. Nature 434: 525-529.

26. Kinjo Y, Wu D, Kim G, Xing G, Poles M, Ho D, Tsuji M, Kawahara K, Wong C, Kronenberg M (2005) Recognition of bacterial glycosphingolipids by natural killer T cells. Nature 434: 520-525.

27. Galli G, Pittoni P, Tonti E, Malzone C, Uematsu Y, Tortoli M, Maione D, Volpini G, Finco O, Nuti S, et al. (2007) Invariant NKT cells sustain specific B cell responses and memory. Proc Natl Acad Sci USA 104: 3984-3989.

28. Tupin E, Kinjo Y, Kronenberg M (2007) The unique role of natural killer T cells in the response to microorganisms. Nat Rev Microbiol 5: 405-417.

29. Hansen D, Siomos M, De Koning-Ward T, Buckingham L, Crabb B, Schofield L (2003) CD1d-restricted NKT cells contribute to malarial splenomegaly and enhance parasite-specific antibody responses. Eur J Immunol 33: 2588-2598.

30. Kinjo Y, Tupin E, Wu D, Fujio M, Garcia-Navarro R, Benhnia M, Zajonc D, Ben-Menachem G, Ainge G, Painter G, et al. (2006) Natural killer T cells recognize diacylglycerol antigens from pathogenic bacteria. Nat Immunol 7: 978-986.

31. Brigl M, Bry L, Kent S, Gumperz J, Brenner M (2003) Mechanism of CD1d-restricted natural killer T cell activation during microbial infection. Nat Immunol 4: 1230-1237.

32. Salio M, Speak A, Shepherd D, Polzella P, Illarionov P, Veerapen N, Besra G, Platt F, Cerundolo V (2007) Modulation of human natural killer T cell ligands on TLR-mediated antigen-presenting cell activation. Proc Natl Acad Sci USA 104: 20490-20495.

33. Paget C, Mallevaey T, Speak A, Torres D, Fontaine J, Sheehan K, Capron M, Ryffel B, Faveeuw C, Leite de Moraes M, et al. (2007) Activation of invariant NKT cells by toll-like receptor 9-stimulated dendritic cells requires type I interferon and charged glycosphingolipids. Immunity 27: 597-609.

34. Roark J, Park S, Jayawardena J, Kavita U, Shannon M, Bendelac A (1998) CD1.1 expression by mouse antigen-presenting cells and marginal zone B cells. J Immunol 160: 3121-3127.

35. Martin F, Kearney J (2002) Marginal-zone B cells. Nat Rev Immunol 2: 323-335.

36. Yuan W, Qi X, Tsang P, Kang S, Illarionov P, Illaniorov P, Besra G, Gumperz J, Cresswell P (2007) Saposin B is the dominant saposin that facilitates lipid binding to human CD1d molecules. Proc Natl Acad Sci USA 104: 5551-5556.

37. Phan T, Amesbury M, Gardam S, Crosbie J, Hasbold J, Hodgkin P, Basten A, Brink R (2003) B cell receptor-independent stimuli trigger immunoglobulin (Ig) class switch recombination and production of IgG autoantibodies by anergic self-reactive B cells. J Exp Med 197: 845-860.

38. Chiron D, Bekeredjian-Ding I, Pellat-Deceunynck C, Bataille R and Jego G (2008) Toll-like receptors: lessons to learn from normal and malignant human B cells. Blood epub Jun 30

## Claims

**1.** A method for eliciting an antigen-specific immune response *in vitro,* the method comprising the step of contacting a B cell *in vitro* with a product comprising:

(i) a polymer or silica bead having a size of from 1 nm to 10 $\mu$m;
(ii) a B-cell receptor binding antigen attached to the surface of the bead, and
(iii) an immunostimulant attached to the surface of the bead, wherein the immunostimulant is $\alpha$-galactosylceramide ($\alpha$Gal-Cer) or a CpG oligodeoxynucleotide (ODN);

wherein the product is internalised by a B cell to elicit an antigen-specific BCR-mediated immune response to said B-cell receptor binding antigen

**2.** A method according to claim 1, wherein the bead is coated with liposome.

**3.** A method according to any one of the preceding claims, wherein the antigen and the immunostimulant are attached to the bead via a linker.

**4.** A method according to claim 3, wherein the linker is an avidin-biotin linker.

**5.** A method according to any one of the preceding claims, wherein the immunostimulant comprises at least two different immunostimulants.

**6.** A method according to any one of the preceding claims, wherein the antigen comprises at least two different antigens.

7. A method according to any one of the preceding claims, wherein the antigen comprises a protein, a polypeptide, a peptide, a phosphopeptide, a polysaccharide, such as a glycan, a polysaccharide-conjugate, a peptide or non-peptide mimic of a polysaccharide, a small molecule, a lipid, a glycolipid or a carbohydrate.

**Patentansprüche**

1. Verfahren zum Auslösen einer antigenspezifischen Immunreaktion in vitro, wobei das Verfahren den Schritt des Kontaktierens einer B-Zelle in vitro mit einem Produkt umfasst, das Folgendes umfasst:

   (i) ein Polymer- oder Silica-Kügelchen mit einer Größe von 1 nm bis 10 μm;
   (ii) ein B-Zellen-Rezeptorbindungsantigen, das an die Oberfläche des Kügelchens gebunden ist, und
   (iii) ein Immunstimulationsmittel, das an die Oberfläche des Kügelchens gebunden ist, worin das Immunstimulationsmittel α-Galactosylceramid (α-Gal-Cer) oder ein CpG-Oligodesoxynucleotid (ODN) ist;

   worin das Produkt von einer B-Zelle aufgenommen wird, um eine antigenspezifische BCR-vermittelte Immunreaktion auf das B-Zellen-Rezeptorbindungsantigen auszulösen.

2. Verfahren nach Anspruch 1, worin das Kügelchen mit Liposom beschichtet ist.

3. Verfahren nach einem der vorangegangenen Ansprüche, worin das Antigen und das Immunstimulationsmittel über einen Linker an das Kügelchen gebunden sind.

4. Verfahren nach Anspruch 3, worin der Linker ein Avidin-Biotin-Linker ist.

5. Verfahren nach einem der vorangegangenen Ansprüche, worin das Immunstimulationsmittel zumindest zwei verschiedene Immunstimulationsmittel umfasst.

6. Verfahren nach einem der vorangegangenen Ansprüche, worin das Antigen zumindest zwei verschiedene Antigene umfasst.

7. Verfahren nach einem der vorangegangenen Ansprüche, worin das Antigen ein Protein, ein Polypeptid, ein Peptid, ein Phosphopeptid, ein Polysaccharid wie ein Glycan, ein Polysaccharid-Konjugat, ein Peptid- oder Nicht-Peptid-Imitat eines Polysaccharids, ein kleines Molekül, ein Lipid, ein Glykolipid oder ein Kohlenhydrat umfasst.

**Revendications**

1. Procédé pour obtenir une réponse immunitaire spécifique à un antigène *in vitro,* le procédé comprenant l'étape consistant à mettre en contact une cellule B *in vitro* avec un produit comprenant

   (i) une bille de polymère ou de silice ayant une taille de 1 nm à 10 μm ;
   (ii) un antigène de liaison à un récepteur de cellules B fixé à la surface de la bille, et
   (iii) un immunostimulant fixé sur la surface de la bille, dans lequel l'immunostimulant est de l'α-galactosylcéramide (αGal-Cer) ou un CpG oligodéoxynucléotide (ODN) ;

   dans lequel le produit est internalisé par une cellule B pour obtenir une réponse immunitaire favorisée par BCR spécifique à un antigène dudit antigène de liaison au récepteur de cellules B.

2. Procédé selon la revendication 1, dans lequel la bille est revêtue d'un liposome.

3. Procédé selon l'une quelconque des revendications précédentes, dans lequel l'antigène et l'immunostimulant sont fixés sur la bille via un liant.

4. Procédé selon la revendication 3, dans lequel le liant est un liant d'avidine-biotine.

5. Procédé selon l'une quelconque des revendications précédentes, dans lequel l'immunostimulant comporte au moins deux immunostimulants différents.

6. Procédé selon l'une quelconque des revendications précédentes, dans lequel l'antigène comporte au moins deux antigènes différents.

7. Procédé selon l'une quelconque des revendications précédentes, dans lequel l'antigène comporte une protéine, un polypeptide, un peptide, un phosphopeptide, un polysaccharide, comme un glycane, un conjugué de polysaccharide, un imitateur peptidique ou non peptidique d'un polysaccharide, une petite molécule, un lipide, un glycolipide ou un hydrate de carbone.

Fig 1

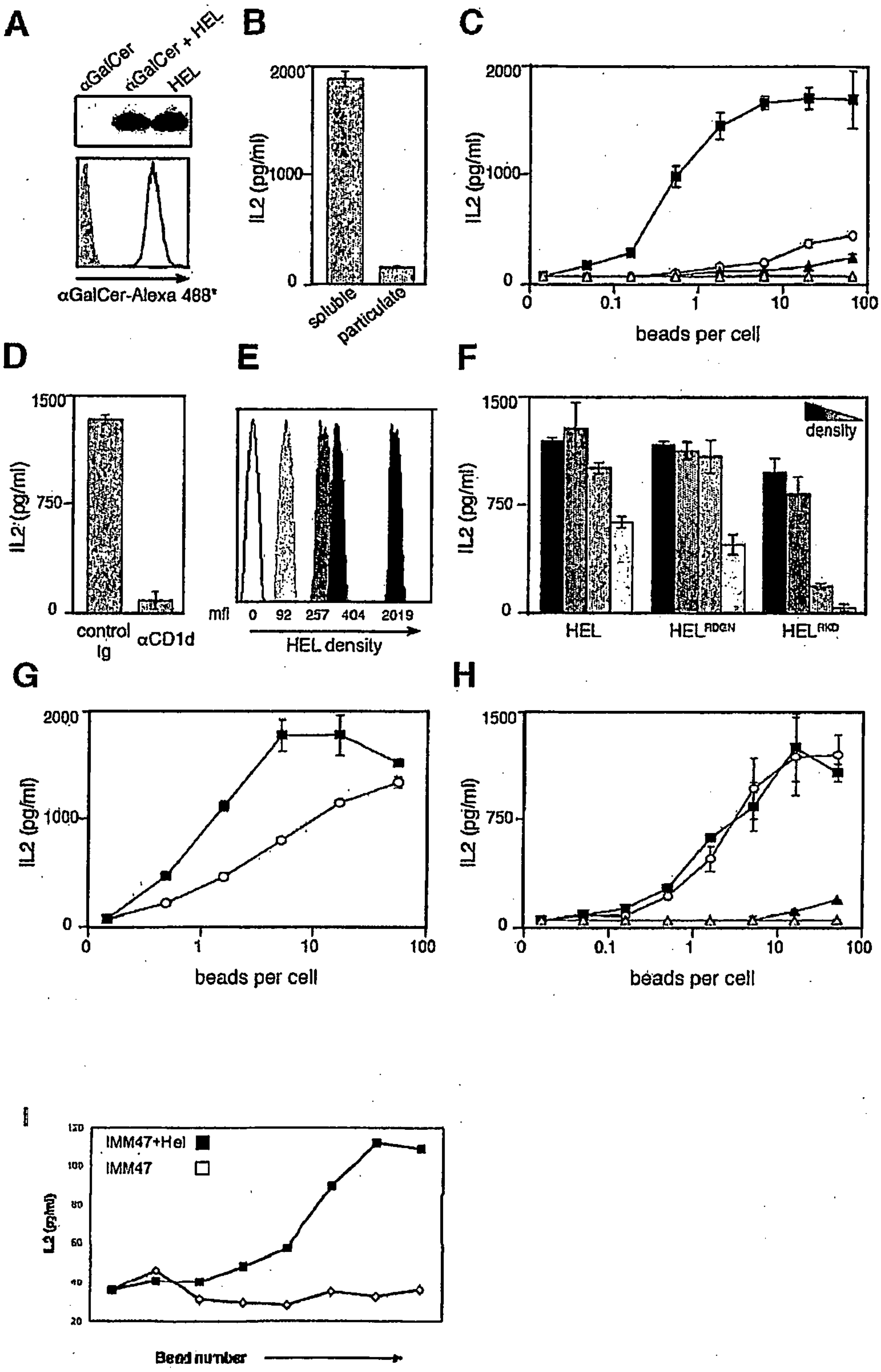
A
αGalCer
αGalCer + HEL
HEL
αGalCer-Alexa 488+
B
IL2 (pg/ml)
2000
1000
0
soluble
particulate
C
IL2 (pg/ml)
beads per cell
D
IL2 (pg/ml)
1500
750
0
control Ig
αCD1d
E
mfl 0 92 257 404 2019
HEL density
F
density
HEL
HEL^RDGN
HEL^RKD
G
beads per cell
H
beads per cell
I
IMM47+Hel
IMM47
IL2 (pg/ml)
Bead number

Fig 2

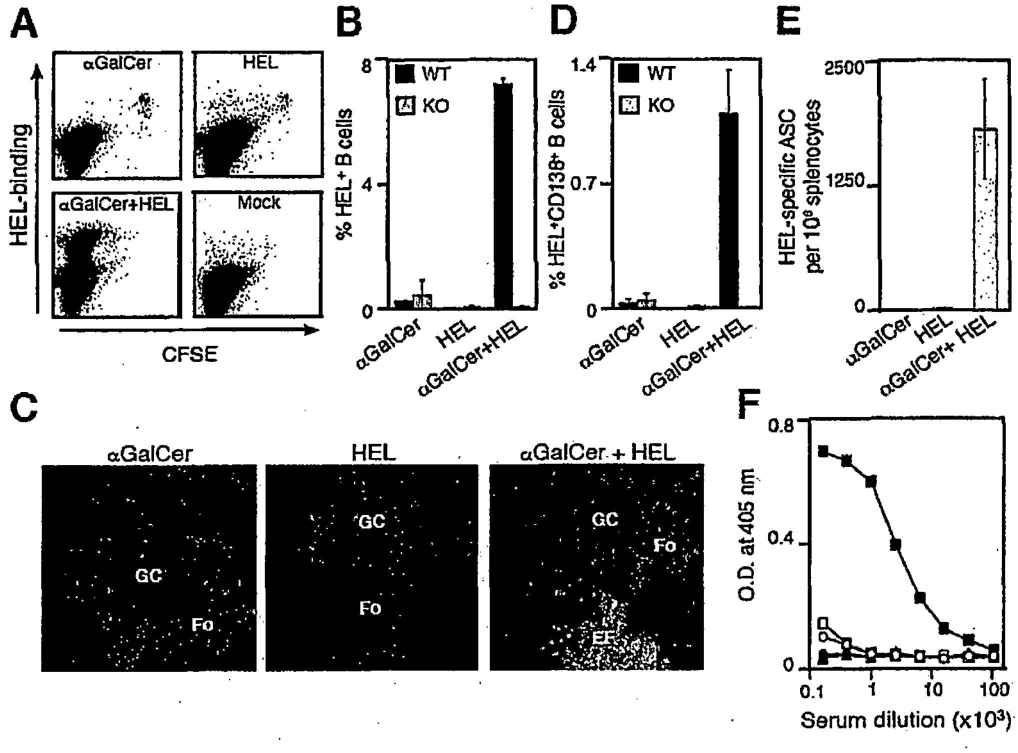
A
αGalCer
HEL
αGalCer+HEL
Mock
HEL-binding
CFSE
B
WT
KO
% HEL+ B cells
αGalCer
HEL
αGalCer+HEL
D
WT
KO
% HEL+CD138+ B cells
E
HEL-specific ASC per 10^6 splenocytes
αGalCer+ HEL
C
GC
Fo
EF
F
O.D. at 405 nm
Serum dilution (x10^3)

Fig 3

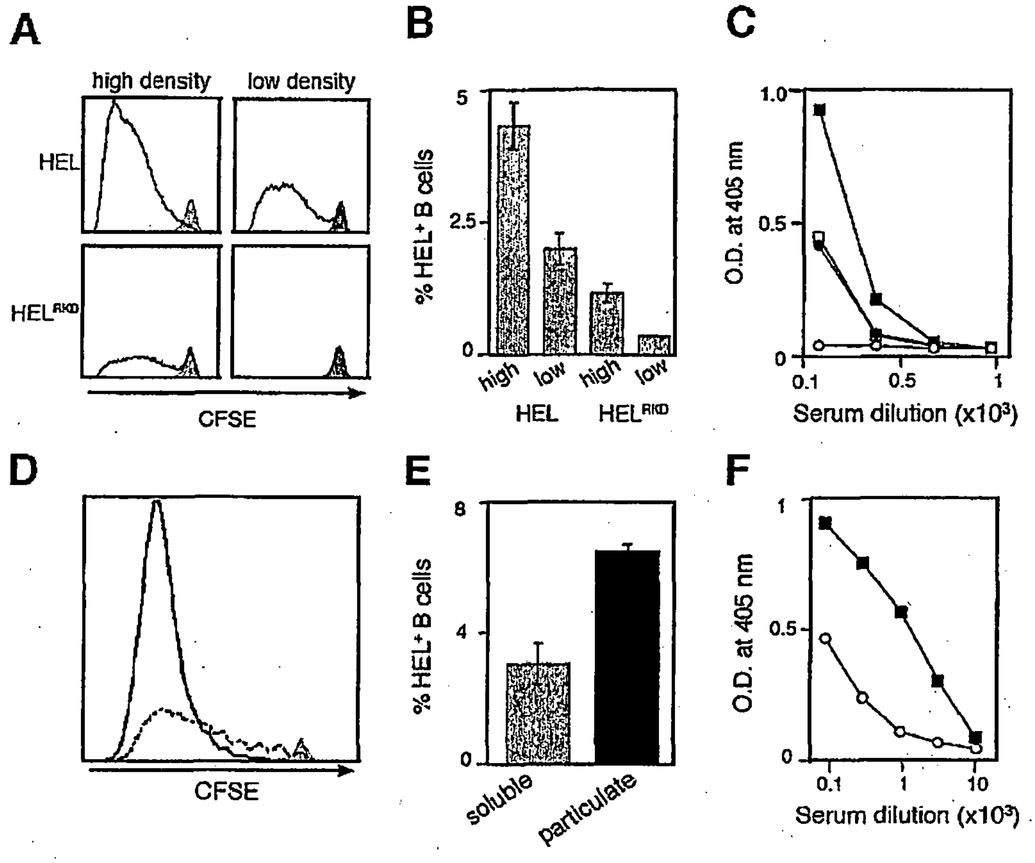
A
high density
low density
HEL
HEL^RKD
CFSE
B
% HEL+ B cells
5
2.5
0
high
low
high
low
HEL
HEL^RKD
C
O.D. at 405 nm
1.0
0.5
0
0.1
0.5
1
Serum dilution (x10³)
D
CFSE
E
% HEL+ B cells
8
4
0
soluble
particulate
F
O.D. at 405 nm
1
0.5
0
0.1
1
10
Serum dilution (x10³)

Fig 4

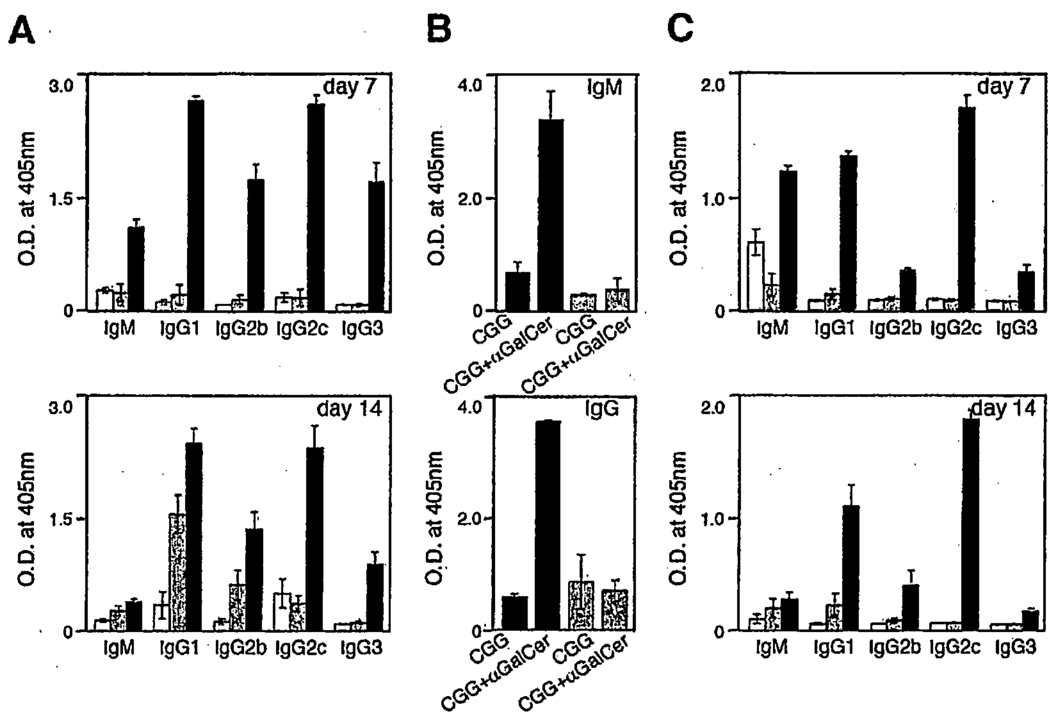
A
B
C
day 7
day 14
IgM
IgG
O.D. at 405nm
3.0
1.5
0
4.0
2.0
1.0
IgM IgG1 IgG2b IgG2c IgG3
CGG
CGG+αGalCer
CGG
CGG+αGalCer

Fig 5

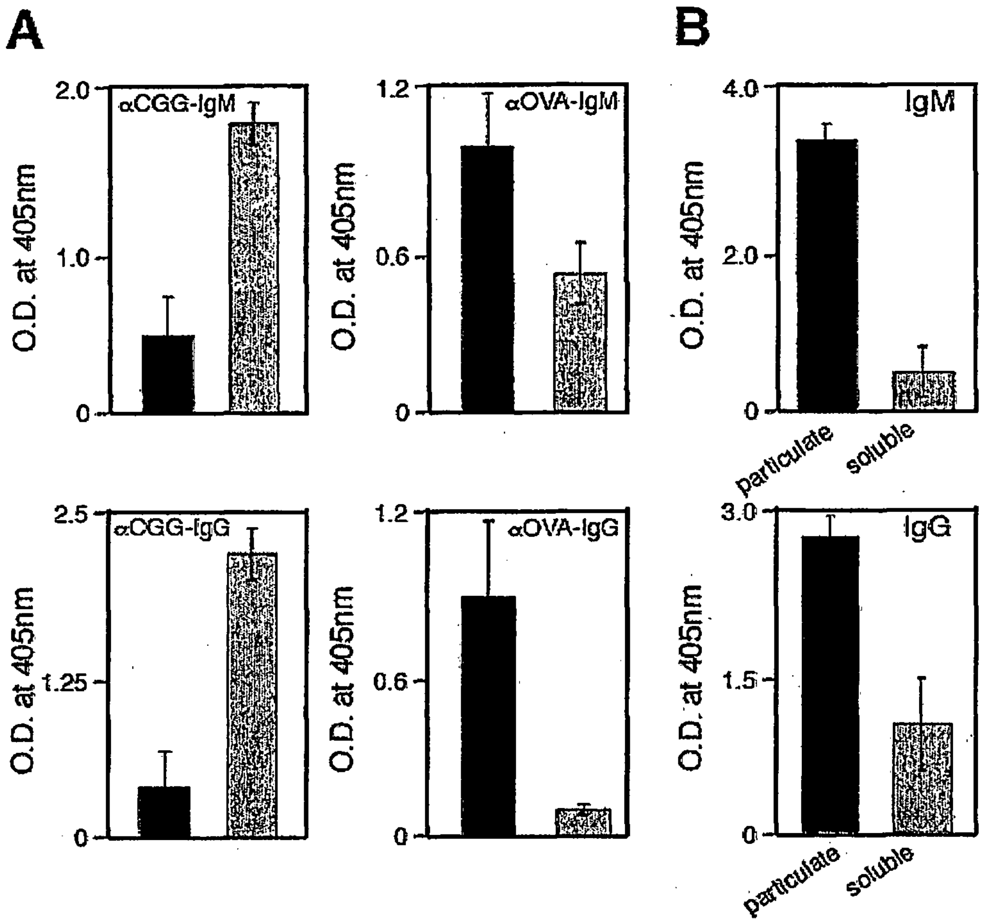
A
B
2.0
1.0
0
O.D. at 405nm
αCGG-IgM
1.2
0.6
0
αOVA-IgM
4.0
2.0
IgM
particulate
soluble
2.5
1.25
αCGG-IgG
αOVA-IgG
3.0
1.5
IgG

Fig 6

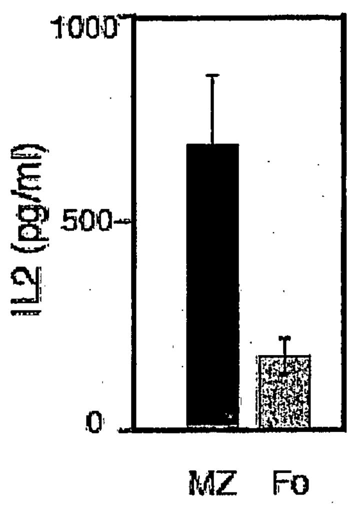

Fig 7

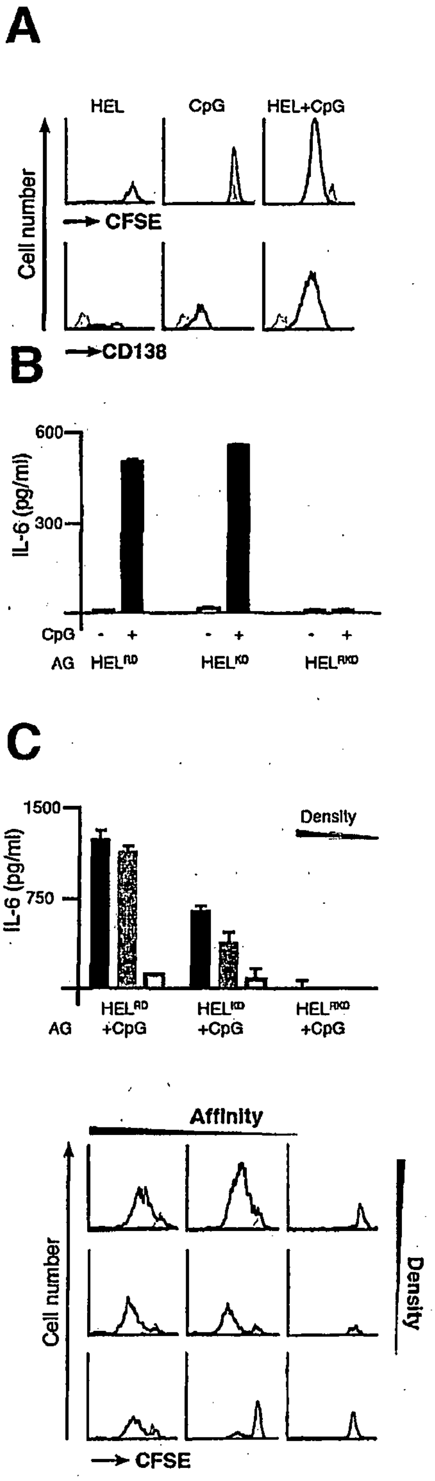
A
HEL
CpG
HEL+CpG
Cell number
CFSE
CD138
IL-6 (pg/ml)
600
300
HEL + - -
CpG - + -
HEL+CpG - - +
IgM (OD405nm)
4
2
B
IL-6 (pg/ml)
CpG - + - + - +
AG HEL^RD HEL^KD HEL^RKD
IgM (OD 405nm)
C
1500
750
Density
HEL^RD +CpG
HEL^KD +CpG
HEL^RKD +CpG
AG
Affinity
Density
Cell number
CFSE

Fig 8

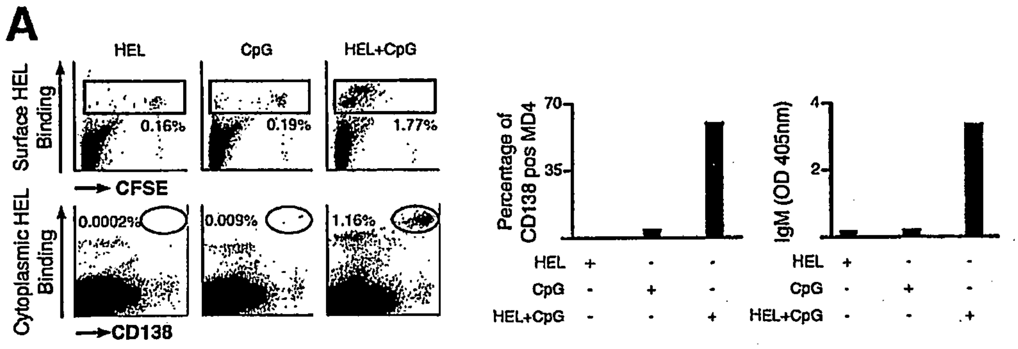
A
HEL
CpG
HEL+CpG
Surface HEL Binding
0.16%
0.19%
1.77%
CFSE
Cytoplasmic HEL Binding
0.0002%
0.009%
1.16%
CD138
Percentage of CD138 pos MD4
70
35
IgM (OD 405nm)
4
2
HEL + - -
CpG - + -
HEL+CpG - - +
HEL + - -
CpG - + -
HEL+CpG - - +

Fig 9

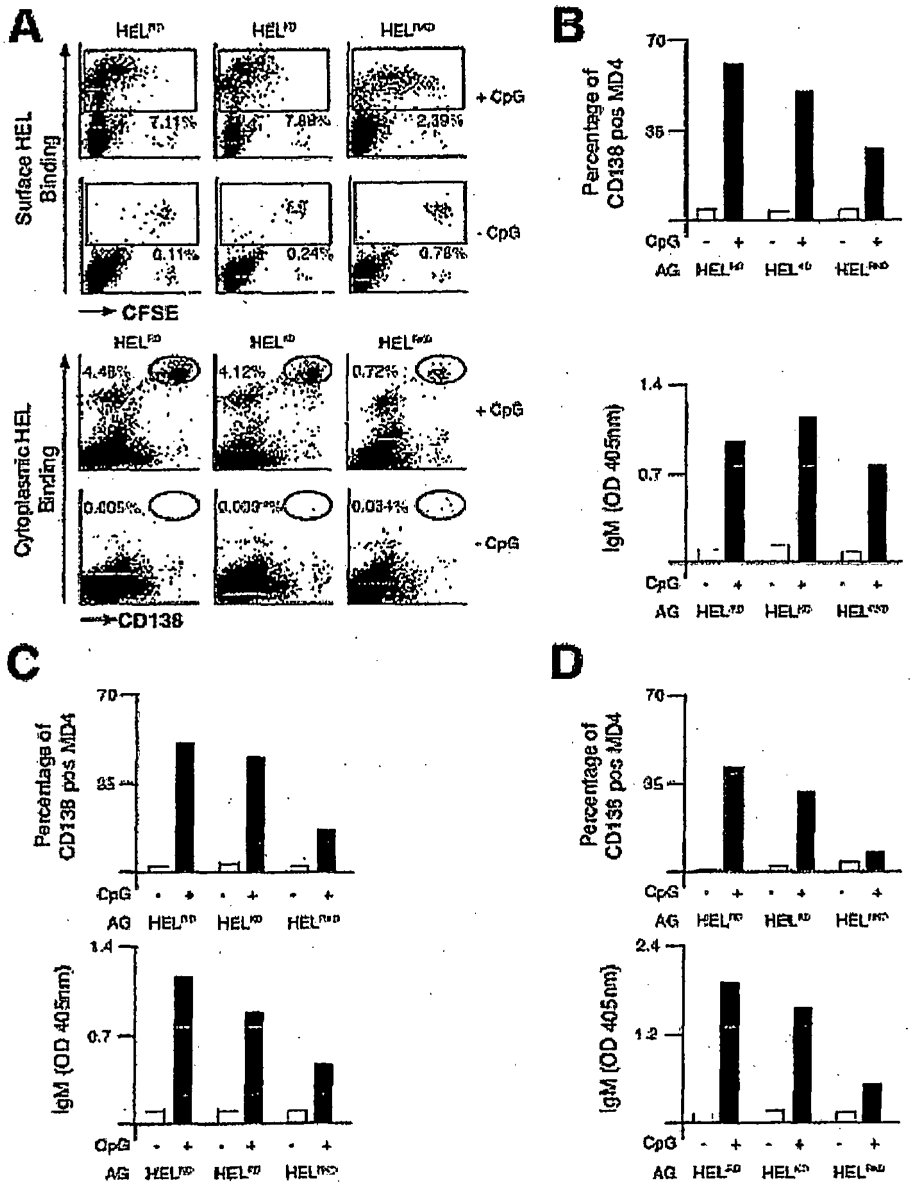
A
B
C
D
Surface HEL Binding
CFSE
Cytoplasmic HEL Binding
CD138
+CpG
-CpG
Percentage of CD138 pos MD4
IgM (OD 405nm)
CpG
AG

Fig 10

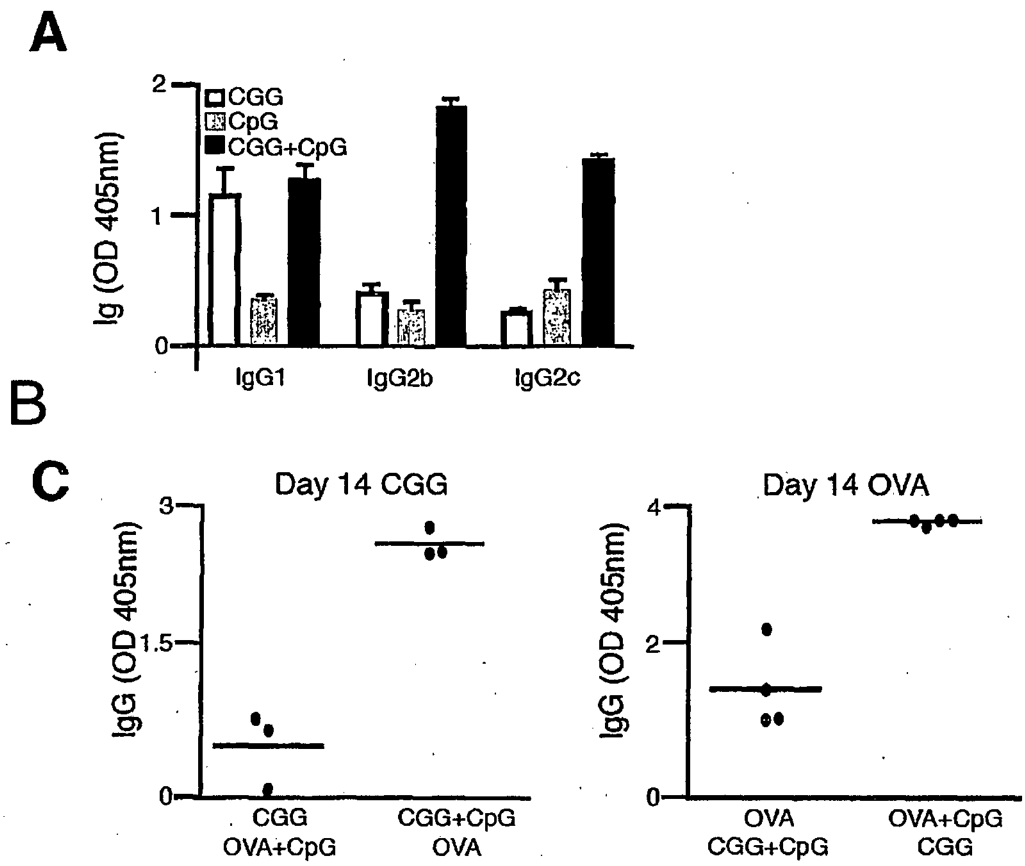
A
Ig (OD 405nm)
2
1
0
CGG
CpG
CGG+CpG
IgG1
IgG2b
IgG2c
B
C
Day 14 CGG
IgG (OD 405nm)
3
1.5
0
CGG
OVA+CpG
CGG+CpG
OVA
Day 14 OVA
IgG (OD 405nm)
4
2
0
OVA
CGG+CpG
OVA+CpG
CGG

Fig 11

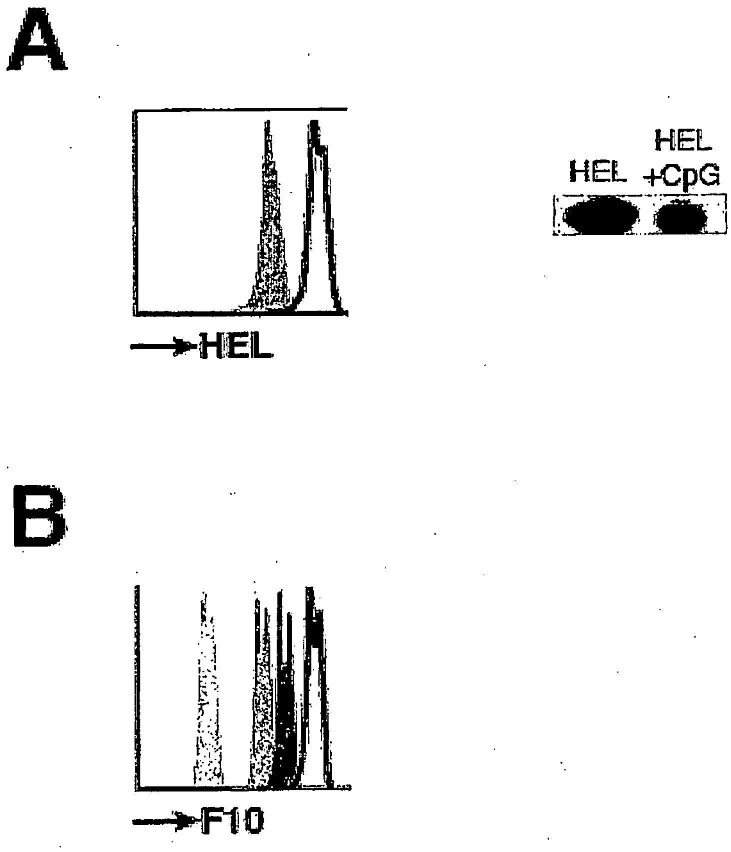

Figure 12

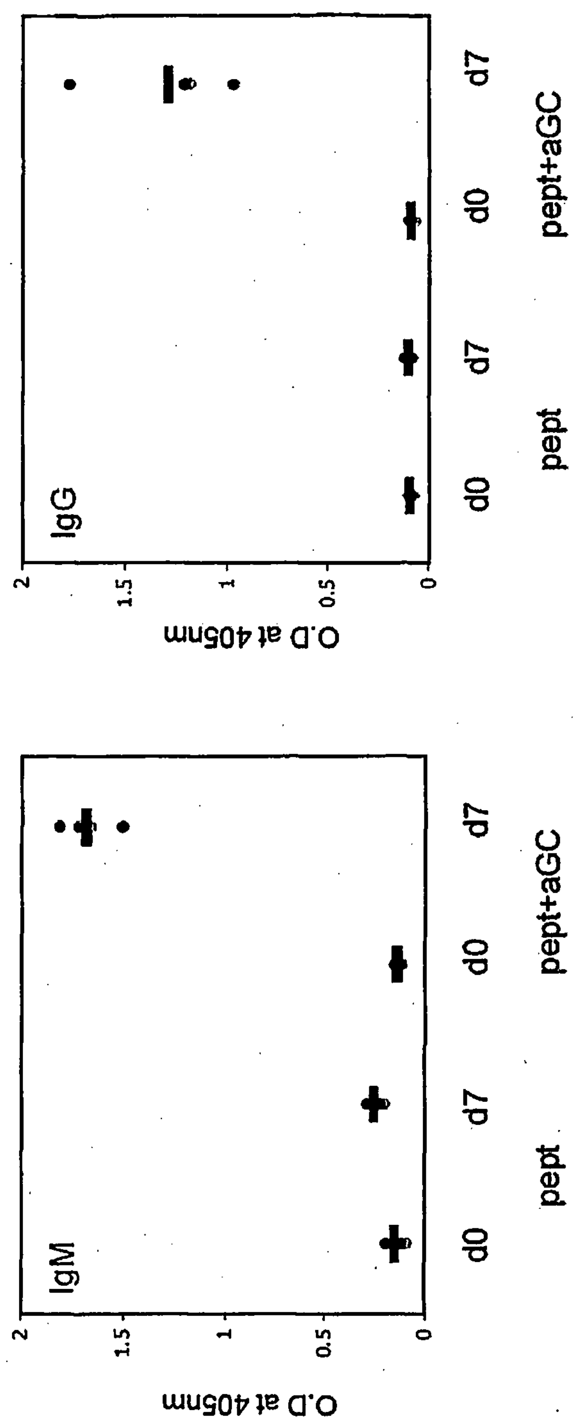

Fig 13

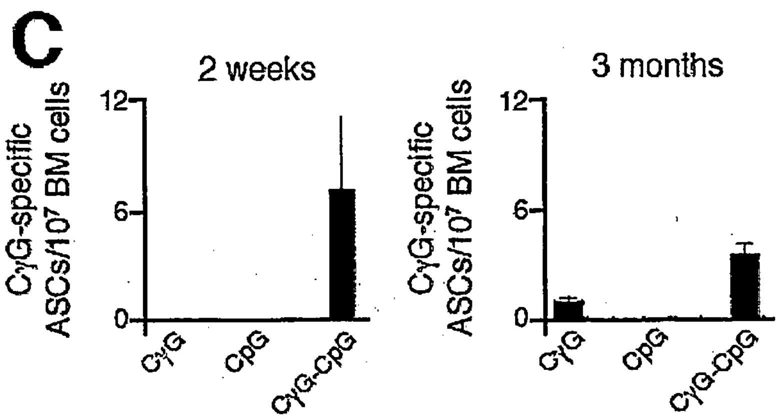
C
2 weeks
3 months
CγG-specific ASCs/10^7 BM cells
12
6
0
CγG
CpG
CγG-CpG

Fig 14

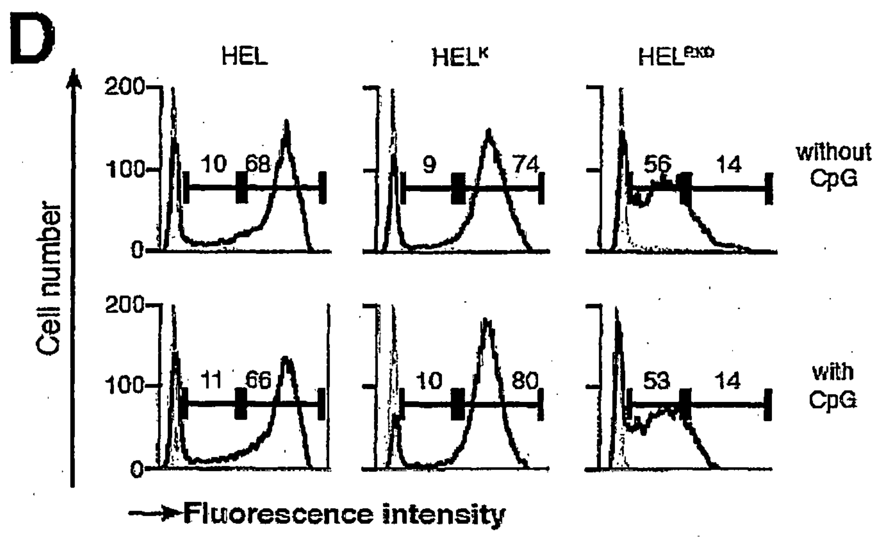
D
HEL
HEL^K
HEL^PKD
200
100
0
10
68
9
74
56
14
without CpG
11
66
10
80
53
14
with CpG
Cell number
Fluorescence intensity

Fig 15

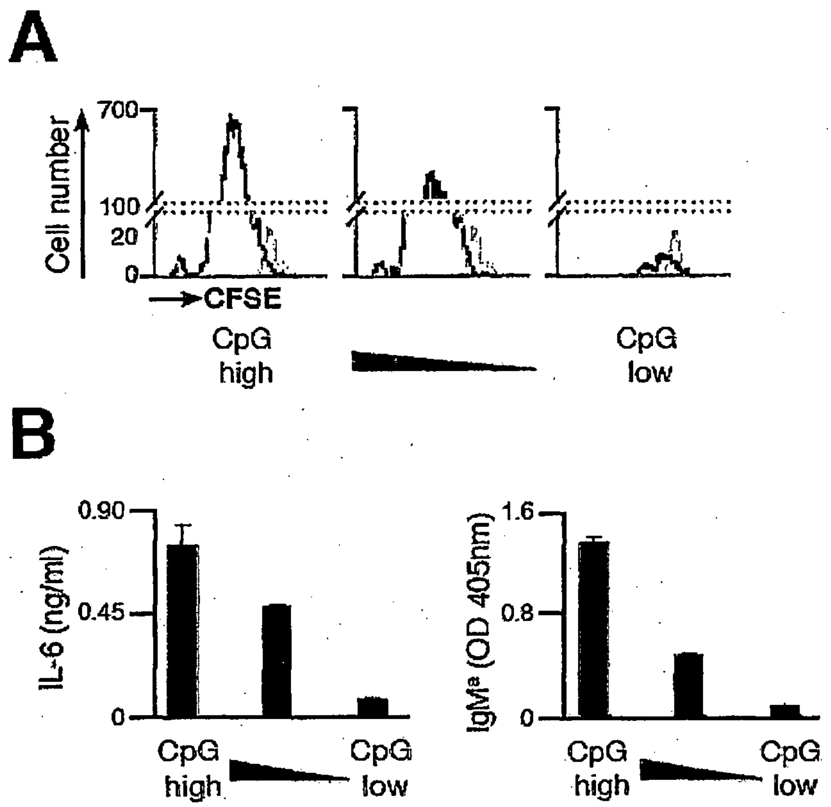
A
Cell number
700
100
20
0
CFSE
CpG
high
CpG
low
B
IL-6 (ng/ml)
0.90
0.45
0
CpG
high
CpG
low
IgMa (OD 405nm)
1.6
0.8
0
CpG
high
CpG
low

Fig 16

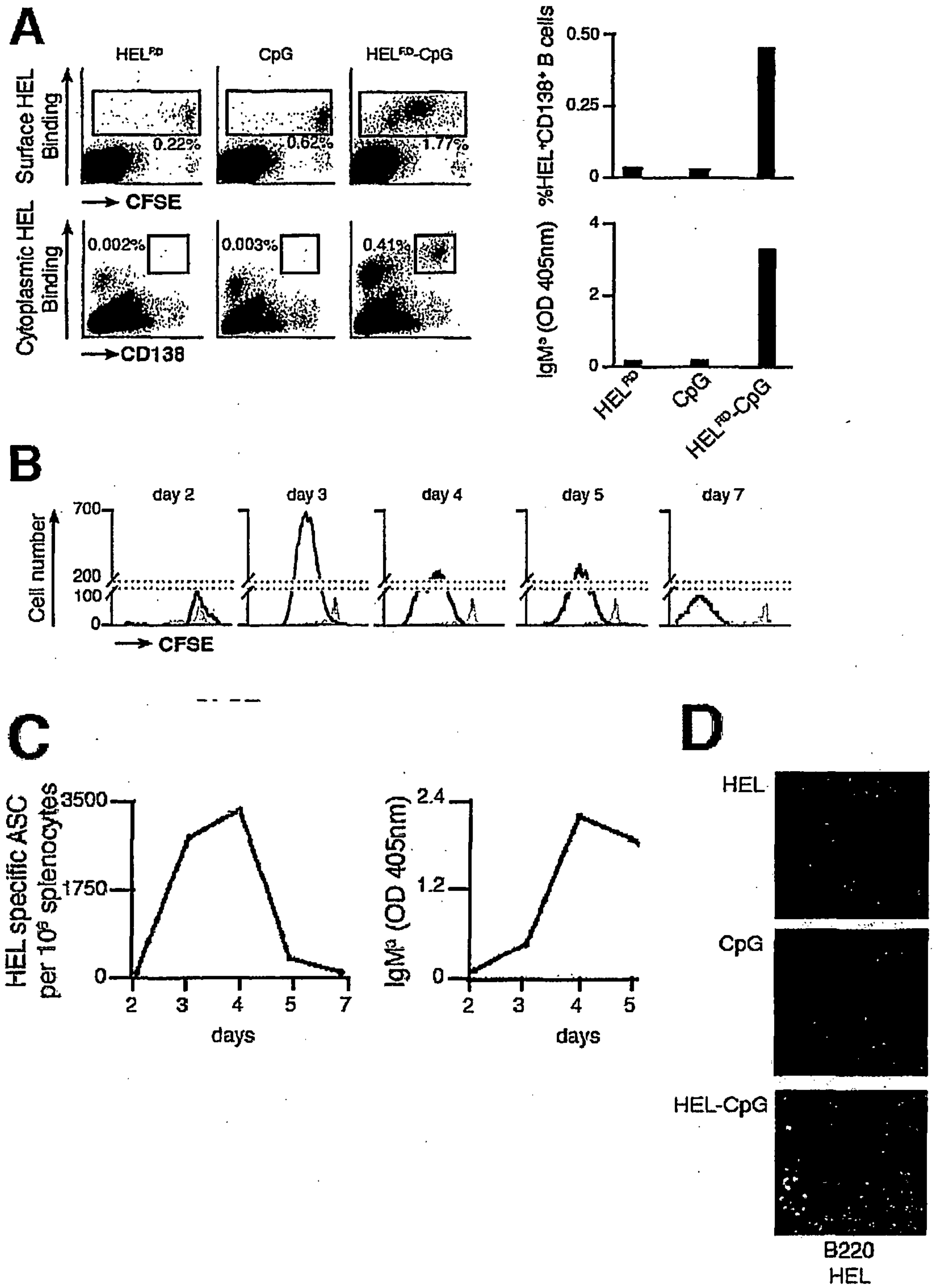
A
HEL^FD
CpG
HEL^FD-CpG
Surface HEL Binding
0.22%
0.62%
1.77%
CFSE
Cytoplasmic HEL Binding
0.002%
0.003%
0.41%
CD138
%HEL+CD138+ B cells
0.50
0.25
0
IgMa (OD 405nm)
4
2
0
HEL^FD
CpG
HEL^FD-CpG
B
day 2
day 3
day 4
day 5
day 7
Cell number
700
200
100
0
CFSE
C
HEL specific ASC per 10^6 splenocytes
3500
1750
0
2
3
4
5
7
days
IgMa (OD 405nm)
2.4
1.2
0
2
3
4
5
days
D
HEL
CpG
HEL-CpG
B220
HEL

Fig 17

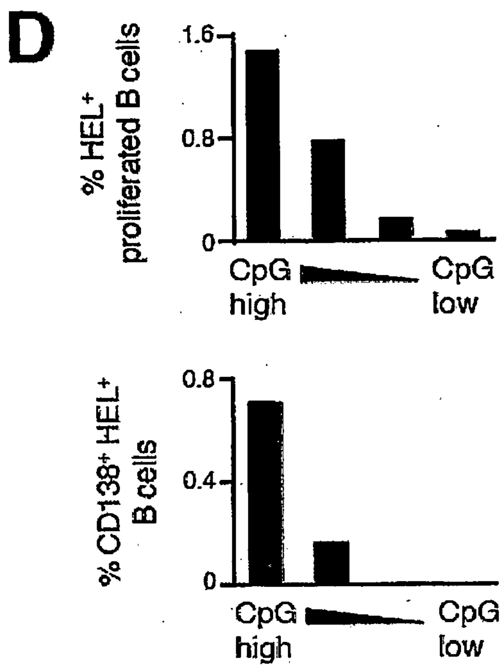
D
% HEL⁺ proliferated B cells
1.6
0.8
0
CpG high
CpG low
% CD138⁺ HEL⁺ B cells
0.8
0.4
0
CpG high
CpG low

Fig 18

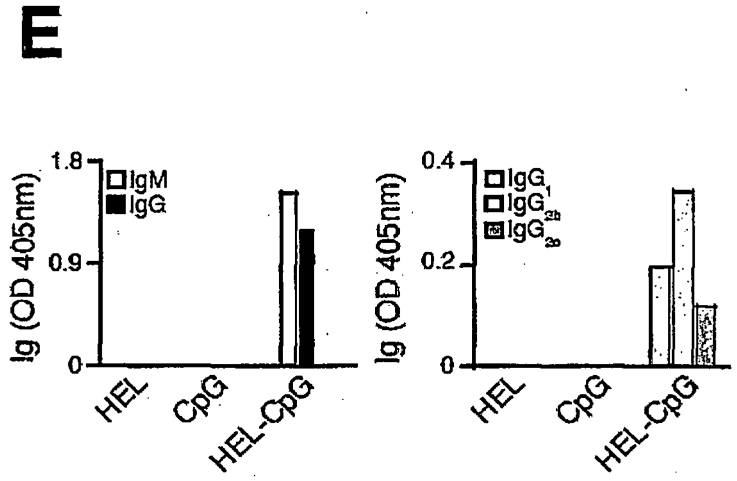
E
Ig (OD 405nm)
1.8
0.9
0
IgM
IgG
HEL
CpG
HEL-CpG
Ig (OD 405nm)
0.4
0.2
0
IgG1
IgG2b
IgG2a
HEL
CpG
HEL-CpG

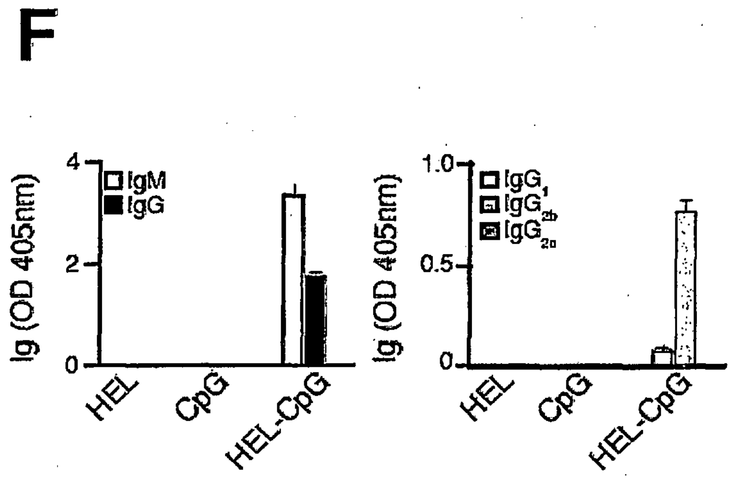
F
Ig (OD 405nm)
4
2
0
IgM
IgG
HEL
CpG
HEL-CpG
Ig (OD 405nm)
1.0
0.5
0
IgG1
IgG2b
IgG2a
HEL
CpG
HEL-CpG

Fig 19

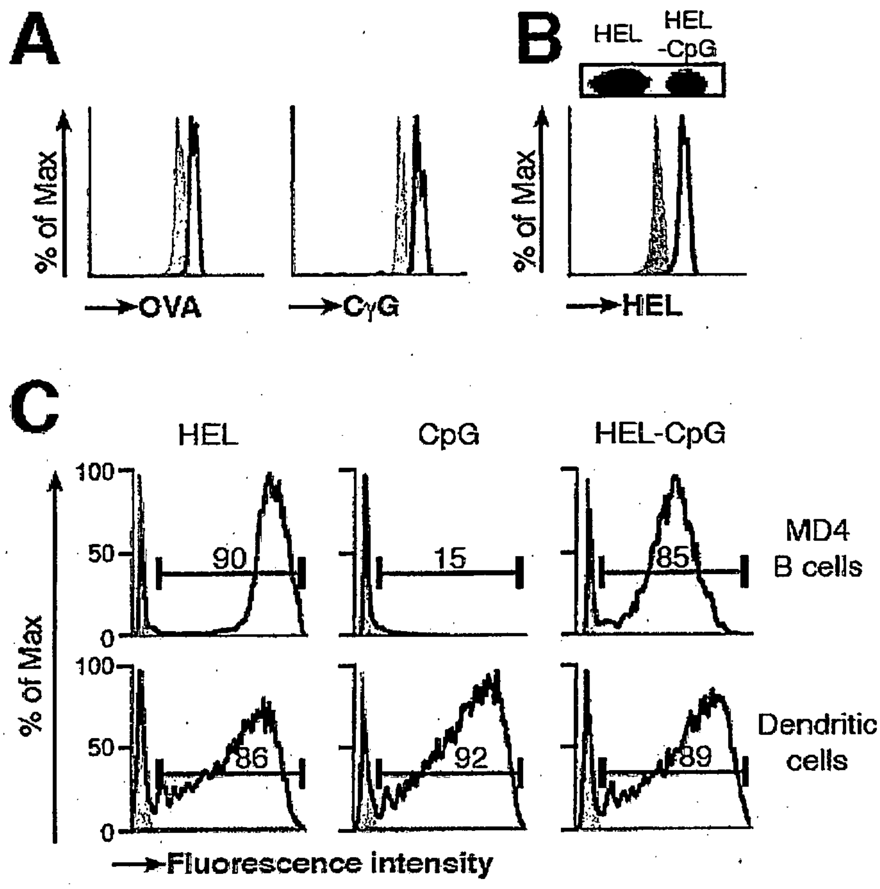
A
% of Max
OVA
CγG
B
HEL
HEL
-CpG
% of Max
HEL
C
HEL
CpG
HEL-CpG
100
50
0
90
15
85
MD4
B cells
% of Max
100
50
0
86
92
89
Dendritic
cells
Fluorescence intensity

Fig 20

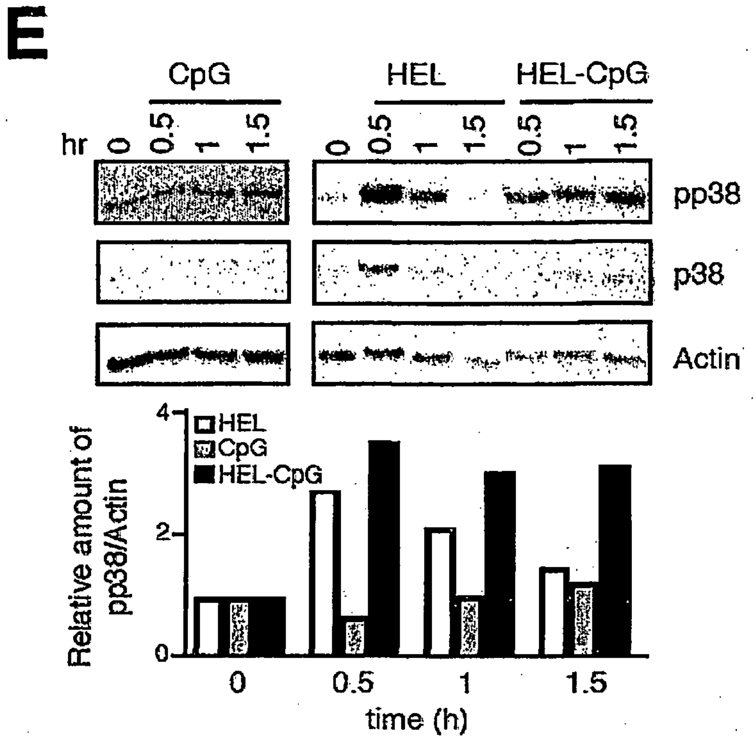
E
CpG
HEL
HEL-CpG
hr
0
0.5
1
1.5
0
0.5
1
1.5
0.5
1
1.5
pp38
p38
Actin
HEL
CpG
HEL-CpG
Relative amount of pp38/Actin
4
2
0
0
0.5
1
1.5
time (h)

Fig 21

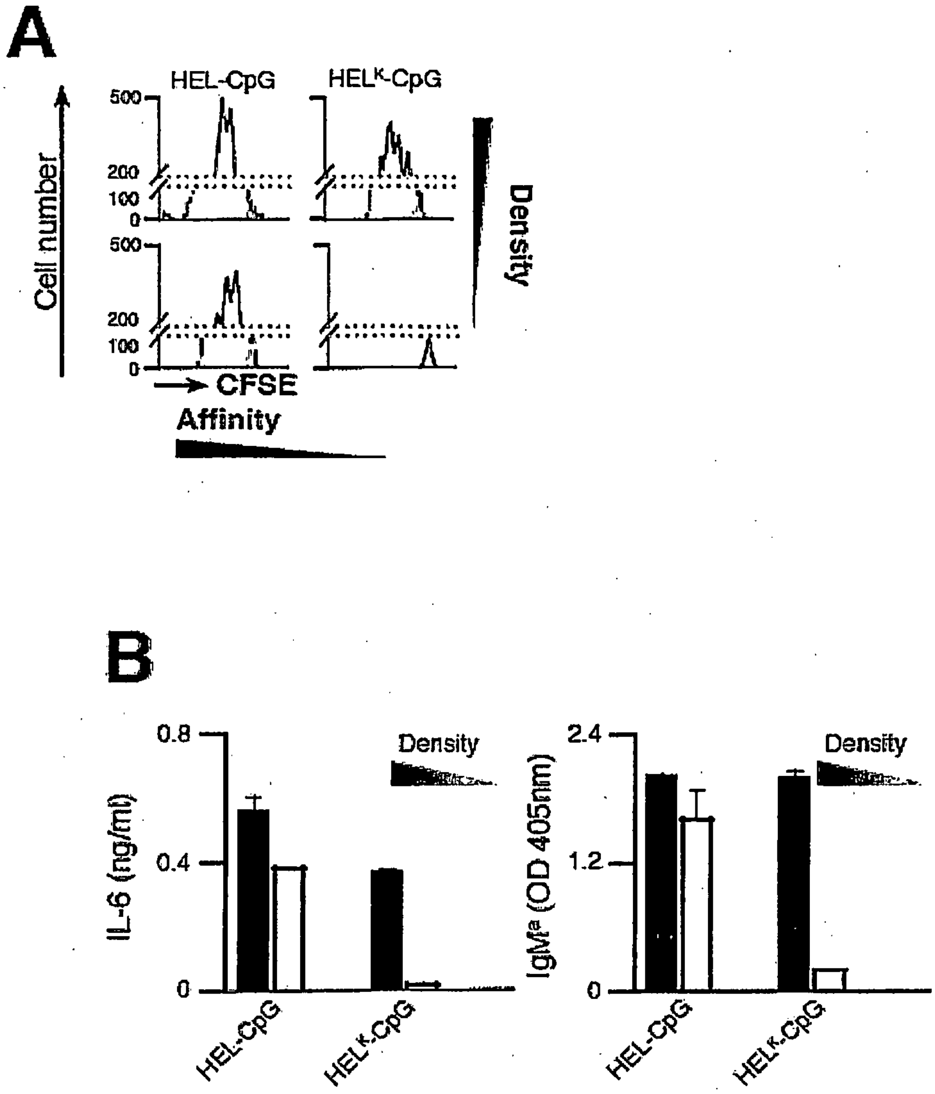
A
HEL-CpG
HEL^K-CpG
500
200
100
0
Cell number
Density
CFSE
Affinity
B
0.8
0.4
0
IL-6 (ng/ml)
Density
HEL-CpG
HEL^K-CpG
2.4
1.2
0
IgM^a (OD 405nm)
Density
HEL-CpG
HEL^K-CpG

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030157135 A
- WO 2007051004 A
- US 20070104776 A, Ishii
- WO 2006002642 A
- WO 2007050668 A
- WO 2004076677 A
- WO 9214488 A
- US 5523089 A
- WO 9004411 A
- WO 9109870 A
- WO 9304175 A
- WO 9606165 A
- WO 9308306 A
- US 9208697 W
- US 20070231344 A
- US 5776427 A
- WO 9201047 A


### Non-patent literature cited in the description


- **ULEVITCH.** *Nature reviews,* July 2004, vol. 4, 512-520
- **BANGHAM et al.** *J Mol Biol,* 1965, vol. 13, 238
- **ZAJONC ; KRENENBERG.** *Current Opinion in Structural Biology,* 2007, vol. 17, 521-529
- **SILK et al.** *Cutting Edge J. Immunol,* 2008
- **LANZAVECCHIA et al.** *Curr Opin Biotechnol,* 11 December 2007, vol. 18 (6), 523-8
- **TRAGGIAI E ; BECKER S ; SUBBARAO K ; KOLESNIKOVA L ; UEMATSU Y ; GISMONDO MR ; MURPHY BR ; RAPPUOLI R ; LANZAVECCHIA A.** *Nature Medicine,* 2004, vol. 10, 871-5
- **SHIRAHATA et al.** *Methods Cell Biol,* 1998, vol. 57, 111-45
- **WIESNER et al.** *PLoS ONE,* January 2008, vol. 1, 1-13
- **LI et al.** *PNAS,* 07 March 2006, vol. 103, 3557-3562
- **BERGSTROM et al.** *Mol. Microbiol.,* 1989, vol. 3, 479-486
- **JOHNSON et al.** *Infect. and Immun.,* 1992, vol. 60, 1845-1853
- **JOHNSON et al.** *Vaccine,* 1995, vol. 13, 1086-1094
- The Sixth International Conference on Lyme Borreliosis: Progress on the Development of Lyme Disease Vaccine. *Vaccine,* 1995, vol. 13, 133-135
- **C. G. A THOMAS.** *Medical Microbiology,* 1983
- Remington's Pharmaceutical Sciences. Mack Publishing Co, 1990
- *N. Engl. J. Med.,* 2001, vol. 345 (16), 1177-83
- *Arch. Intern. Med,* 1994, vol. 154 (22), 2545-57
- *Arch. Intern. Med,* 1994, vol. 154 (4), 373-7
- Roitt's Essential Immunology. Blackwell Publishing
- **ARMITAGE et al.** *Nature,* 1992, vol. 357, 80-82
- **RAJEWSKY K.** Clonal selection and learning in the antibody system. *Nature,* 1996, vol. 381, 751-758
- **RETH M ; WIENANDS J.** Initiation and processing of signals from the B cell antigen receptor. *Annu Rev Immunol,* 1997, vol. 15, 453-479
- **KUROSAKI T.** Regulation of B cell fates by BCR signaling components. *Curr Opin Immunol,* 2002, vol. 14, 341-347
- **LANZAVECCHIA.** Antigen-specific interaction between T and B cells. *Nature,* 1985, vol. 314, 537-539
- **MACLENNAN I ; TOELLNER K ; CUNNINGHAM A ; SERRE K ; SZE D ; ZÚÑIGA E ; COOK M ; VINUESA C.** Extrafollicular antibody responses. *Immunol Rev,* 2003, vol. 194, 8-18
- **MOLLER.** Role of somatic mutation in the generation of lymphocyte diversity. *Immunological reviews,* 1987, vol. 96, 1-162
- **AMIGORENA S ; DRAKE J ; WEBSTER P ; MELLMAN I.** Transient accumulation of new class II MHC molecules in a novel endocytic compartment in B lymphocytes. *Nature,* 1994, vol. 369, 113-120
- **BRIGL M ; BRENNER M.** CD1: antigen presentation and T cell function. *Annu Rev Immunol,* 2004, vol. 22, 817-890
- **CALABI F ; MILSTEIN C.** The molecular biology of CD1. *Semin Immunol,* 2000, vol. 12, 503-509
- **MOODY D ; PORCELLI S.** Intracellular pathways of CD1 antigen presentation. *Nat Rev Immunol,* 2003, vol. 3, 11-22
- **BENDELAC A ; SAVAGE P ; TEYTON L.** The biology of NKT cells. *Annu Rev Immunol,* 2007, vol. 25, 297-336

- **GODFREY D ; KRONENBERG M.** Going both ways: immune regulation via CD1d-dependent NKT cells. *J Clin Invest,* 2004, vol. 114, 1379-1388
- **VAN KAER L.** NKT cells: T lymphocytes with innate effector functions. *Curr Opin Immunol,* 2007, vol. 19, 354-364
- **KAWANO T ; CUI J ; KOEZUKA Y ; TOURA I ; KANEKO Y ; MOTOKI K ; UENO H ; NAKAGAWA R ; SATO H ; KONDO E et al.** CD1d-restricted and TCR-mediated activation of valpha14 NKT cells by glycosylceramides. *Science,* 1997, vol. 278, 1626-1629
- **SPADA F ; KOEZUKA Y ; PORCELLI S.** CD1d-restricted recognition of synthetic glycolipid antigens by human natural killer T cells. *J Exp Med,* 1998, vol. 188, 1529-1534
- **GALLI G ; NUTI S ; TAVARINI S ; GALLI-STAMPINO L ; DE LALLA C ; CASORATI G ; DELLABONA P ; ABRIGNANI S.** CD1d-restricted help to B cells by human invariant natural killer T lymphocytes. *J Exp Med,* 2003, vol. 197, 1051-1057
- **BEZBRADICA J ; STANIC A ; MATSUKI N ; BOUR-JORDAN H ; BLUESTONE J ; THOMAS J ; UNUTMAZ D ; VAN KAER L ; JOYCE S.** Distinct roles of dendritic cells and B cells in Va14Ja18 natural T cell activation in vivo. *J Immunol,* 2005, vol. 174, 4696-4705
- **FLEIRE SJ ; GOLDMAN JP ; CARRASCO YR ; WEBER M ; BRAY D ; BATISTA FD.** B cell ligand discrimination through a spreading and contraction response. *Science,* 2006, vol. 312, 738-741
- **PRIGOZY T ; NAIDENKO O ; QASBA P ; ELEWAUT D ; BROSSAY L ; KHURANA A ; NATORI T ; KOEZUKA Y ; KULKARNI A ; KRONENBERG M.** Glycolipid antigen processing for presentation by CD1d molecules. *Science,* 2001, vol. 291, 664-667
- **GAMMON G ; SHASTRI N ; COGSWELL J ; WILBUR S ; SADEGH-NASSERI S ; KRZYCH U ; MILLER A ; SERCARZ E.** The choice of T-cell epitopes utilized on a protein antigen depends on multiple factors distant from, as well as at the determinant site. *Immunol Rev,* 1987, vol. 98, 53-73
- **VAN DEN ELZEN P ; GARG S ; LEÓN L ; BRIGL M ; LEADBETTER E ; GUMPERZ J ; DASCHER C ; CHENG T ; SACKS F ; ILLARIONOV P et al.** Apolipoprotein-mediated pathways of lipid antigen presentation. *Nature,* 2005, vol. 437, 906-910
- **VIDARD L ; KOVACSOVICS-BANKOWSKI M ; KRAEFT S ; CHEN L ; BENACERRAF B ; ROCK K.** Analysis of MHC class II presentation of particulate antigens of B lymphocytes. *J Immunol,* 1996, vol. 156, 2809-2818
- **BATISTA F ; NEUBERGER M.** B cells extract and present immobilized antigen: implications for affinity discrimination. *EMBO J,* 2000, vol. 19, 513-520
- **LIN K ; FREEMAN S ; ZABETIAN S ; BRUGGER H ; WEBER M ; LEI V ; DANG-LAWSON M ; TSE K ; SANTAMARIA R ; BATISTA F et al.** The rap GTPases regulate B cell morphology, immune-synapse formation, and signaling by particulate B cell receptor ligands. *Immunity,* 2008, vol. 28, 75-87
- **MATTNER J ; DEBORD K ; ISMAIL N ; GOFF R ; CANTU C ; ZHOU D ; SAINT-MEZARD P ; WANG V ; GAO Y ; YIN N et al.** Exogenous and endogenous glycolipid antigens activate NKT cells during microbial infections. *Nature,* 2005, vol. 434, 525-529
- **KINJO Y ; WU D ; KIM G ; XING G ; POLES M ; HO D ; TSUJI M ; KAWAHARA K ; WONG C ; KRONENBERG M.** Recognition of bacterial glycosphingolipids by natural killer T cells. *Nature,* 2005, vol. 434, 520-525
- **GALLI G ; PITTONI P ; TONTI E ; MALZONE C ; UEMATSU Y ; TORTOLI M ; MAIONE D ; VOLPINI G ; FINCO O ; NUTI S et al.** Invariant NKT cells sustain specific B cell responses and memory. *Proc Natl Acad Sci USA,* 2007, vol. 104, 3984-3989
- **TUPIN E ; KINJO Y ; KRONENBERG M.** The unique role of natural killer T cells in the response to microorganisms. *Nat Rev Microbiol,* 2007, vol. 5, 405-417
- **HANSEN D ; SIOMOS M ; DE KONING-WARD T ; BUCKINGHAM L ; CRABB B ; SCHOFIELD L.** CD1d-restricted NKT cells contribute to malarial splenomegaly and enhance parasite-specific antibody responses. *Eur J Immunol,* 2003, vol. 33, 2588-2598
- **KINJO Y ; TUPIN E ; WU D ; FUJIO M ; GARCIA-NAVARRO R ; BENHNIA M ; ZAJONC D ; BEN-MENACHEM G ; AINGE G ; PAINTER G et al.** Natural killer T cells recognize diacylglycerol antigens from pathogenic bacteria. *Nat Immunol,* 2006, vol. 7, 978-986
- **BRIGL M ; BRY L ; KENT S ; GUMPERZ J ; BRENNER M.** Mechanism of CD1d-restricted natural killer T cell activation during microbial infection. *Nat Immunol,* 2003, vol. 4, 1230-1237
- **SALIO M ; SPEAK A ; SHEPHERD D ; POLZELLA P ; ILLARIONOV P ; VEERAPEN N ; BESRA G ; PLATT F ; CERUNDOLO V.** Modulation of human natural killer T cell ligands on TLR-mediated antigen-presenting cell activation. *Proc Natl Acad Sci USA,* 2007, vol. 104, 20490-20495
- **PAGET C ; MALLEVAEY T ; SPEAK A ; TORRES D ; FONTAINE J ; SHEEHAN K ; CAPRON M ; RYFFEL B ; FAVEEUW C ; LEITE DE MORAES M et al.** Activation of invariant NKT cells by toll-like receptor 9-stimulated dendritic cells requires type I interferon and charged glycosphingolipids. *Immunity,* 2007, vol. 27, 597-609

- **ROARK J ; PARK S ; JAYAWARDENA J ; KAVITA U ; SHANNON M ; BENDELAC A.** CD1.1 expression by mouse antigen-presenting cells and marginal zone B cells. *J Immunol,* 1998, vol. 160, 3121-3127
- **MARTIN F ; KEARNEY J.** Marginal-zone B cells. *Nat Rev Immunol,* 2002, vol. 2, 323-335
- **YUAN W ; QI X ; TSANG P ; KANG S ; ILLARIONOV P ; ILLANIOROV P ; BESRA G ; GUMPERZ J ; CRESSWELL P.** Saposin B is the dominant saposin that facilitates lipid binding to human CD1d molecules. *Proc Natl Acad Sci USA,* 2007, vol. 104, 5551-5556
- **PHAN T ; AMESBURY M ; GARDAM S ; CROSBIE J ; HASBOLD J ; HODGKIN P ; BASTEN A ; BRINK R.** B cell receptor-independent stimuli trigger immunoglobulin (Ig) class switch recombination and production of IgG autoantibodies by anergic self-reactive B cells. *J Exp Med,* 2003, vol. 197, 845-860
- **CHIRON D ; BEKEREDJIAN-DING I ; PELLAT-DECEUNYNCK C ; BATAILLE R ; JEGO G.** Toll-like receptors: lessons to learn from normal and malignant human B cells. *Blood,* 30 June 2008